(19) Europäisches Patentamt
European Patent Office
Office européen des brevets

(11) EP 4 105 201 A1

(12) EUROPEAN PATENT APPLICATION

(43) Date of publication:
**21.12.2022 Bulletin 2022/51**

(21) Application number: **21180305.1**

(22) Date of filing: **18.06.2021**

(51) International Patent Classification (IPC):
***C07D 213/61*** *(2006.01)* ***C07D 209/86*** *(2006.01)*
***C09K 11/06*** *(2006.01)* ***C09B 57/00*** *(2006.01)*
***C09B 57/10*** *(2006.01)* ***H01L 31/00*** *(2006.01)*
***H01L 31/04*** *(2014.01)* ***H01L 51/00*** *(2006.01)*
***H01L 51/50*** *(2006.01)* ***H01M 14/00*** *(2006.01)*

(52) Cooperative Patent Classification (CPC):
**H01L 51/0091; C07D 209/86; C07D 213/61; C09B 57/00; C09B 57/10; C09K 11/06;**
H01L 51/5056; H01L 51/506; H01L 51/5064

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Novaled GmbH**
**01099 Dresden (DE)**

(72) Inventors:
- **UVAROV, Vladimir**
  **01099 Dresden (DE)**
- **HEGGEMANN, Ulrich**
  **01099 Dresden (DE)**
- **WILLMANN, Steffen**
  **01099 Dresden (DE)**
- **LUSCHTINETZ, Regina**
  **01099 Dresden (DE)**

(74) Representative: **Michalski Hüttermann & Partner Patentanwälte mbB**
**Kaistraße 16A**
**40221 Düsseldorf (DE)**

# (54) AN ORGANIC ELECTROLUMINESCENT DEVICE COMPRISING A SUBSTRATE, AN ANODE LAYER AND A CATHODE LAYER, AT LEAST ONE LIGHT EMITTING LAYER, AND AT LEAST ONE SEMICONDUCTOR LAYER THAT COMPRISES AT LEAST ONE METAL COMPOUND OF A METAL AND AT LEAST ONE LIGAND

(57) The present invention relates to an organic electroluminescent device comprising a substrate, an anode layer and a cathode layer, at least one light emitting layer, and at least one semiconductor layer or more, wherein the at least one semiconductor layer comprises at least one metal compound, wherein the metal compound comprises a metal M and at least one ligand L.

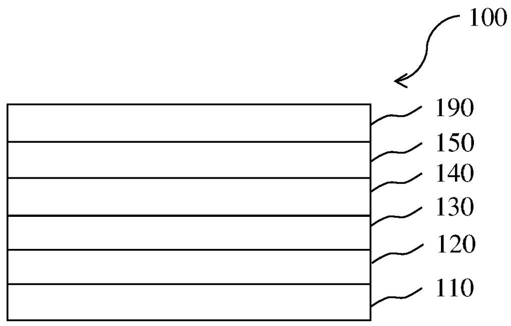


Fig. 1

EP 4 105 201 A1

## Description

### Technical Field

The present invention relates to an organic electroluminescent device comprising a substrate, an anode layer and a cathode layer, at least one light emitting layer, and at least one semiconductor layer that comprises at least one metal compound of a metal and at least one ligand, and an organic electronic device comprising the electroluminescent device .

### Background Art

Organic light-emitting diodes OLEDs, which are self-emitting devices, have a wide viewing angle, excellent contrast, quick response, high brightness, excellent operating voltage characteristics, and color reproduction. A typical OLED comprises an anode layer, a hole injection layer HIL, a hole transport layer HTL, a light emitting layer EML, an electron transport layer ETL, and a cathode layer, which are sequentially stacked on a substrate. In this regard, the HIL, the HTL, the EML, and the ETL are thin films formed from organic compounds.

When a voltage is applied to the anode and the cathode, holes injected from the anode move to the EML, via the HIL and HTL, and electrons injected from the cathode move to the EML, via the ETL. The holes and electrons recombine in the EML to generate excitons. When the excitons drop from an excited state to a ground state, light is emitted. The injection and flow of holes and electrons should be balanced, so that an OLED having the above-described structure has low operating voltage, excellent efficiency and/or a long lifetime.

Performance of an organic light emitting diode device may be affected by characteristics of the hole injection layer, and among them, may be affected by characteristics of the hole transport compound and the metal complexes which are contained in the hole injection layer.

CN 111018921 relates to metal complex and an electroluminescent device comprising the same, wherein the metal complex contains a novel structure composed of a metal and a ligand.

There remains a need to improve performance of light-emitting diode and organic electronic devices by providing semiconductor layer with improved performance, in particular to achieve improved operating voltage, improved lifetime, current efficiency and/or improved operating voltage stability over time through improving the characteristics of the compounds comprised therein.

Further there remains a need to provide semiconductor layer, for example hole injection layers (HIL) and/or hole transport layers (HTL), which enable injection into adjacent layers comprising compounds with a HOMO level further away from vacuum level.

It is a further objective to provide compounds with improved thermal properties as well as semiconductor layers, for example hole injection layers (HIL) and/or hole transport layers (HTL), which have an improved thermal stability.

It is a further objective to provide a semiconductor layer, for example hole injection layers (HIL) and/or hole transport layers (HTL), which comprising compounds which can be deposited through vacuum thermal evaporation under conditions suitable for mass production.

DISCLOSURE

An aspect provides an organic electroluminescent device comprising a substrate, an anode layer and a cathode layer, at least one light emitting layer, and at least one semiconductor layer or more, wherein the semiconductor layer comprises at least one metal compound, wherein the at least one semiconductor layer comprises at least one metal compound, wherein the metal compound comprises a metal M and at least one ligand L or the metal compound has the formula (I):

$$M^{p\oplus}\left[{}^{\ominus}L\right]_{w} \quad (I),$$

wherein

M is a metal ion;
p is the valency of M;
w is n
L is a ligand;

wherein the metal compound comprises

- a metal, and
- at least one ligand, wherein the at least one ligand comprises a coordinating linker X and at least one ligand fragment Y, wherein the coordinating linker X and the at least one ligand fragment Y are linked to each other by at least one bond, wherein

  - the coordinating linker X is selected from formula (IIa)

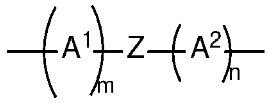
(IIa),

wherein

m and n are independently selected from 0 or 1;
Z is selected from the group comprising $CR^1$, O, N; and
$A^1$ and $A^2$ are independently selected from the group comprising C=O, C-O, $C=NR^2$, $C-NR^3$, SO, $SO_2$, or $P(=O)R^4$;
wherein $A^1$ and $A^2$ together optional form a cycle with Z;
wherein $R^1$, $R^2$, $R^3$ and $R^4$ are independently selected from H, D, CN, substituted or unsubstituted $C_6$ to $C_{24}$ aryl, substituted or unsubstituted $C_2$ to $C_{24}$ heteroaryl, substituted or unsubstituted $C_1$ to $C_{24}$ alkyl, substituted or unsubstituted $C_3$ to $C_{24}$ carbocyclyl, or substituted or unsubstituted $C_2$ to $C_{24}$ heterocyclyl, ligand fragment Y, wherein
the substituents are selected from the group comprising electron-withdrawing group, preferably halogen, F, Cl, CN, perfluorinated $C_1$ to $C_8$ alkyl, perfluorinated $C_6$ to $C_{12}$ aryl, or $C_2$ to $C_{12}$ heteroaryl and/or;

  - the at least one ligand fragment Y is independently selected from substituted or unsubstituted $C_6$ to $C_{24}$ aryl, substituted or unsubstituted $C_2$ to $C_{24}$ heteroaryl, substituted or unsubstituted $C_1$ to $C_{24}$ alkyl, substituted or unsubstituted $C_3$ to $C_{24}$ carbocyclyl, substituted or unsubstituted $C_2$ to $C_{24}$ heterocyclyl, perfluorinated $C_1$ to $C_4$ alkyl, substituted $C_6$ to $C_{24}$ aryl in 3-position, substituted $C_6$ to $C_{24}$ aryl in 4-position, substituted 4-pyridyl, or substituted 5-pyrimidyl, wherein
    the at least one ligand fragment Y has a directed dipole moment of less than -3.6 debye, and wherein the at least one ligand L is negatively charged; wherein

the anode layer comprises a first anode sub-layer and a second anode sub-layer, wherein

- the first anode sub-layer comprises a first metal having a work function in the range of $\geq 4$ and $\leq 6$ eV, and
- the second anode sub-layer comprises a transparent conductive oxide (TCO); and the at least one semiconductor layer is arranged between the first light emitting layer and the anode layer,
- the first anode sub-layer is arranged closer to the substrate, and
- the second anode sub-layer is arranged closer to the at least one semiconductor layer;

wherein the following ligand fragments Y are excluded:

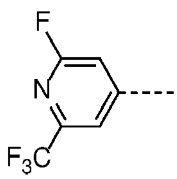
,
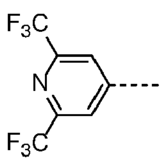
,
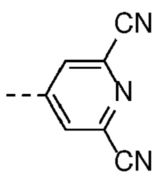
,
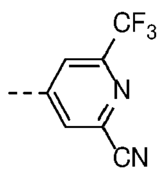
,
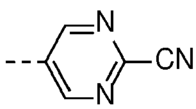
,

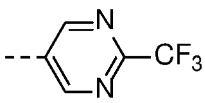 , 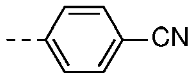 , 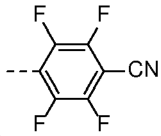 and 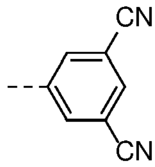 .

According to one embodiment the organic electroluminescent device comprising a substrate, an anode layer and a cathode layer, at least one light emitting layer, and at least one or more semiconductor layer, wherein the at least one or more semiconductor layer comprises a hole injection layer, or a hole injection layer and a hole transport layer, comprising at least one metal compound, wherein the metal compound comprises a metal M and at least one ligand L or the metal compound has the formula (I), wherein
the metal compound comprises

- a metal M, and
- at least one ligand L, wherein the at least one ligand L comprises a coordinating linker X and at least one ligand fragment Y,
  wherein the coordinating linker X and the at least one ligand fragment Y are linked to each other by at least one bond, wherein

  - the coordinating linker X is selected from formula (IIa)

$$-\left(A^1\right)_m-Z-\left(A^2\right)_n- \quad \text{(IIa)},$$

  wherein

    m and n are independently selected from 0 or 1;
    Z is selected from the group comprising $CR^1$, O, N; and
    $A^1$ and $A^2$ are independently selected from the group comprising C=O, C-O, $C=NR^2$, $C-NR^3$, SO, $SO_2$, or $P(=O)R^4$;
    wherein $A^1$ and $A^2$ together optional form a cycle with Z;
    wherein $R^1$, $R^2$, $R^3$ and $R^4$ are independently selected from H, D, CN, substituted or unsubstituted $C_6$ to $C_{24}$ aryl, substituted or unsubstituted $C_2$ to $C_{24}$ heteroaryl, substituted or unsubstituted $C_1$ to $C_{24}$ alkyl, substituted or unsubstituted $C_3$ to $C_{24}$ carbocyclyl, or substituted or unsubstituted $C_2$ to $C_{24}$ heterocyclyl, ligand fragment Y, wherein
    the substituents are selected from the group comprising electron-withdrawing group, preferably halogen, F, Cl, CN, perfluorinated $C_1$ to $C_8$ alkyl, perfluorinated $C_6$ to $C_{12}$ aryl, or $C_2$ to $C_{12}$ heteroaryl and/or;

  - the at least one ligand fragment Y is independently selected from substituted or unsubstituted $C_6$ to $C_{24}$ aryl, substituted or unsubstituted $C_2$ to $C_{24}$ heteroaryl, substituted or unsubstituted $C_1$ to $C_{24}$ alkyl, substituted or unsubstituted $C_3$ to $C_{24}$ carbocyclyl, substituted or unsubstituted $C_2$ to $C_{24}$ heterocyclyl, perfluorinated $C_1$ to $C_4$ alkyl, substituted $C_6$ to $C_{24}$ aryl in 3-position, substituted $C_6$ to $C_{24}$ aryl in 4-position, substituted 4-pyridyl, or substituted 5-pyrimidyl, and
  - more preferred the at least one ligand fragment Y is independently selected from substituted or unsubstituted $C_6$ to $C_{24}$ aryl, substituted or unsubstituted $C_2$ to $C_{24}$ heteroaryl, wherein

    the substituents are selected from the group comprising an electron-withdrawing group, halogen, F, Cl, and/or CN;

  and
  wherein the at least one ligand fragment Y has a directed dipole moment of less than -3.6 debye, and wherein the at least one ligand L is negatively charged; wherein

the anode layer comprises a first anode sub-layer and a second anode sub-layer, wherein

- the first anode sub-layer comprises a first metal having a work function in the range of ≥ 4 and ≤ 6 eV, and
- the second anode sub-layer comprises a transparent conductive oxide (TCO); and the hole injection layer is arranged between the first light emitting layer and the anode layer,
- the first anode sub-layer is arranged closer to the substrate, and
- the second anode sub-layer is arranged closer to the hole injection layer;

wherein the following ligand fragments Y are excluded:

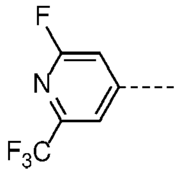,
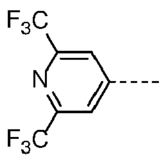,
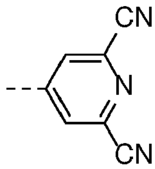,
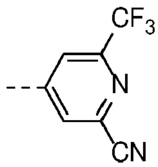,
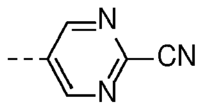,

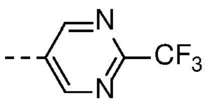,
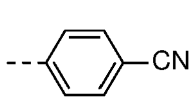,
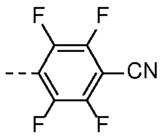
and
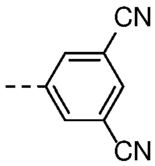.

According to one embodiment the organic electroluminescent device comprising a substrate, an anode layer and a cathode layer, at least one light emitting layer, and at least one or more semiconductor layer comprisinga hole transport layer, or a hole injection layer and a hole transport layer, wherein
the hole transport layer, or a hole injection layer and a hole transport layer comprises at least one metal compound, wherein the metal compound comprises a metal M and at least one ligand L or the metal compound has the formula (I), wherein
the metal compound comprises

- a metal M, and
- at least one ligand L, wherein the at least one ligand L comprises a coordinating linker X and at least one ligand fragment Y,
  wherein the coordinating linker X and the at least one ligand fragment Y are linked to each other by at least one bond, wherein

  - the coordinating linker X is selected from formula (IIa)

$$-\left(A^1\right)_m-Z-\left(A^2\right)_n- \quad \text{(IIa)},$$

  wherein

  m and n are independently selected from 0 or 1;
  Z is selected from the group comprising $CR^1$, O, N; and
  $A^1$ and $A^2$ are independently selected from the group comprising C=O, C-O, $C=NR^2$, $C-NR^3$, SO, $SO_2$, or $P(=O)R^4$;
  wherein $A^1$ and $A^2$ together optional form a cycle with Z;
  wherein $R^1$, $R^2$, $R^3$ and $R^4$ are independently selected from H, D, CN, substituted or unsubstituted $C_6$ to $C_{24}$ aryl, substituted or unsubstituted $C_2$ to $C_{24}$ heteroaryl, substituted or unsubstituted $C_1$ to $C_{24}$ alkyl, substituted or unsubstituted $C_3$ to $C_{24}$ carbocyclyl, or substituted or unsubstituted $C_2$ to $C_{24}$ heterocyclyl, ligand fragment Y, wherein
  the substituents are selected from the group comprising electron-withdrawing group, preferably halogen, F, Cl, CN, perfluorinated $C_1$ to $C_8$ alkyl, perfluorinated $C_6$ to $C_{12}$ aryl, or $C_2$ to $C_{12}$ heteroaryl and/or;

  - the at least one ligand fragment Y is independently selected from substituted or unsubstituted $C_6$ to $C_{24}$ aryl,

substituted or unsubstituted $C_2$ to $C_{24}$ heteroaryl, substituted or unsubstituted $C_1$ to $C_{24}$ alkyl, substituted or unsubstituted $C_3$ to $C_{24}$ carbocyclyl, substituted or unsubstituted $C_2$ to $C_{24}$ heterocyclyl, perfluorinated $C_1$ to $C_4$ alkyl, substituted $C_6$ to $C_{24}$ aryl in 3-position, substituted $C_6$ to $C_{24}$ aryl in 4-position, substituted 4-pyridyl, or substituted 5-pyrimidyl, and

- more preferred the at least one ligand fragment Y is independently selected from substituted or unsubstituted $C_6$ to $C_{24}$ aryl, substituted or unsubstituted $C_2$ to $C_{24}$ heteroaryl,
  wherein
  the substituents are selected from the group comprising an electron-withdrawing group, halogen, F, Cl, and/or CN; and
  wherein the at least one ligand fragment Y has a directed dipole moment of less than -3.6 debye, and wherein the at least one ligand L is negatively charged; wherein

the anode layer comprises a first anode sub-layer and a second anode sub-layer, wherein

- the first anode sub-layer comprises a first metal having a work function in the range of ≥ 4 and ≤ 6 eV, and
- the second anode sub-layer comprises a transparent conductive oxide (TCO); and the hole transport layer is arranged between the first light emitting layer and the anode layer,
- the first anode sub-layer is arranged closer to the substrate, and
- the second anode sub-layer is arranged closer to the hole transport layer;

wherein the following ligand fragments Y are excluded:

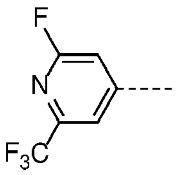, 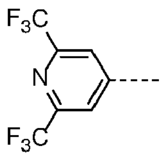, 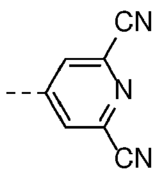, 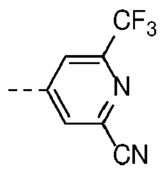, 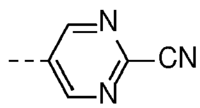,

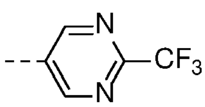, 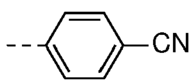, 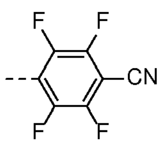 and 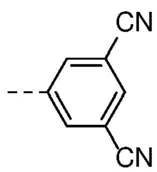.

According to one embodiment the organic electroluminescent device comprising a substrate, an anode layer and a cathode layer, at least one light emitting layer, and at least one or more semiconductor layer, which are selected from the group comprising a hole transport layer and a hole injection layer, wherein
the semiconductor layer comprises at least one metal compound, wherein
the metal compound comprises a metal M and at least one ligand L or the metal compound has the formula (I), comprising

- a metal, and
- at least one ligand, wherein the at least one ligand comprises a coordinating linker X and at least one ligand fragment Y, wherein the coordinating linker X and the at least one ligand fragment Y are linked to each other by at least one bond, wherein

  - the coordinating linker X is selected from formula (IIa)

$$-\left(A^1\right)_m-Z-\left(A^2\right)_n- \quad \text{(IIa)},$$

wherein

m and n are independently selected from 0 or 1;
Z is selected from the group comprising $CR^1$, O, N; and

$A^1$ and $A^2$ are independently selected from the group comprising C=O, C-O, C=$NR^2$, C-$NR^3$, SO, $SO_2$, or P(=O)$R^4$;
wherein $A^1$ and $A^2$ together optional form a cycle with Z;
wherein $R^1$, $R^2$, $R^3$ and $R^4$ are independently selected from H, D, CN, substituted or unsubstituted $C_6$ to $C_{24}$ aryl, substituted or unsubstituted $C_2$ to $C_{24}$ heteroaryl, substituted or unsubstituted $C_1$ to $C_{24}$ alkyl, substituted or unsubstituted $C_3$ to $C_{24}$ carbocyclyl, or substituted or unsubstituted $C_2$ to $C_{24}$ heterocyclyl, ligand fragment Y, wherein
the substituents are selected from the group comprising electron-withdrawing group, preferably halogen, F, Cl, CN, perfluorinated $C_1$ to $C_8$ alkyl, perfluorinated $C_6$ to $C_{12}$ aryl, or $C_2$ to $C_{12}$ heteroaryl and/or;

- the at least one ligand fragment Y is independently selected from substituted or unsubstituted $C_6$ to $C_{24}$ aryl, substituted or unsubstituted $C_2$ to $C_{24}$ heteroaryl, substituted or unsubstituted $C_1$ to $C_{24}$ alkyl, substituted or unsubstituted $C_3$ to $C_{24}$ carbocyclyl, substituted or unsubstituted $C_2$ to $C_{24}$ heterocyclyl, perfluorinated $C_1$ to $C_4$ alkyl, substituted $C_6$ to $C_{24}$ aryl in 3-position, substituted $C_6$ to $C_{24}$ aryl in 4-position, substituted 4-pyridyl, or substituted 5-pyrimidyl, and
- more preferred the at least one ligand fragment Y is independently selected from substituted or unsubstituted $C_6$ to $C_{24}$ aryl, substituted or unsubstituted $C_2$ to $C_{24}$ heteroaryl,
wherein
the substituents are selected from the group comprising an electron-withdrawing group, halogen, F, Cl, and/or CN;
and
wherein the at least one ligand fragment Y has a directed dipole moment of less than -3.6 debye, and wherein the at least one ligand L is negatively charged; wherein

the anode layer comprises a first anode sub-layer and a second anode sub-layer, wherein

- the first anode sub-layer comprises a first metal having a work function in the range of ≥ 4 and ≤ 6 eV, and
- the second anode sub-layer comprises a transparent conductive oxide (TCO); and the hole transport layer and the hole injection layer are arranged between the first light emitting layer and the anode layer,
- the first anode sub-layer is arranged closer to the substrate, and
- the second anode sub-layer is arranged closer to the hole injection layer;

wherein the following ligand fragments Y are excluded:

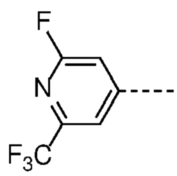,
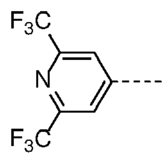,
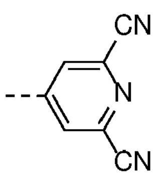,
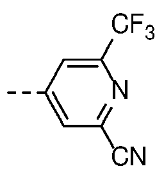,
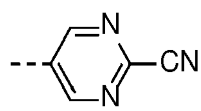,

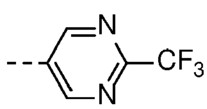,
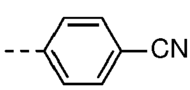,
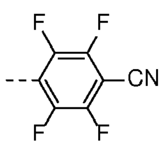 and
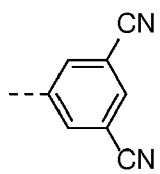.

According to one embodiment the at least one semiconductor layer can be a hole injection layer and/or hole transport layer, and preferably the at least one semiconductor layer, which can be a hole injection layer, is non-emissive.
In the context of the present specification the term "consisting essentially of " especially means and/or includes a concentration of ≥ 90% (vol/vol) more preferred ≥ 95% (vol/vol) and most preferred ≥ 99% (vol/vol).
It should be noted that throughout the application and the claims any $B^n$,$R^n$ etc. always refer to the same moieties, unless otherwise noted.
In the present specification, the term "ligand" refers to an anionic molecule which binds to a cationic metal either by a dative bond or ionic interaction, preferably a dative bond, wherein the nature of the dative bond can have a character ranging from a covalent bond to an ionic bond.

In the present specification, when a definition is not otherwise provided, "partially fluorinated" refers to an alkyl group, an aryl group, a heteroaryl group etc. in which only part of the hydrogen atoms is replaced by fluorine atoms.

In the present specification, when a definition is not otherwise provided, "perfluorinated" refers to an alkyl group, an aryl group, a heteroaryl group etc. in which all hydrogen atoms are replaced by fluorine atoms.

Unless otherwise noted the term "metal" as used herein includes metal cations if the metal is referred to together with an anion, wherein the positive charge of the metal cation and the negative charge of the anion balance each other.

In the present specification, an electron withdrawing group (EWG) is a group that reduces electron density in a molecule through the carbon atom it is bonded to. Typical examples of electron withdrawing groups are halogen, in particular F and Cl, -COR, -COH, -COOR, -COOH, partially fluorinated or perfluorinated alkyl, partially fluorinated or perfluorinated aryl, partially fluorinated or perfluorinated heteroaryl, partially fluorinated or perfluorinated carbocyclyl, partially fluorinated or perfluorinated $C_2$ to $C_{20}$ heterocyclyl, $-NO_2$, and -CN.

According to one embodiment, wherein the electron-withdrawing group is independently selected from the group comprising halogen, wherein the halogen is preferably selected F, $C_1$ to C3 perhalogenated, wherein the $C_1$ to $C_3$ perhalogenated is preferably perfluorinated, partially or perfluorinated $C_1$ to $C_6$ alkoxy, or $-(O)_1-C_qH_{2q}-C_pHal_{p2p+1}$ with 1 = 0 or 1, preferably 0, q = 1 or 2, preferably 1 and p = 1 to 3, preferably 1 or 2 and Hal = halogen, preferably F.

According to one embodiment the electron-withdrawing group is independently selected from substituted with $C_1$ to $C_8$ alkyl or $C_1$ to $C_8$ alkoxy and the substituents of the $C_1$ to $C_8$ alkyl or $C_1$ to $C_8$ alkoxy moiety are fluorine with the number $n_F$ (of fluorine substituents) and $n_H$ (of hydrogens) follow the equation: $n_F > n_H + 2$.

According to one embodiment the electron-withdrawing group is independently selected from perfluorinated $C_1$ to $C_8$ alkyl or perfluorinated $C_1$ to $C_8$ alkoxy.

According to one embodiment the electron-withdrawing group is independently selected from partially or perfluorinated $C_3$ to $C_6$ alkyl.

According to one embodiment, the electron-withdrawing group is independently selected from $CF_3$ or CN.

According to one embodiment the electron-withdrawing group is independently selected from $CF_3$, $C_2F_5$, $C_3F_7$ or $C_4F_9$, preferably $CF_3$.

In the present specification, when a definition is not otherwise provided, "substituted" refers to one substituted with a deuterium, $C_6$ to $C_{12}$ aryl, $C_3$ to $C_{11}$ heteroaryl, and $C_1$ to $C_{12}$ alkyl, D, $C_1$ to $C_{12}$ alkoxy, $C_3$ to $C_{12}$ branched alkyl, $C_3$ to $C_{12}$ cyclic alkyl, $C_3$ to $C_{12}$ branched alkoxy, $C_3$ to $C_{12}$ cyclic alkoxy, partially or perfluorinated $C_1$ to $C_{12}$ alkyl, partially or perfluorinated $C_1$ to $C_{12}$ alkoxy, partially or perdeuterated $C_1$ to $C_{12}$ alkyl, partially or perdeuterated $C_1$ to $C_{12}$ alkoxy, halogen, CN or $PY(R^{10})_2$, wherein Y is selected from O, S or Se, preferably O and $R^{10}$ is independently selected from $C_6$ to $C_{12}$ aryl, $C_3$ to $C_{12}$ heteroaryl, $C_1$ to $C_6$ alkyl, $C_1$ to $C_6$ alkoxy, partially or perfluorinated $C_1$ to $C_6$ alkyl, partially or perfluorinated $C_1$ to C6 alkoxy, partially or perdeuterated $C_1$ to $C_6$ alkyl, partially or perdeuterated $C_1$ to $C_6$ alkoxy, and preferably refers to one substituted with a deuterium, also named D, halogen, Cl, F, CN, partially or perfluorinated $C_1$ to $C_8$ alkyl, partially or perfluorinated $C_1$ to $C_8$ alkoxy.

In the present specification, "aryl group" refers to a hydrocarbyl group which can be created by formal abstraction of one hydrogen atom from an aromatic ring in the corresponding aromatic hydrocarbon. Aromatic hydrocarbon refers to a hydrocarbon which contains at least one aromatic ring or aromatic ring system. Aromatic ring or aromatic ring system refers to a planar ring or ring system of covalently bound carbon atoms, wherein the planar ring or ring system comprises a conjugated system of delocalized electrons fulfilling Hückel's rule. Examples of aryl groups include monocyclic groups like phenyl or tolyl, polycyclic groups which comprise more aromatic rings linked by single bonds, like biphenyl, and polycyclic groups comprising fused rings, like naphthyl or fluorenyl. Preferably the term "substituted or unsubstituted aryl" or "substituted or unsubstituted $C_6$ to $C_{24}$ aryl" are includes aryl compounds of "fused aryl rings" or "condensed aryl rings".

Analogously, under heteroaryl, it is especially where suitable understood a group derived by formal abstraction of one ring hydrogen from a heterocyclic aromatic ring in a compound comprising at least one such ring.

Further the term "substituted or unsubstituted heteroaryl" or "substituted or unsubstituted $C_2$ to $C_{24}$ heteroaryl" are includes heteroaryl compounds of "fused heteroaryl rings" or "condensed aryl rings" or "fused heteroaryl rings comprising at least one non-heteroaryl ring" or "condensed aryl rings comprising at least one non-heteroaryl ring".

The term "fused aryl rings" or "condensed aryl rings" is understood the way that two aryl rings are considered fused or condensed when they share at least two common $sp^2$-hybridized carbon atoms.

Correspondingly, in the present specification "heteroaryl substituted" refers to a substitution with one or more heteroaryl groups, which themselves may be substituted with one or more aryl and/or heteroaryl groups.

In the present specification, when a definition is not otherwise provided, an "alkyl group" refers to a saturated aliphatic hydrocarbyl group. The alkyl group may be a $C_1$ to $C_{12}$ alkyl group or $C_1$ to $C_8$ alkyl group. More specifically, the alkyl group may be a $C_1$ to $C_{10}$ alkyl group or a $C_1$ to $C_6$ alkyl group. For example, a $C_1$ to $C_4$ alkyl group includes 1 to 4 carbons in alkyl chain, and may be selected from methyl, ethyl, propyl, iso-propyl, n-butyl, iso-butyl, sec-butyl, and tert-butyl.

Specific examples of the alkyl group may be a methyl group, an ethyl group, a propyl group, an iso-propyl

group, a butyl group, an iso-butyl group, a tert-butyl group, a pentyl group, a hexyl group.

The term "cycloalkyl" refers to saturated hydrocarbyl groups derived from a cycloalkane by formal abstraction of one hydrogen atom from a ring atom comprised in the corresponding cycloalkane. Examples of the cycloalkyl group may be a cyclopropyl group, a cyclobutyl group, a cyclopentyl group, a cyclohexyl group, a methyl cyclohexyl group, an adamantly group and the like.

The term "hetero" is understood the way that at least one carbon atom, in a structure which may be formed by covalently bound carbon atoms, is replaced by another polyvalent atom. Preferably, the heteroatoms are selected from B, Si, N, P, O, S; more preferably from N, P, O, S, and most preferred N.

The term "fused ring system" is understood to mean a ring system wherein two or more rings share at least two atoms.

The term "5-, 6- or 7-member ring" is understood to mean a ring comprising 5, 6 or 7 atoms. The atoms may be selected from C and one or more hetero-atoms.

In the present specification, the single bond refers to a direct bond.

In the context, "different" means that the compounds do not have an identical chemical structure.

The term "free of", "does not contain", "does not comprise" does not exclude impurities which may be present in the compounds prior to deposition. Impurities have no technical effect with respect to the object achieved by the present invention.

The term "contacts" or "contacting" refers to an arrangement of at least two layers whereby a first layer is in direct contact with an adjacent second layer.

The term "photoactive layer" may comprise "light-absorbing layer" and "light absorption layer" are used synonymously.

The term "photoactive layer" may comprise layer , preferably "light-emitting layer", "light emitting layer" and "light emitting layer".

The terms "OLED" and "organic light-emitting diode" are used synonymously.

The terms anode and anode electrode are used synonymously. The term "at least two anode sub-layers" is understood to mean two or more anode sub-layers, for example two or three anode sub-layers.

The terms cathode and cathode electrode are used synonymously.

The term "hole transport layer" is understood to mean a layer which transports holes between the hole injection layer and further layers arranged between the hole injection layer and the cathode layer.

The operating voltage U is measured in Volt.

In the context of the present specification the term "essentially non-emissive" or "non-emissive" means that the contribution of metal compound, the matrix compound or metal complex and/or layer, wherein the layer is a hole injection layer, hole transport layer, anode layer and cathode layer, to the visible emission spectrum from an organic electronic device, , preferably OLED or display device, is less than 10 %, preferably less than 5 % relative to the visible emission spectrum. The visible emission spectrum is an emission spectrum with a wavelength of about ≥ 380 nm to about ≤ 780 nm.

In the specification, hole characteristics refer to an ability to donate an electron to form a hole when an electric field is applied and that a hole formed in the anode may be easily injected into the light emitting layer and transported in the light emitting layer due to conductive characteristics according to a highest occupied molecular orbital (HOMO) level.

In addition, electron characteristics refer to an ability to accept an electron when an electric field is applied and that electrons formed in the cathode may be easily injected into the light emitting layer and transported in the light emitting layer due to conductive characteristics according to a lowest unoccupied molecular orbital (LUMO) level.

The term "HOMO level" is understood to mean the highest occupied molecular orbital and is determined in eV (electron volt).

The term "HOMO level further away from vacuum level" is understood to mean that the absolute value of the HOMO level is higher than the absolute value of the HOMO level of the reference compound. For example, the term "further away from vacuum level than the HOMO level of N2,N2,N2',N2',N7,N7,N7',N7'-octakis(4-methoxyphenyl)-9,9'-spirobi[fluorene]-2,2',7,7'-tetraamine is understood to mean that the absolute value of the HOMO level of the covalent matrix compound or substantially covalent matrix compound of the hole injection layer is higher than the HOMO level of N2,N2,N2',N2', N7,N7, N7',N7'-octakis(4-methoxyphenyl)-9,9'-spirobi[fluorene]-2,2',7, 7'-tetraamine.

The term "absolute value" is understood to mean the value without the "-" symbol. According to one embodiment, wherein the HOMO level of the covalent matrix compound or substantially covalent matrix compound, preferably the matrix compound of the hole injection layer may be calculated by quantum mechanical methods.

The work function of the first metal is measured in eV (electron volt). Tabulated values of work functions can be found for example in CRC Handbook of Chemistry and Physics version 2008, p. 12-114. Further, tabulated values of work functions can be found for example at https://en.wikipedia.org/wiki/Work_function#cite_note-12.

In the specification, hole characteristics refer to an ability to donate an electron to form a hole when an electric field is applied and that a hole formed in the anode may be easily injected into the light emitting layer and transported

in the light emitting layer due to conductive characteristics according to a highest occupied molecular orbital (HOMO) level.

In addition, electron characteristics refer to an ability to accept an electron when an electric field is applied and that electrons formed in the cathode may be easily injected into the light emitting layer and transported in the light emitting layer due to conductive characteristics according to a lowest unoccupied molecular orbital (LUMO) level.

The different embodiments described herein may separately or in combination of two or more embodiments be material for realizing the invention.

Advantageous Effects

Surprisingly, it was found that the organic electroluminescent device according to the invention solves the problem underlying the present invention by enabling organic electroluminescent devices, preferably organic light-emitting diodes LEDs, in various aspects superior over the organic electroluminescent devices known in the art, in particular with respect to the reduction of the increase of operating voltage.

Additionally, it was found that the problem underlying the present invention can be solved by providing compounds which may be suitable for deposition through vacuum thermal evaporation under conditions suitable for mass production. In particular, the rate onset temperature of the metal complex and the matrix compound may be in a range suitable for mass production.

In addition, it was found that the metal compounds as described herein have improved thermal properties and that organic electronic devices containing the same as a semiconductor layer, preferably as hole injection layer or hole transport layer, have improved performance in particular current efficiency, stability and life time.

**Coordinating linker X**

m and n

According to one embodiment, wherein m and n in the coordinating linker X of formula (IIa) or (IIb) is $m+n \geq 1$.

According to one embodiment, wherein m and n in the coordinating linker X of formula (IIa) or (IIb) are selected from 1. According to one embodiment, wherein $m = 1$ and $n = 0$ in the coordinating linker X of formula (IIa) or (IIb). According to one embodiment, wherein $m = 0$ and $n = 1$ in the coordinating linker X of formula (IIa) or (IIb). According to one embodiment, wherein m and n in the coordinating linker X of formula (IIa) or (IIb) are selected from 0. According to another embodiment, wherein m and n in the coordinating linker X of formula (IIa) or (IIb) are selected $m+n \geq 1$.

Z

According to one embodiment, wherein Z is selected from the group comprising $CR^1$, O, or N. According to one embodiment, wherein Z is selected from the group comprising $CR^1$ or N. According to one embodiment, wherein Z is selected from the group comprising $CR^1$ or O. According to one embodiment, wherein Z is selected $CR^1$. According to one embodiment, wherein Z is selected O. According to one embodiment, wherein Z is selected N.

$A^1$ and $A^2$

According to one embodiment, wherein $A^1$ and $A^2$ are independently selected from the group comprising C=O, C-O, $C=NR^2$, $C-NR^3$, SO, $SO_2$, or $P(=O)R^4$. According to one embodiment, wherein $A^1$ and $A^2$ are independently selected from the group comprising C=O, CO. According to one embodiment, wherein $A^1$ and $A^2$ are independently selected from the group comprising $C=NR^2$, $C-NR^3$. According to one embodiment, wherein $A^1$ and $A^2$ are independently selected from the group comprising SO or $SO_2$. According to one embodiment, wherein $A^1$ and $A^2$ are independently selected from the group comprising C=O, C-O, $C=NR^2$, $C-NR^3$, $SO_2$, or $P(=O)R^4$. According to one embodiment, wherein $A^1$ and $A^2$ are independently selected from the group comprising C=O, C-O, $C=NR^2$, $C-NR^3$, $SO_2$, or $P(=O)R^4$.

According to one embodiment, wherein $A^1$ and $A^2$ together form a cycle with Z.

$R^1$. $R^2$, $R^3$ and $R^4$

According to one embodiment, wherein $R^1$, $R^2$, $R^3$ and $R^4$ are independently selected from H, D, CN, substituted or unsubstituted $C_6$ to $C_{24}$ aryl, substituted or unsubstituted $C_2$ to $C_{24}$ heteroaryl, substituted or unsubstituted $C_1$ to $C_{24}$ alkyl, substituted or unsubstituted $C_3$ to $C_{24}$ carbocyclyl, or substituted or unsubstituted $C_2$ to $C_{24}$ heterocyclyl, ligand fragment Y. According to one embodiment, wherein $R^1$, $R^2$, $R^3$ and $R^4$ are independently selected from H, D, CN, substituted or unsubstituted $C_6$ to $C_{18}$ aryl, substituted or unsubstituted $C_2$ to $C_{18}$ heteroaryl, ligand fragment Y. According to one embodiment, wherein $R^1$, $R^2$, $R^3$ and $R^4$ are independently selected from H, D, CN, substituted or unsubstituted

$C_6$ to $C_{18}$ aryl, substituted or unsubstituted $C_2$ to $C_{18}$ heteroaryl. According to one embodiment, wherein $R^1$, $R^2$, $R^3$ and $R^4$ are independently selected from H, D, CN, substituted or unsubstituted $C_6$ to $C_{12}$ aryl, substituted or unsubstituted $C_2$ to $C_{128}$ heteroaryl, ligand fragment Y. According to one embodiment, wherein $R^1$, $R^2$, $R^3$ and $R^4$ are independently selected from H, D, CN, substituted or unsubstituted $C_6$ to $C_{12}$ aryl, substituted or unsubstituted $C_2$ to $C_{12}$ heteroaryl.

According to one embodiment, wherein the substituents on $R^1$, $R^2$, $R^3$ and $R^4$ are independently selected from substituted or unsubstituted $C_6$ to $C_{24}$ aryl, substituted or unsubstituted $C_2$ to $C_{24}$ heteroaryl, substituted or unsubstituted $C_1$ to $C_{24}$ alkyl, substituted or unsubstituted $C_3$ to $C_{24}$ carbocyclyl, substituted or unsubstituted $C_2$ to $C_{24}$ heterocyclyl, perfluorinated $C_1$ to $C_4$ alkyl, substituted $C_6$ to $C_{24}$ aryl in 3-position, substituted $C_6$ to $C_{24}$ aryl in 4-position, substituted 4-pyridyl, or substituted 5-pyrimidyl, and more preferred the at least one ligand fragment Y is independently selected from substituted or unsubstituted $C_6$ to $C_{24}$ aryl, substituted or unsubstituted $C_2$ to $C_{24}$ heteroaryl, wherein the substituents are selected from the group comprising an electron-withdrawing group, halogen, F, Cl, and/or CN.

According to one embodiment, wherein the substituents on $R^1$, $R^2$, $R^3$ and $R^4$ are independently selected from substituted or unsubstituted $C_6$ to $C_{18}$ aryl, substituted or unsubstituted $C_2$ to $C_{18}$ heteroaryl, substituted or unsubstituted $C_1$ to $C_{18}$ alkyl, substituted or unsubstituted $C_3$ to $C_{18}$ carbocyclyl, substituted or unsubstituted $C_2$ to $C_{18}$ heterocyclyl, perfluorinated $C_1$ to $C_4$ alkyl, substituted $C_6$ to $C_{18}$ aryl in 3-position, substituted $C_6$ to $C_{18}$ aryl in 4-position, substituted 4-pyridyl, or substituted 5-pyrimidyl, and more preferred the at least one ligand fragment Y is independently selected from substituted or unsubstituted $C_6$ to $C_{18}$ aryl, substituted or unsubstituted $C_2$ to $C_{18}$ heteroaryl, wherein the substituents are selected from the group comprising an electron-withdrawing group, halogen, F, Cl, and/or CN.

According to one embodiment, wherein the substituents on $R^1$, $R^2$, $R^3$ and $R^4$ are independently selected from substituted or unsubstituted $C_6$ to $C_{18}$ aryl, substituted or unsubstituted $C_2$ to $C_{18}$ heteroaryl, substituted $C_6$ to $C_{18}$ aryl in 3-position, substituted $C_6$ to $C_{18}$ aryl in 4-position, substituted 4-pyridyl, or substituted 5-pyrimidyl, and more preferred the at least one ligand fragment Y is independently selected from substituted or unsubstituted $C_6$ to $C_{18}$ aryl, substituted or unsubstituted $C_2$ to $C_{18}$ heteroaryl, wherein the substituents are selected from the group comprising an electron-withdrawing group, halogen, F, Cl, and/or CN.

According to one embodiment, wherein the substituents on $R^1$, $R^2$, $R^3$ and $R^4$ are independently selected substituted or unsubstituted $C_1$ to $C_{18}$ alkyl, substituted or unsubstituted $C_3$ to $C_{18}$ carbocyclyl, substituted or unsubstituted $C_2$ to $C_{18}$ heterocyclyl, perfluorinated $C_1$ to $C_4$ alkyl, substituted $C_6$ to $C_{18}$ aryl in 4-position, substituted 4-pyridyl, or substituted 5-pyrimidyl, and more preferred the at least one ligand fragment Y is independently selected from substituted or unsubstituted $C_6$ to $C_{18}$ aryl, substituted or unsubstituted $C_2$ to $C_{18}$ heteroaryl, wherein the substituents are selected from the group comprising an electron-withdrawing group, halogen, F, Cl, and/or CN.

According to one embodiment, wherein the substituents on $R^1$, $R^2$, $R^3$ and $R^4$ are independently selected from substituted $C_6$ to $C_{18}$ aryl in 3-position, substituted $C_6$ to $C_{18}$ aryl in 4-position, substituted 4-pyridyl, or substituted 5-pyrimidyl, and more preferred the at least one ligand fragment Y is independently selected from substituted or unsubstituted $C_6$ to $C_{18}$ aryl, substituted or unsubstituted $C_2$ to $C_{18}$ heteroaryl, wherein the substituents are selected from the group comprising an electron-withdrawing group, halogen, F, Cl, and/or CN.

According to one embodiment, wherein the coordinating linker X may be selected from formula (IIa):

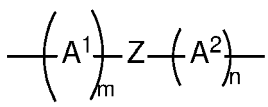

(IIa),

wherein

| | |
|---|---|
| m and n | are independently selected from 0 or 1; |
| Z | is selected from the group comprising $CR^1$, O, N; and |
| $A^1$ and $A^2$ | are independently selected from the group comprising C=O, C-O, SO or $SO_2$; |
| $R^1$ | is independently selected from H, D, substituted or unsubstituted $C_6$ to $C_{24}$ aryl, substituted or unsubstituted $C_2$ to $C_{24}$ heteroaryl, substituted or unsubstituted $C_1$ to $C_{24}$ alkyl, ligand fragment Y; |

wherein $A^1$ and $A^2$ together optional form a cycle with Z.

According to another embodiment, wherein the coordinating linker X may be selected from formula (IIa):

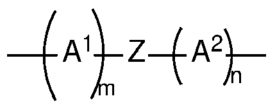

(IIa),

wherein m and n are selected 1;

Z is selected from the group comprising $CR^1$, N; and

$A^1$ and $A^2$ are independently selected from the group comprising C=O, C-O or $SO_2$;

$R^1$ is independently selected from H, D, substituted or unsubstituted $C_6$ to $C_{24}$ aryl, substituted or unsubstituted $C_2$ to $C_{24}$ heteroaryl, substituted or unsubstituted $C_1$ to $C_{24}$ alkyl, ligand fragment Y;

wherein $A^1$ and $A^2$ together optional form a cycle with Z.

Ligand fragment Y

According to one embodiment, wherein the ligand fragment Y is the at least one ligand fragment Y is independently selected from substituted or unsubstituted $C_6$ to $C_{24}$ aryl, substituted or unsubstituted $C_2$ to $C_{24}$ heteroaryl, substituted or unsubstituted $C_1$ to $C_{24}$ alkyl, substituted or unsubstituted $C_3$ to $C_{24}$ carbocyclyl, substituted or unsubstituted $C_2$ to $C_{24}$ heterocyclyl, perfluorinated $C_1$ to $C_4$ alkyl, substituted $C_6$ to $C_{24}$ aryl in 3-position, substituted $C_6$ to $C_{24}$ aryl in 4-position, substituted 4-pyridyl, or substituted 5-pyrimidyl, wherein the at least one ligand fragment Y has a directed dipole moment of less than -3.6 debye, and wherein the at least one ligand L is negatively charged.

According to one embodiment, wherein the ligand fragment Y is a substituted $C_6$ to $C_{24}$ aryl or substituted $C_2$ to $C_{24}$ heteroaryl, perfluorinated $C_1$ to $C_4$ alkyl, substituted $C_6$ to $C_{24}$ aryl in 3-position, substituted $C_6$ to $C_{24}$ aryl in 4-position, substituted 4-pyridyl, or substituted 5-pyrimidyl, and more preferred substituted $C_6$ to $C_{24}$ aryl in 4-position, wherein the substituents are selected from the group comprising halogen, F, Cl, and/or CN.

According to one embodiment, wherein the ligand fragment Y is a substituted $C_6$ to $C_{18}$ aryl or substituted $C_2$ to $C_{18}$ heteroaryl, perfluorinated $C_1$ to $C_4$ alkyl, substituted $C_6$ to $C_{18}$ aryl in 3-position, substituted $C_6$ to $C_{18}$ aryl in 4-position, substituted 4-pyridyl, or substituted 5-pyrimidyl, and more preferred substituted $C_6$ to $C_{18}$ aryl in 4-position, wherein the substituents are selected from the group comprising halogen, F, Cl, and/or CN.

According to one embodiment, wherein the ligand fragment Y is a substituted $C_6$ to $C_{12}$ aryl or substituted $C_2$ to $C_{12}$ heteroaryl, perfluorinated $C_1$ to $C_4$ alkyl, substituted $C_6$ to $C_{18}$ aryl in 3-position, substituted $C_6$ to $C_{12}$ aryl in 4-position, substituted 4-pyridyl, or substituted 5-pyrimidyl, and more preferred substituted $C_6$ to $C_{12}$ aryl in 4-position, wherein the substituents are selected from the group comprising halogen, F, Cl, and/or CN.

According to one embodiment, wherein the substituents on ligand fragment Y is an electron-withdrawing group, F, Cl, CN, SCN, -OCF3, $NO_2$, $SF_5$ substituted or unsubstituted $C_6$ to $C_{24}$ aryl, substituted or unsubstituted $C_2$ to $C_{24}$ heteroaryl, substituted or unsubstituted $C_1$ to $C_{24}$ alkyl, substituted or unsubstituted $C_3$ to $C_{24}$ carbocyclyl, or substituted or unsubstituted $C_2$ to $C_{24}$ heterocyclyl;
wherein the substituent is an electron-withdrawing group such F, Cl, CN.

According to one embodiment, wherein the substituents on ligand fragment Y is an electron-withdrawing group, F, Cl, CN, SCN, $-OCF_3$, $NO_2$, $SF_5$, perfluorinated $C_1$ to $C_4$ alkyl, $CF_3$, €substituted aryl in 3-position and 4-position most preferably in 4-position, substituted 4-pyridyl, and substituted 5-pyrimidyl.

According to one embodiment, wherein the substituents on ligand fragment Y is a F, Cl, CN, perfluorinated $C_1$ to $C_4$ alkyl, $CF_3$, substituted aryl in 3-position and 4-position most preferably in 4-position, substituted 4-pyridyl, and substituted 5-pyrimidyl.

Directed dipole moment of Y

According to one embodiment, wherein the ligand fragment Y has a directed dipole moment of less than -3.6 debye, and wherein the at least one ligand L is negatively charged. According to one embodiment, wherein the ligand fragment Y has a directed dipole moment selected from the group of ≤ -4.0 debye, ≤ -4.2 debye, ≤ -4.4 debye, ≤ -4.6 debye, ≤ -4.8 debye, ≤ -5.0 debye, ≤ -5.1 debye, ≤ -5.2 debye, ≤ -5.3 debye, ≤ -5.4 debye, ≤ -5.5 debye, ≤ -5.6 debye, ≤ -5.7 debye, or ≤ - 5.8 debye, more preferred the ligand fragment Y has a directed dipole moment less than -5.5 debye. According to one embodiment, wherein the ligand fragment Y has a directed dipole moment selected from the group of ≤ -4.0 debye, ≤ -4.2 debye, ≤ -4.4 debye, ≤ - 4.6 debye, ≤ -4.8 debye, ≤ -5.0 debye, ≤ -5.1 debye, ≤ -5.2 debye, ≤ -5.3 debye, ≤ -5.4 debye, ≤ -5.5 debye, ≤ -5.6 debye, ≤ -5.7 debye, or ≤ - 5.8 debye, more preferred the ligand fragment Y has a directed dipole moment less than -5.5 debye, and wherein the at least one ligand L is negatively charged.

Fragment Y is represented by formula (IV)

According to one embodiment, wherein the ligand fragment Y is represented by formula (IV)

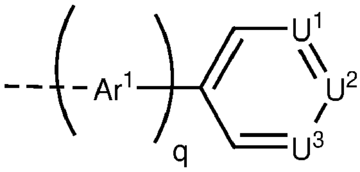

(IV),

wherein q is selected from 0, 1, 2, and preferably 0 or 1;

$Ar^1$ is selected from substituted or unsubstituted phenyl, substituted or unsubstituted naphthyl, or substituted or unsubstituted anthracenyl, preferably $Ar^1$ is phenyl;

$U^1$ is selected from N or CR';

$U^2$ is selected from N or CR";

$U^3$ is selected from N or CR"'; and wherein R', R" and R'" are independently selected from H, D, electron-withdrawing group, preferably F, perfluorinated $C_1$ to $C_{24}$ alkyl, preferably $CF_3$, CN, SCN, $OCF_3$, $-NO_2$, $-SF_5$.

According to one embodiment, wherein the ligand fragment Y is represented by formula (IV)

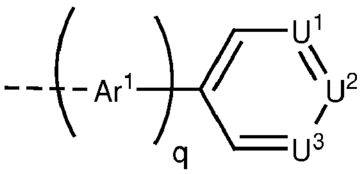

(IV),

wherein q is selected from 0, 1, 2, and preferably 0 or 1;

$Ar^1$ is selected from substituted or unsubstituted phenyl, substituted or unsubstituted naphthyl, or substituted or unsubstituted anthracenyl, preferably $Ar^1$ is phenyl;

$U^1$ is selected from N or CR';

$U^2$ is selected from N or CR";

$U^3$ is selected from N or CR"'; and wherein R', R" and R'" are independently selected from H, D, electron-withdrawing group, preferably F, perfluorinated $C_1$ to $C_{24}$ alkyl, preferably $CF_3$, CN, SCN, $OCF_3$, $-NO_2$, $-SF_5$; and

the at least one ligand fragment Y has a directed dipole moment of less than -3.6 debye, and wherein the at least one ligand L is negatively charged.

According to one embodiment, wherein the ligand fragment Y is represented by formula (IV)

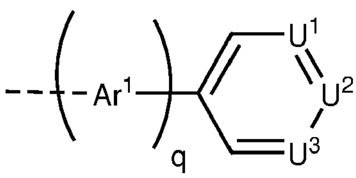

(IV),

wherein

q is selected from 0, 1, 2, and preferably 0 or 1;
$Ar^1$ is selected from substituted or unsubstituted phenyl, substituted or unsubstituted naphthyl, or substituted or unsubstituted anthracenyl, preferably $Ar^1$ is phenyl;
$U^1$ is selected from N or CR';

$U^2$ is selected from N or CR";
$U^3$ is selected from N or CR"'; and

wherein R', R" and R"' are independently selected from H, D, electron-withdrawing group, preferably F, perfluorinated $C_1$ to $C_{24}$ alkyl, preferably $CF_3$, CN, SCN, $OCF_3$, $-NO_2$, $-SF_5$; and
wherein the at least one ligand fragment Y has a directed dipole moment of less than -3.6 debye, and wherein the at least one ligand L is negatively charged.

According to one embodiment, wherein the ligand fragment Y is represented by formula (IV)

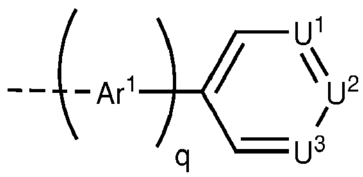

(IV),

wherein

| | |
|---|---|
| q | is selected from 0, 1, 2, and preferably 0 or 1; |
| $Ar^1$ | is selected from substituted or unsubstituted phenyl, substituted or unsubstituted naphthyl, or substituted or unsubstituted anthracenyl, preferably $Ar^1$ is substituted or unsubstituted phenyl, wherein the substituents are selected from the group comprising an electron-withdrawing group, preferably halogen, F, Cl, perfluorinated $C_1$ to $C_{24}$ alkyl, preferably $CF_3$, CN, SCN, $OCF_3$, $-NO_2$, $-SF_5$, halogen, F, Cl, and/or CN, preferably selected from the group comprising halogen, F, Cl, and/or CN; |
| $U^1$ | is selected from N or CR'; |
| $U^2$ | is selected from N or CR"; |
| $U^3$ | is selected from N or CR"'; and |
| wherein R', R" and R"' | are independently selected from H, D, electron-withdrawing group, preferably F, perfluorinated $C_1$ to $C_{24}$ alkyl, preferably $CF_3$, CN, SCN, $OCF_3$, $-NO_2$, $-SF_5$; and |

wherein the at least one ligand fragment Y has a directed dipole moment of less than -3.6 debye, and wherein the at least one ligand L is negatively charged.

According to one embodiment, wherein the ligand fragment Y is represented by formula (IV)

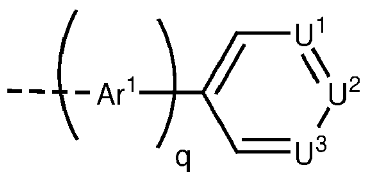

(IV),

wherein

q is selected from 0, 1, 2, and preferably 0 or 1;
$Ar^1$ is selected from substituted or unsubstituted phenyl, substituted or unsubstituted naphthyl, or substituted or unsubstituted anthracenyl, preferably $Ar^1$ is phenyl, wherein the substituents on $Ar^1$ are selected from the group comprising halogen, F, Cl, and/or CN
$U^1$ is selected from N or CR';
$U^2$ is selected from N or CR";
$U^3$ is selected from N or CR"'; and

wherein R', R" and R"' are independently selected from H, D, electron-withdrawing group, preferably F, perfluorinated $C_1$ to $C_{24}$ alkyl, preferably $CF_3$, CN, SCN, $OCF_3$, $-NO_2$, $-SF_5$; and wherein the at least one ligand fragment Y has a directed dipole moment of less than -3.6 debye, and wherein the at least one ligand L is negatively charged.

According to one embodiment, wherein the ligand fragment Y is represented by formula (IV):

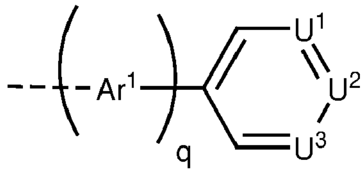 (IV),

wherein

q is selected from 0, 1, 2, and preferably 0 or 1;
$Ar^1$ is selected from unsubstituted phenyl, unsubstituted naphthyl, or unsubstituted anthracenyl, preferably $Ar^1$ is phenyl;
$U^1$ is selected from N or CR';
$U^2$ is selected from N or CR";
$U^3$ is selected from N or CR"'; and wherein R', R" and R"' are independently selected from H, D, electron-withdrawing group, preferably F, perfluorinated $C_1$ to $C_{24}$ alkyl, preferably $CF_3$, CN, SCN, $OCF_3$, $-NO_2$, $-SF_5$.

According to one embodiment, wherein the ligand fragment Y is represented by formula (IV):

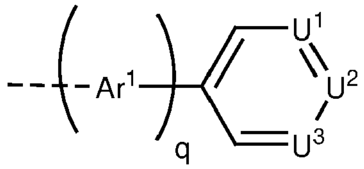 (IV),

wherein

q is selected from 0, 1, 2, and preferably 0 or 1;
$Ar^1$ is selected from unsubstituted phenyl, unsubstituted naphthyl, or unsubstituted anthracenyl, preferably $Ar^1$ is phenyl;
$U^1$ is selected from N or CR';
$U^2$ is selected from N or CR";
$U^3$ is selected from N or CR"'; and

wherein R', R" and R"' are independently selected from H, D, electron-withdrawing group, preferably F, perfluorinated $C_1$ to $C_{24}$ alkyl, preferably $CF_3$, CN, SCN, $OCF_3$, $-NO_2$, $-SF_5$; and wherein the at least one ligand fragment Y has a directed dipole moment of less than -3.6 debye, and wherein the at least one ligand L is negatively charged.

According to one embodiment, wherein the ligand fragment Y is selected from a group D1 to D11:

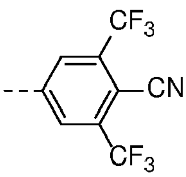 (D1), 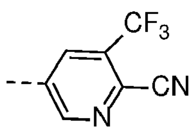 (D2), 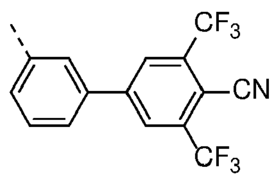 (D3),

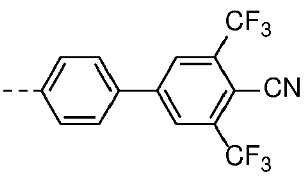 (D4), 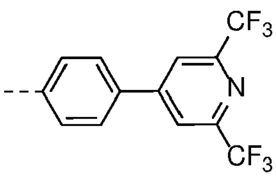 (D5), 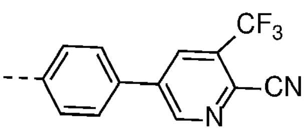 (D6),

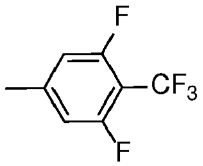 (D7), 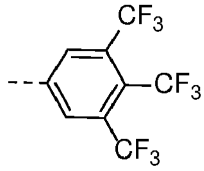 (D8), 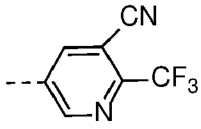 (D9),

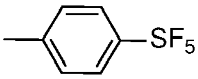 (D10), 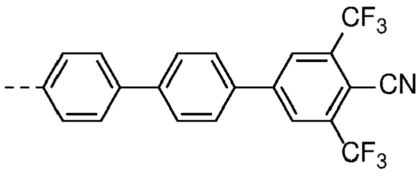 (D11);

wherein more preferred the ligand fragment Y is selected from the group of D1 to D6, D1 to D4 or D5 and D6.

Metal

According to one embodiment, wherein the valency p of the metal M can be 1, 2, 3 or 4.

According to one embodiment, wherein the metal M has an atomic mass of ≥ 22Da, alternatively ≥ 24Da.

According to one embodiment, wherein the metal M may be selected from a metal ion wherein the corresponding metal has an electronegativity value according to Allen of less than 2.4, preferably less than 2, more preferred less than 1.9. Thereby, particularly good performance in organic electronic devices may be achievable.

The term "electronegativity according to Allen" especially refers to Allen, Leland C. (1989). "Electronegativity is the average one-electron energy of the valence-shell electrons in ground-state free atoms". Journal of the American Chemical Society. 111 (25): 9003-9014.

According to one embodiment the valency p of M is 1 or 2.

According to one embodiment, wherein the metal M is selected from a metal ion wherein the corresponding metal has an electronegativity value according to Allen of less than 2.4, preferably less than 2, more preferred less than 1.9, and the valency p of the metal M is 1 or 2.

According to one embodiment, wherein the metal M is selected from an alkali, alkaline earth, rare earth or transition metal, alternatively M is selected from alkali, alkaline earth, or a period 4 or 5 transition metal.

According to one embodiment, wherein the metal M is selected from a metal ion wherein the corresponding metal has an electronegativity value according to Allen of less than 2.4, preferably less than 2, more preferred less than 1.9 and M is selected from alkali, alkaline earth, rare earth or a period 4 or 5 transition metal and the metal M has an atomic mass of ≥ 22Da, alternatively ≥ 24Da.

According to one embodiment, wherein the metal M is selected from the group comprising Li, Na, K, Cs, Mg, Ca, Sr, Ba, Al, In, Bi, Sc, Ti, Zr, Hf, V, Cr, Mo, W, Mn, Re, Fe, Ru, Co Rh, Ir, Ni, Pd, Pt, Cu, Ag, Au, Zn, La, Ce, Eu, or Yb, preferably the metal is selected from the group comprising Al, Bi, Cr, Mn, Fe, Co, Ni, Cu, Zn, Mo, Ag, Re, Au or Ce, and more preferred the metal is selected from the group comprising Al, Mn, Fe, Cu, Mo, Ag, Au or Ce.

According to one embodiment, wherein the metal M is selected from an alkaline earth metal, transition metal or rare earth metal, preferably Cu(II), Ag(I), Zn(II), Fe(II), Fe(III), Ce(IV) and more preferred Fe(II).

According to one embodiment, wherein the metal M is selected from Li, Na, K, Cs, Mg, Mn, Cu, Zn, Ag and Mo; preferably M is selected from Na, K, Cs, Mg, Mn, Cu, Zn and Ag; also preferred M is selected from Na, K, Mg, Mn, Cu, Zn and Ag, wherein if the metal M is Cu, the valency p is 2.

Coordinating linker X of formula (IIb)

According to one embodiment, wherein the at least one coordinating linker X is represented by formula (IIb): $R^a\{A^1\}_mZ\{A^2\}_nR^b$ (IIb), wherein

Z is selected from $CR^1$, N;

$A^1$ and $A^2$ are independently selected from C=O, C-O, or $SO_2$, and wherein $A^1$ and $A^2$ together optional form a cycle with Z;

$R^1$ is selected from H, D, CN, substituted or unsubstituted $C_6$ to $C_{24}$ aryl, substituted or unsubstituted $C_2$ to $C_{24}$ heteroaryl, substituted or unsubstituted $C_1$ to $C_{24}$ alkyl, substituted or unsubstituted $C_3$ to $C_{24}$ carbo-

cyclyl, or substituted or unsubstituted $C_2$ to $C_{24}$ heterocyclyl, ligand fragment Y;

$R^a$ and $R^b$ are independently selected from substituted or unsubstituted $C_6$ to $C_{24}$ aryl, substituted or unsubstituted $C_2$ to $C_{24}$ heteroaryl, substituted or unsubstituted $C_1$ to $C_{24}$ alkyl, substituted or unsubstituted $C_3$ to $C_{24}$ carbocyclyl, or substituted or unsubstituted $C_2$ to $C_{24}$ heterocyclyl, ligand fragment Y, wherein the substituents are selected from the group comprising an electron-withdrawing group, halogen, F, Cl, CN, partially fluorinated or perfluorinated $C_1$ to $C_8$ alkyl, partially fluorinated or perfluorinated $C_6$ to $C_{12}$ aryl, or partially fluorinated or perfluorinated $C_2$ to $C_{12}$ heteroaryl;

wherein at least one of $R^1$, $R^a$ and $R^b$ contains a ligand fragment Y;
wherein at least one of the at least one ligand fragment Y is independently selected from substituted $C_6$ to $C_{24}$ aryl or unsubstituted or substituted $C_2$ to $C_{24}$ heteroaryl, and wherein the at least one of the at least one ligand fragment Y has a directed dipole moment of less than -3.6 debye, and wherein the at least one ligand L is negatively charged.

According to one embodiment, wherein the at least one ligand L is represented by formula (IIb):

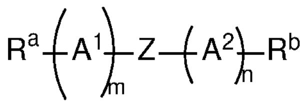

(IIb),

wherein

Z is selected from $CR^1$, N;

$A^1$ and $A^2$ are independently selected from C=O, C-O, or $SO_2$, and wherein $A^1$ and $A^2$ together optional form a cycle with Z;

$R^1$ is selected from H, D, CN, substituted or unsubstituted $C_6$ to $C_{24}$ aryl, substituted or unsubstituted $C_2$ to $C_{24}$ heteroaryl, substituted or unsubstituted $C_1$ to $C_{24}$ alkyl, substituted or unsubstituted $C_3$ to $C_{24}$ carbocyclyl, or substituted or unsubstituted $C_2$ to $C_{24}$ heterocyclyl, ligand fragment Y, wherein the substituents are selected from the group comprising an electron-withdrawing group, halogen, F, Cl, CN, partially fluorinated or perfluorinated $C_1$ to $C_8$ alkyl, partially fluorinated or perfluorinated $C_6$ to $C_{12}$ aryl, or partially fluorinated or perfluorinated $C_2$ to $C_{12}$ heteroaryl;

$R^a$ and $R^b$ are independently selected from substituted or unsubstituted $C_6$ to $C_{24}$ aryl, substituted or unsubstituted $C_2$ to $C_{24}$ heteroaryl, substituted or unsubstituted $C_1$ to $C_{24}$ alkyl, substituted or unsubstituted $C_3$ to $C_{24}$ carbocyclyl, or substituted or unsubstituted $C_2$ to $C_{24}$ heterocyclyl, ligand fragment Y, wherein the substituents are selected from the group comprising an electron-withdrawing group, halogen, F, Cl, CN, partially fluorinated or perfluorinated $C_1$ to $C_8$ alkyl, partially fluorinated or perfluorinated $C_6$ to $C_{12}$ aryl, or partially fluorinated or perfluorinated $C_2$ to $C_{12}$ heteroaryl;

wherein at least one of $R^1$, $R^a$ and $R^b$ contains a ligand fragment Y;
wherein at least one of the at least one ligand fragment Y is independently selected from substituted $C_6$ to $C_{24}$ aryl or unsubstituted or substituted $C_2$ to $C_{24}$ heteroaryl, and wherein the at least one of the at least one ligand fragment Y has a directed dipole moment of less than -3.6 debye, and wherein the at least one ligand L is negatively charged.

According to one embodiment, wherein $R^a$ and $R^b$ are independently selected from substituted or unsubstituted $C_6$ to $C_{24}$ aryl, substituted or unsubstituted $C_2$ to $C_{24}$ heteroaryl, substituted or unsubstituted $C_1$ to $C_{24}$ alkyl, ligand fragment Y.

According to one embodiment, wherein the at least one ligand L is represented by formula(III):

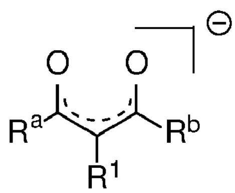

(III),

wherein

$R^1$ is selected from H, D, CN, substituted or unsubstituted $C_6$ to $C_{24}$ aryl, substituted or unsubstituted $C_2$ to $C_{24}$ heteroaryl, substituted or unsubstituted $C_1$ to $C_{24}$ alkyl, substituted or unsubstituted $C_3$ to $C_{24}$ carbocyclyl, or substituted or unsubstituted $C_2$ to $C_{24}$ heterocyclyl, ligand fragment Y;

$R^a$ and $R^b$ are independently selected from substituted or unsubstituted $C_6$ to $C_{24}$ aryl, substituted or unsubstituted $C_2$ to $C_{24}$ heteroaryl, substituted or unsubstituted $C_1$ to $C_{24}$ alkyl, substituted or unsubstituted $C_3$ to $C_{24}$ carbocyclyl, or substituted or unsubstituted $C_2$ to $C_{24}$ heterocyclyl, ligand fragment Y, wherein the substituents are selected from the group comprising an electron-withdrawing group, halogen, F, Cl, CN, partially fluorinated or perfluorinated $C_1$ to $C_8$ alkyl, perfluorinated $C_6$ to $C_{12}$ aryl, or partially fluorinated or perfluorinated $C_2$ to $C_{12}$ heteroaryl;

wherein at least one of $R^1$, $R^a$ and $R^b$ contains a ligand fragment Y;
wherein at least one of the at least one ligand fragment Y is independently selected from substituted $C_6$ to $C_{24}$ aryl or unsubstituted or substituted $C_2$ to $C_{24}$ heteroaryl, and wherein the at least one of the at least one ligand fragment Y has a directed dipole moment of less than -3.6 debye, and wherein the at least one ligand L is negatively charged.

According to one embodiment, wherein the at least one ligand L is represented by formula(III):

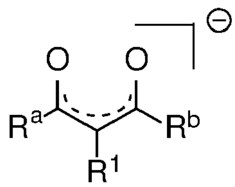

(III),

wherein

$R^1$ is selected from H, D, CN, substituted or unsubstituted $C_6$ to $C_{24}$ aryl, substituted or unsubstituted $C_2$ to $C_{24}$ heteroaryl, substituted or unsubstituted $C_1$ to $C_{24}$ alkyl, substituted or unsubstituted $C_3$ to $C_{24}$ carbocyclyl, or substituted or unsubstituted $C_2$ to $C_{24}$ heterocyclyl, ligand fragment Y, wherein the substituents are selected from the group comprising an electron-withdrawing group, halogen, F, Cl, CN, partially fluorinated or perfluorinated $C_1$ to $C_8$ alkyl, partially fluorinated or perfluorinated $C_6$ to $C_{12}$ aryl, or partially fluorinated or perfluorinated $C_2$ to $C_{12}$ heteroaryl;

$R^a$ and $R^b$ are independently selected from substituted or unsubstituted $C_6$ to $C_{24}$ aryl, substituted or unsubstituted $C_2$ to $C_{24}$ heteroaryl, substituted or unsubstituted $C_1$ to $C_{24}$ alkyl, substituted or unsubstituted $C_3$ to $C_{24}$ carbocyclyl, or substituted or unsubstituted $C_2$ to $C_{24}$ heterocyclyl, ligand fragment Y, wherein the substituents are selected from the group comprising an electron-withdrawing group, halogen, F, Cl, CN, partially fluorinated or perfluorinated $C_1$ to $C_8$ alkyl, perfluorinated $C_6$ to $C_{12}$ aryl, or partially fluorinated or perfluorinated $C_2$ to $C_{12}$ heteroaryl;

wherein at least one of $R^1$, $R^a$ and $R^b$ contains a ligand fragment Y;
wherein at least one of the at least one ligand fragment Y is independently selected from substituted $C_6$ to $C_{24}$ aryl or unsubstituted or substituted $C_2$ to $C_{24}$ heteroaryl, and wherein the at least one of the at least one ligand fragment Y has a directed dipole moment of less than -3.6 debye, and wherein the at least one ligand L is negatively charged.

According to one embodiment, wherein $R^1$ is or contains the ligand fragment Y. According to one embodiment, wherein $R^1$ contains the ligand fragment Y.

Metal compound

The metal compound is non-emissive. In the context of the present specification the term "essentially non-emissive" or "non-emissive" means that the contribution of the metal compound to the visible emission spectrum from an organic electronic device, preferably OLED or display device, is less than 10 %, preferably less than 5 % relative to the visible emission spectrum. The visible emission spectrum is an emission spectrum with a wavelength of about ≥ 380 nm to about ≤ 780 nm.

Ligand fragment Y

The ligand fragmen Y can be selected from Table 1. The dotted line represents the binding position to the coordinating linker X. Table 1 shows suitable ligand fragments Y that can be used according to the invention of formula K1 to K31.

Table 1: Ligand fragments Y of formula K1 to K31

| No | Structure | total Dipole [Debye] | directed Dipole Dx [Debye] |
|---|---|---|---|
| K1 | 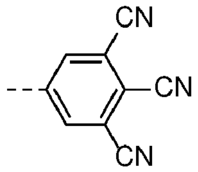 | 7.51 | -7.50 |
| K2 | 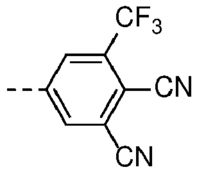 | 7.02 | -6.80 |
| K3 | 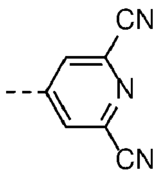 | 6.33 | -6.17 |
| K4 | 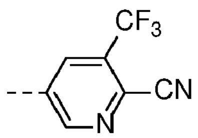 | 6.09 | -6.09 |
| K5 | 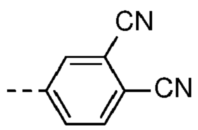 | 7.08 | -6.03 |
| K6 | 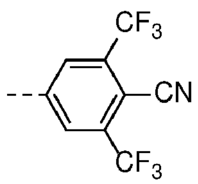 | 5.88 | -5.88 |
| K7 | 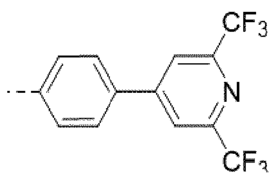 | 5.80 | -5.79 |
| K8 | 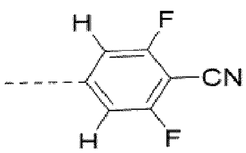 | 5.58 | -5.58 |

(continued)

| No | Structure | total Dipole [Debye] | directed Dipole Dx [Debye] |
|---|---|---|---|
| K9 | 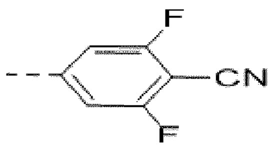 | 5.58 | -5.58 |
| K10 | 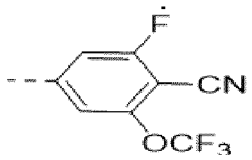 | 5.51 | -5.51 |
| K11 | 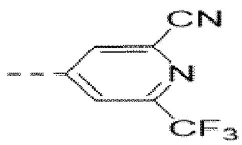 | 5.64 | -5.44 |
| K12 | 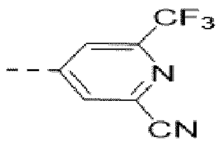 | 5.64 | -5.43 |
| K13 | 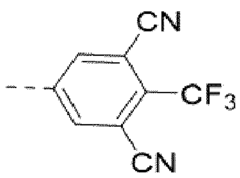 | 5.41 | -5.40 |
| K14 | 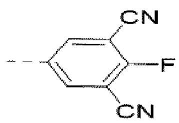 | 5.30 | -5.30 |
| K15 | 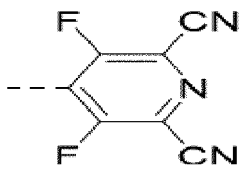 | 5.20 | -5.20 |
| K16 | 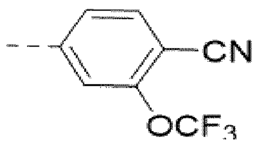 | 5.16 | -5.01 |
| K17 | 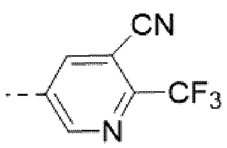 | 5.27 | -4.97 |

(continued)

| No | Structure | total Dipole [Debye] | directed Dipole Dx [Debye] |
|---|---|---|---|
| K18 | 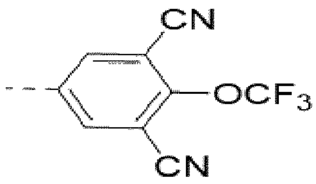 | 4.62 | -4.62 |
| K19 | 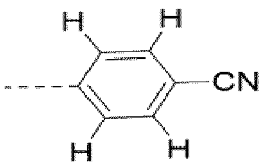 | 4.56 | -4.56 |
| K20 | 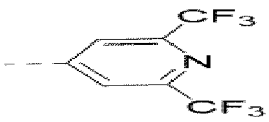 | 4.51 | -4.51 |
| K21 | 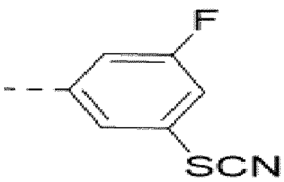 | 4.36 | -4.37 |
| K22 | 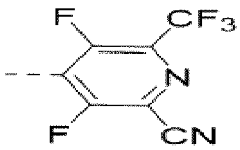 | 4.62 | -4.34 |
| K23 | 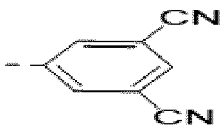 | 4.28 | -4.28 |
| K24 | 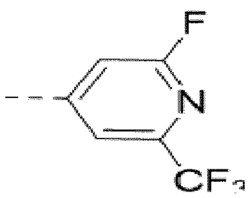 | 4.16 | -4.10 |
| K25 | 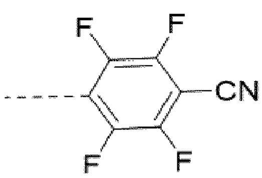 | 4.06 | -4.06 |
| K26 | 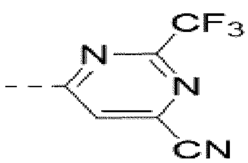 | 4.10 | -4.04 |

(continued)

| No | Structure | total Dipole [Debye] | directed Dipole Dx [Debye] |
|---|---|---|---|
| K27 | 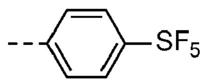 | 4.03 | -4.02 |
| K28 | 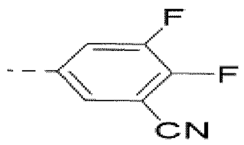 | 4.81 | -3.99 |
| K29 | 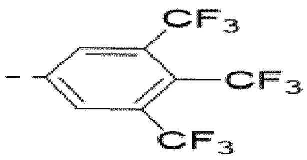 | 3.82 | -3.81 |
| K30 | 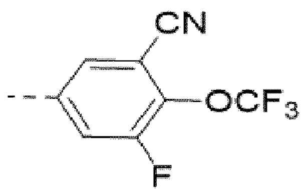 | 4.59 | -3.68 |
| K31 | 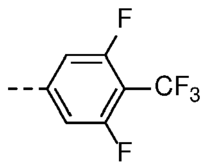 | 3.65 | -3.64 |

Semiconductor layer

According to one embodiment the at least one semiconductor layer comprising the semiconductor layer is an organic semiconductor layer, and preferably the at least one semiconductor layer is non-emissive.

According to one embodiment of the invention, at least one semiconductor layer is arranged and/or provided adjacent to the anode.

According to one embodiment of the invention, at least one semiconductor layer is in direct contact with the anode.

According to one embodiment of the invention, wherein the at least one semiconductor layer is a hole-injection layer.

According to one embodiment of the invention, wherein the at least one of the semiconductor layers is a hole-injection layer, which consists of at least one metal compound or comprises at least one metal compound.

In case the at least one semiconductor layer is a hole-injection layer and/ or is arranged and/or provided adjacent to the anode then it is especially preferred that this layer consists essentially of at least one metal compound.

According to another aspect, the at least one semiconductor layer may have a layer thickness of at least about ≥ 0.5 nm to about ≤ 10 nm, preferably of about ≥ 2 nm to about ≤ 8 nm, also preferred of about ≥ 3 nm to about ≤ 5 nm.

According to one embodiment, wherein the semiconductor layer comprises at least one matrix compound, wherein the at least one matrix compound is a non-polymeric compound, preferably the matrix compound is a covalent matrix compound or a substantially covalent matrix compound.

Preferably at least one semiconductor layer comprising a covalent matrix compound or substantially covalent matrix compound is arranged on or provided adjacent to the anode.

Preferred examples of covalent matrix compounds are organic compounds, consisting predominantly from covalently bound C, H, O, N, S, which may optionally comprise also covalently bound B, P, As, Se. Organometallic compounds comprising covalent bonds carbon-metal, metal complexes comprising organic ligands and metal salts of organic acids are further examples of organic compounds that may serve as organic covalent matrix compound or substantially covalent matrix compounds.

In one embodiment, wherein the covalent matrix compound or substantially covalent matrix compound lacks metal atoms and majority of its skeletal atoms is selected from C, O, S, N. Alternatively, the covalent matrix compound or substantially covalent matrix compound lacks metal atoms and majority of its skeletal atoms is selected from C and N.

In one embodiment, wherein the HOMO level of the covalent matrix compound or substantially covalent matrix compound may be more negative than the HOMO level of N2,N2,N2',N2',N7,N7,N7',N7'-octakis(4-methoxyphenyl)-9,9'-spirobi[fluorene]-2,2',7,7'-tetraamine (CAS 207739-72-8) when determined under the same conditions.

In one embodiment, wherein the calculated HOMO level of the covalent matrix compound or substantially covalent matrix compound may be more negative than -4.27 eV, preferably more negative than -4.3 eV, alternatively more negative than -4.5 eV, alternatively more negative than -4.6 eV, alternatively more negative than -4.65 eV.

According to another aspect, the semiconductor layer further comprises a covalent matrix compound or substantially covalent matrix compound with an oxidation potential more positive than - 0.2 V and more negative than 1.22 V, when measured by cyclic voltammetry in dichloromethane vs. Fc/Fc+, preferably more positive than - 0.18 V and more negative than 1.12 V. Under these conditions the oxidation potential of spiro-MeO-TAD (CAS 207739-72-8) is - 0.07 V.

In one embodiment, wherein the HOMO level of the covalent matrix compound or substantially covalent matrix compound may be more negative than the HOMO level of N2,N2,N2',N2',N7,N7,N7',N7'-octakis(4-methoxyphenyl)-9,9'-spirobi[fluorene]-2,2',7,7'-tetraamine (CAS 207739-72-8) and more positive than the HOMO level of N4,N4'''-di(naphthalen-1-yl)-N4,N4'''-diphenyl-[1,1':4',1":4",1'''-quaterphenyl]-4,4'''-diamine when determined under the same conditions.

In one embodiment, wherein the covalent matrix compound or substantially covalent matrix compound may be free of alkoxy groups.

In one embodiment, wherein the calculated HOMO level of the covalent matrix compound or substantially covalent matrix compound may be selected in the range of < -4.27 eV and > -4.84 eV, alternatively in the range of < -4.3 eV and > -4.84 eV, alternatively in the range of < -4.5 eV and > -4.84 eV, alternatively in the range of < -4.5 eV and > -4.84 eV, alternatively in the range of < -4.6 eV and > -4.84 eV.

In one embodiment, wherein the calculated HOMO level of the covalent matrix compound or substantially covalent matrix compound may be selected in the range of < -4.27 eV and > -4.8 eV, alternatively in the range of < -4.3 eV and > -4.8 eV, alternatively in the range of < -4.5 eV and > -4.8 eV, alternatively in the range of < -4.5 eV and > -4.8 eV, alternatively in the range of < -4.6 eV and > -4.8 eV, alternatively in the range of < -4.65 eV and > -4.8 eV.

Preferably, the covalent matrix compound or substantially covalent matrix compound comprises at least one arylamine compound, alternatively a diarylamine compound, alternatively a triarylamine compound.

According to an embodiment of the organic electronic device, wherein the covalent matrix compound or substantially covalent matrix compound, preferably of the hole injection layer, may comprise a compound of formula (2) or formula (3):

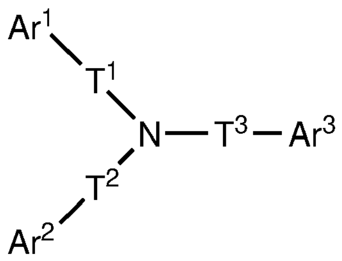
(2),
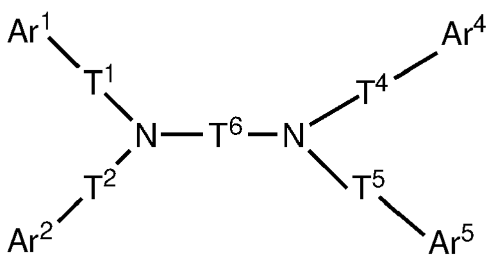
(3),

wherein

| | |
|---|---|
| $T^1$, $T^2$, $T^3$, $T^4$ and $T^5$ | may be independently selected from a single bond, phenylene, biphenylene, terphenylene or naphthenylene, preferably a single bond or phenylene; |
| $T^6$ | is selected from substituted or unsubstituted phenylene, biphenylene, terphenylene or naphthenylene; wherein the substituent on phenylene is selected from $C_6$ to $C_{18}$ aryl , $C_3$ to $C_{18}$ heteroaryl, phenyl, biphenyl, carbazole, phenyl carbazole, preferably biphenyl or 9-phenyl carbazole; |
| $Ar^1$, $Ar^2$, $Ar^3$, $Ar^4$ and $Ar^5$ | may be independently selected from from substituted or unsubstituted $C_6$ to $C_{20}$ aryl, or substituted or unsubstituted $C_3$ to $C_{20}$ heteroarylene, substituted or unsubstituted biphenylene, substituted or unsubstituted fluorene, substituted 9-fluorene, substituted 9,9-fluorene, substituted or unsubstituted naphthalene, substituted or unsubstituted anthracene, substituted or unsubstituted phenanthrene, substituted or unsubstituted pyrene, substituted |

or unsubstituted perylene, substituted or unsubstituted triphenylene, substituted or unsubstituted tetracene, substituted or unsubstituted tetraphene, substituted or unsubstituted dibenzofurane, substituted or unsubstituted dibenzothiophene, substituted or unsubstituted xanthene, substituted or unsubstituted carbazole, substituted 9-phenylcarbazole, substituted or unsubstituted azepine, substituted or unsubstituted dibenzo[b,f]azepine, substituted or unsubstituted 9,9'-spirobi[fluorene], substituted or unsubstituted 9,9'-spirobi[fluorene] with at least one fused aromatic ring, substituted or unsubstituted spiro[fluorene-9,9'-xanthene], a substituted or unsubstituted aromatic fused ring system comprising two aromatic 6-member rings and two aromatic 5-member rings, wherein at least one 5-member ring comprises a hetero atom, a substituted or unsubstituted aromatic fused ring system comprising two aromatic 6-member rings and two aromatic 5-member rings, wherein at least one 5-member ring comprises an O atom, or a substituted or unsubstituted aromatic fused ring system comprising at least three substituted or unsubstituted aromatic rings selected from the group comprising substituted or unsubstituted non-hetero, substituted or unsubstituted hetero 5-member rings, substituted or unsubstituted 6-member rings and/or substituted or unsubstituted 7-member rings, substituted or unsubstituted fluorene, substituted or unsubstituted fluorene with an annelated substitued or unsubstitued hetero or non-hetero ring system comprising 2 to 6 substituted or unsubstituted 5-, 6- or 7-member rings, or a fused ring system comprising 2 to 6 substituted or unsubstituted 5- to 7-member rings and the rings are selected from the group comprising (i) unsaturated 5- to 7-member ring of a heterocycle, (ii) 5- to 6-member of an aromatic heterocycle, (iii) unsaturated 5- to 7-member ring of a non-heterocycle, (iv) 6-member ring of an aromatic non-heterocycle;

wherein the substituents of $Ar^1$, $Ar^2$, $Ar^3$, $Ar^4$ and $Ar^5$ are selected the same or different from the group comprising H, $C(=O)R^2$, CN, $Si(R^2)_3$, $P(=O)(R^2)_2$, $OR^2$, $S(=O)R^2$, $S(=O)_2R^2$, straight-chain alkyl having 1 to 20 carbon atoms, branched alkyl having 1 to 20 carbon atoms, cyclic alkyl having 3 to 20 carbon atoms, alkenyl or alkynyl groups having 2 to 20 carbon atoms, alkoxy groups having 1 to 20 carbon atoms, $C_6$ to $C_{18}$ aryl, $C_3$ to $C_{18}$ heteroaryl, a fused ring system comprising 2 to 6 unsubstituted 5- to 7-member rings and the rings are selected from the group comprising unsaturated 5- to 7-member ring of a heterocycle, 5- to 6-member of an aromatic heterocycle, unsaturated 5- to 7-member ring of a non-heterocycle, and 6-member ring of an aromatic non-heterocycle.

Preferably, the substituents of $Ar^1$, $Ar^2$, $Ar^3$, $Ar^4$ and $Ar^5$ are selected the same or different from the group comprising H, straight-chain alkyl having 1 to 6 carbon atoms, branched alkyl having 1 to 6 carbon atoms, cyclic alkyl having 3 to 6 carbon atoms, alkenyl or alkynyl groups having 2 to 6 carbon atoms, alkoxy groups having 1 to 6 carbon atoms, $C_6$ to $C_{18}$ aryl, $C_3$ to $C_{18}$ heteroaryl, a fused ring system comprising 2 to 4 unsubstituted 5- to 7-member rings and the rings are selected from the group comprising unsaturated 5- to 7-member ring of a heterocycle, 5- to 6-member of an aromatic heterocycle, unsaturated 5- to 7-member ring of a non-heterocycle, and 6-member ring of an aromatic non-heterocycle; more preferred the substituents are selected the same or different from the group consisting of H, straight-chain alkyl having 1 to 4 carbon atoms, branched alkyl having 1 to 4 carbon atoms, cyclic alkyl having 3 to 4 carbon atoms and/or phenyl. Thereby, the compound of formula (2) or (3) may have a rate onset temperature suitable for mass production.

According to an embodiment of the organic electronic device, wherein covalent matrix compound or substantially covalent matrix compound, preferably the matrix compound of the hole injection layer, comprises a compound of formula (2) or formula (3):

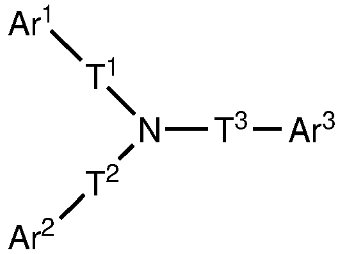
(2),
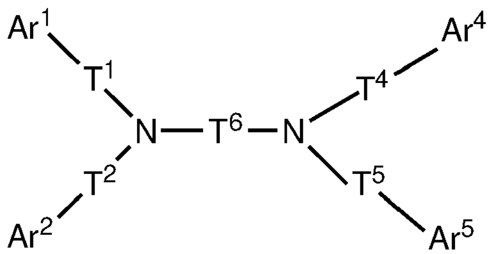
(3),

wherein

| | |
|---|---|
| $T^1$, $T^2$, $T^3$, $T^4$ and $T^5$ | may be independently selected from a single bond, phenylene, biphenylene, terphenylene or naphthenylene, preferably a single bond or phenylene; |

$T^6$ is selected from substituted or unsubstituted phenylene, biphenylene, terphenylene or naphthenylene; wherein the substituent on phenylene is selected from $C_6$ to $C_{18}$ aryl , $C_3$ to $C_{18}$ heteroaryl, phenyl, biphenyl, carbazole, phenyl carbazole, preferably biphenyl or 9-phenyl carbazole;

$Ar^1$, $Ar^2$, $Ar^3$, $Ar^4$ and $Ar^5$ may be independently selected from unsubstituted $C_6$ to $C_{20}$ aryl, or unsubstituted $C_3$ to $C_{20}$ heteroarylene, unsubstituted biphenylene, unsubstituted fluorene, substituted 9-fluorene, substituted 9,9-fluorene, unsubstituted naphthalene, unsubstituted anthracene, unsubstituted phenanthrene, unsubstituted pyrene, unsubstituted perylene, unsubstituted triphenylene, unsubstituted tetracene, unsubstituted tetraphene, unsubstituted dibenzofurane, unsubstituted dibenzothiophene, unsubstituted xanthene, unsubstituted carbazole, substituted 9-phenylcarbazole, unsubstituted azepine, unsubstituted dibenzo[b,f]azepine, unsubstituted 9,9'-spirobi[fluorene], unsubstituted spiro[fluorene-9,9'-xanthene], or a unsubstituted aromatic fused ring system comprising at least three unsubstituted aromatic rings selected from the group comprising unsubstituted non-hetero, unsubstituted hetero 5-member rings, unsubstituted 6-member rings and/or unsubstituted 7-member rings, unsubstituted fluorene, or a fused ring system comprising 2 to 6 unsubstituted 5- to 7-member rings and the rings are selected from the group comprising (i) unsaturated 5- to 7-member ring of a heterocycle, (2) 5- to 6-member of an aromatic heterocycle, (3) unsaturated 5- to 7-member ring of a non-heterocycle, (iv) 6-member ring of an aromatic non-heterocycle.

According to an embodiment of the organic electronic device, wherein covalent matrix compound or substantially covalent matrix compound, preferably of the covalent matrix compound or substantially covalent matrix compound of the hole injection layer, comprises a compound of formula (2) or formula (3):

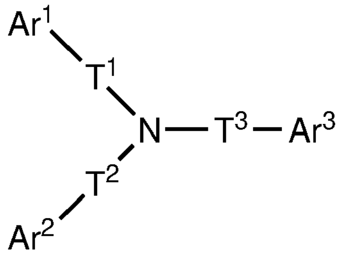
(2),
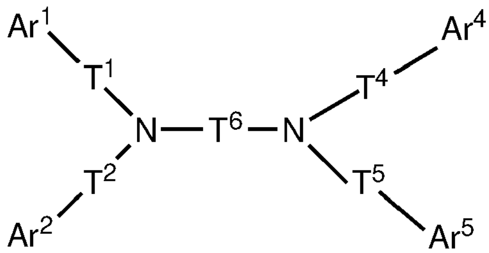
(3),

wherein

$T^1$, $T^2$, $T^3$, $T^4$ and $T^5$ may be independently selected from a single bond, phenylene, biphenylene, terphenylene or naphthenylene, preferably a single bond or phenylene;

$T^6$ is selected from substituted or unsubstituted phenylene, biphenylene, terphenylene or naphthenylene; wherein the substituent on phenylene is selected from $C_6$ to $C_{18}$ aryl , $C_3$ to $C_{18}$ heteroaryl, phenyl, biphenyl, carbazole, phenyl carbazole, preferably biphenyl or 9-phenyl carbazole;

$Ar^1$, $Ar^2$, $Ar^3$, $Ar^4$ and $Ar^5$ may be independently selected from unsubstituted $C_6$ to $C_{20}$ aryl, or unsubstituted $C_3$ to $C_{20}$ heteroarylene, unsubstituted biphenylene, unsubstituted fluorene, substituted 9-fluorene, substituted 9,9-fluorene, unsubstituted naphthalene, unsubstituted anthracene, unsubstituted phenanthrene, unsubstituted pyrene, unsubstituted perylene, unsubstituted triphenylene, unsubstituted tetracene, unsubstituted tetraphene, unsubstituted dibenzofurane, unsubstituted dibenzothiophene, unsubstituted xanthene, unsubstituted carbazole, substituted 9-phenylcarbazole, unsubstituted azepine, unsubstituted dibenzo[b,f]azepine, unsubstituted 9,9'-spirobi[fluorene], unsubstituted spiro[fluorene-9,9'-xanthene].

The substituents of $Ar^1$, $Ar^2$, $Ar^3$, $Ar^4$ and $Ar^5$ if not otherwise mentioned can be selected the same or different from the group comprising H, $C(=O)R^2$, CN, $Si(R^2)_3$, $P(=O)(R^2)_2$, $OR^2$, $S(=O)R^2$, $S(=O)_2R^2$, straight-chain alkyl having 1 to 20 carbon atoms, branched alkyl having 1 to 20 carbon atoms, cyclic alkyl having 3 to 20 carbon atoms, alkenyl or alkynyl groups having 2 to 20 carbon atoms, alkoxy groups having 1 to 20 carbon atoms, $C_6$ to $C_{18}$ aryl, $C_3$ to $C_{18}$ heteroaryl, a fused ring system comprising 2 to 6 unsubstituted 5- to 7-member rings and the rings are selected from the group comprising unsaturated 5- to 7-member ring of a heterocycle, 5- to 6-member of an aromatic heterocycle, unsaturated 5- to 7-member ring of a non-heterocycle, and 6-member ring of an aromatic non-heterocycle.

Thereby, the compound of formula (2) or (3) may have a rate onset temperature suitable for mass production.

According to an embodiment wherein $T^1$, $T^2$, $T^3$, $T^4$ and $T^5$, or $T^1$, $T^2$ and $T^3$ may be independently selected from a single bond, phenylene, biphenylene or terphenylene. According to an embodiment wherein $T^1$, $T^2$, $T^3$, $T^4$ and $T^5$, or $T^1$, $T^2$ and $T^3$ may be independently selected from phenylene, biphenylene or terphenylene and one of $T^1$, $T^2$, $T^3$, $T^4$ and $T^5$, or $T^1$, $T^2$ and $T^3$ are a single bond. According to an embodiment wherein $T^1$, $T^2$, $T^3$, $T^4$ and $T^5$, or $T^1$, $T^2$ and $T^3$ may be independently selected from phenylene or biphenylene and one of $T^1$, $T^2$, $T^3$, $T^4$ and $T^5$, or $T^1$, $T^2$ and $T^3$ are a single bond. According to an embodiment wherein $T^1$, $T^2$, $T^3$, $T^4$ and $T^5$, or $T^1$, $T^2$ and $T^3$ may be independently selected from phenylene or biphenylene and two of $T^1$, $T^2$, $T^3$, $T^4$ and $T^5$, or $T^1$, $T^2$ and $T^3$ are a single bond.

According to an embodiment wherein $T^1$, $T^2$, $T^3$, $T^4$ and $T^5$, or $T^1$, $T^2$ and $T^3$ may be independently selected from phenylene and one of $T^1$, $T^2$, $T^3$, $T^4$ and $T^5$, or $T^1$, $T^2$ and $T^3$ are a single bond. According to an embodiment wherein $T^1$, $T^2$, $T^3$, $T^4$ and $T^5$, or $T^1$, $T^2$ and $T^3$ may be independently selected from phenylene and two of $T^1$, $T^2$, $T^3$, $T^4$ and $T^5$, or $T^1$, $T^2$ and $T^3$ are a single bond.

According to an embodiment wherein $T^6$ may be is selected from substituted or unsubstituted phenylene, biphenylene, terphenylene or naphthenylene; wherein the substituent on phenylene is selected from $C_6$ to $C_{18}$ aryl, $C_3$ to $C_{18}$ heteroaryl, phenyl, biphenyl, carbazole, phenyl carbazole, preferably biphenyl or 9-phenyl carbazole. According to an embodiment wherein $T^6$ may substituted or unsubstituted phenylene, wherein the substituent on phenylene is selected from $C_6$ to $C_{18}$ aryl , $C_3$ to $C_{18}$ heteroaryl, phenyl, biphenyl, carbazole, phenyl carbazole, preferably biphenyl or 9-phenyl carbazole. According to an embodiment wherein $T^6$ may be biphenylene. According to an embodiment wherein $T^6$ may be terphenylene.

According to an embodiment wherein $Ar^1$, $Ar^2$, $Ar^3$, $Ar^4$ and $Ar^5$ may be independently selected from D1 to D16:

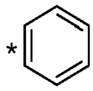 (D1), 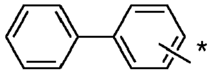 (D2), 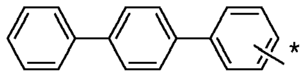 (D3), 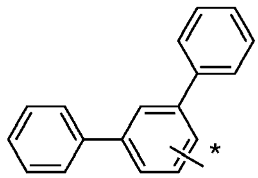 (D4),

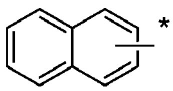 (D5), 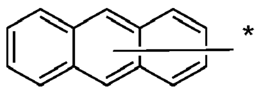 (D6), 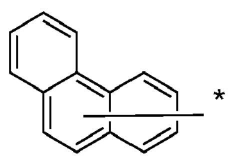 (D7), 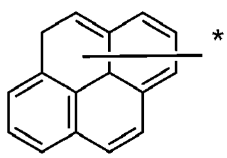 (D8),

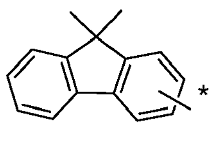 (D9), 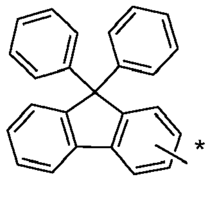 (D10), 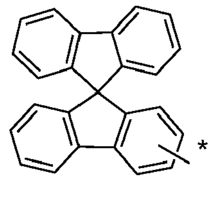 (D11),

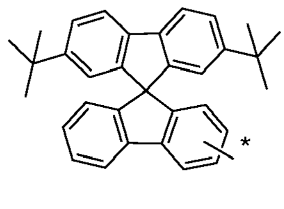 (D12), 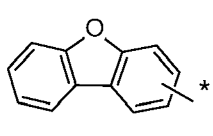 (D13), 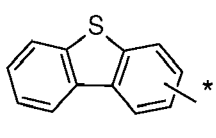 (D14),

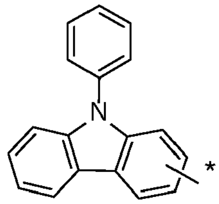 (D15), 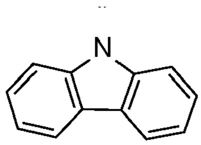 (D16),

wherein the asterix "*" denotes the binding position.

According to an embodiment, wherein $Ar^1$, $Ar^2$, $Ar^3$, $Ar^4$ and $Ar^5$ may be independently selected from D1 to D15; alternatively selected from D1 to D10 and D13 to D15.

According to an embodiment, wherein $Ar^1$, $Ar^2$, $Ar^3$, $Ar^4$ and $Ar^5$ may be independently selected from the group consisting of D1, D2, D5, D7, D9, D10, D13 to D16.

The rate onset temperature may be in a range particularly suited to mass production, when $Ar^1$, $Ar^2$, $Ar^3$, $Ar^4$ and $Ar^5$ are selected in this range.

The "matrix compound of formula (2) or formula (3) " may be also referred to as "hole transport compound".

According to one embodiment the matrix compound of formula (2) or formula (3) may comprise at least ≥ 1 to ≤ 6 substituted or unsubstituted aromatic fused ring systems comprising heteroaromatic rings.

According to one embodiment the matrix compound of formula (2) or formula (3) may comprise at least ≥ 1 to ≤ 6 substituted or unsubstituted aromatic fused ring systems comprising heteroaromatic rings and at least ≥ 1 to ≤ 3 substituted or unsubstituted unsaturated 5- to 7-member ring of a heterocycle, preferably ≥ 2 to ≤ 5 substituted or unsubstituted aromatic fused ring systems comprising heteroaromatic rings.

According to one embodiment the matrix compound of formula (2) or formula (3) may comprise at least ≥ 1 to ≤ 6 substituted or unsubstituted aromatic fused ring systems comprising heteroaromatic rings and at least ≥ 1 to ≤ 3 substituted or unsubstituted unsaturated 5- to 7-member ring of a heterocycle, preferably ≥ 2 to ≤ 5 substituted or unsubstituted aromatic fused ring systems comprising heteroaromatic rings, and at least ≥ 1 to ≤ 3 substituted or unsubstituted unsaturated 5- to 7-member ring of a heterocycle, further preferred 3 or 4 substituted or unsubstituted aromatic fused ring systems comprising heteroaromatic rings and optional at least ≥ 1 to ≤ 3 substituted or unsubstituted unsaturated 5- to 7-member ring of a heterocycle, and additional preferred wherein the aromatic fused ring systems comprising heteroaromatic rings are unsubstituted and optional at least ≥ 1 to ≤ 3 unsubstituted unsaturated 5- to 7-member ring of a heterocycle.

According to one embodiment the matrix compound of formula (2) or formula (3) may comprise at least ≥ 1 to ≤ 6 substituted or unsubstituted aromatic fused ring systems, preferably ≥ 2 to ≤ 5 substituted or unsubstituted aromatic fused ring systems, and further preferred 3 or 4 substituted or unsubstituted aromatic fused ring systems.

According to one embodiment the matrix compound of formula (2) or formula (3) may comprise at least ≥ 1 to ≤ 6 substituted or unsubstituted aromatic fused ring systems, preferably ≥ 2 to ≤ 5 substituted or unsubstituted aromatic fused ring systems, and further preferred 3 or 4 substituted or unsubstituted aromatic fused ring systems, which comprises substituted or unsubstituted heteroaromatic rings.

According to one embodiment the matrix compound of formula (2) or formula (3) may comprise at least ≥ 1 to ≤ 3 or 2 substituted or unsubstituted unsaturated 5- to 7-member ring of a heterocycle.

According to one embodiment the matrix compound of formula (2) or formula (3) may comprise at least ≥ 1 to ≤ 3 or 2 substituted or unsubstituted unsaturated 7-member ring of a heterocycle.

According to one embodiment substituted or unsubstituted aromatic fused ring systems of the covalent matrix compound or substantially covalent matrix compound of formula (2) or formula (3) may comprise at least ≥ 1 to ≤ 3 or 2 substituted or unsubstituted unsaturated 5- to 7-member ring of a heterocycle.

According to one embodiment the substituted or unsubstituted aromatic fused ring systems of the covalent matrix compound or substantially covalent matrix compound of formula (2) or formula (3) may comprise at least ≥ 1 to ≤ 3 or 2 substituted or unsubstituted unsaturated 7-member ring of a heterocycle.

According to one embodiment the matrix compound of formula (2) or formula (3) may comprise at least ≥ 1 to ≤ 6 substituted or unsubstituted aromatic fused ring systems, preferably ≥ 2 to ≤ 5 substituted or unsubstituted aromatic fused ring systems, and further preferred 3 or 4 substituted or unsubstituted aromatic fused ring systems, and wherein the aromatic fused ring system comprises substituted or unsubstituted unsaturated 5- to 7-member ring of a heterocycle.

According to one embodiment the matrix compound of formula (2) or formula (3) may comprise at least ≥ 1 to ≤ 6 substituted or unsubstituted aromatic fused ring systems, preferably ≥ 2 to ≤ 5 substituted or unsubstituted aromatic fused ring systems, and further preferred 3 or 4 substituted or unsubstituted aromatic fused ring systems, which comprises substituted or unsubstituted heteroaromatic rings, and wherein the aromatic fused ring system comprises substituted or unsubstituted unsaturated 5- to 7-member ring of a heterocycle.

According to one embodiment the matrix compound of formula (2) or formula (3) may comprise at least ≥ 1 to ≤ 6 substituted or unsubstituted aromatic fused ring systems, preferably ≥ 2 to ≤ 5 substituted or unsubstituted aromatic fused ring systems, and further preferred 3 or 4 substituted or unsubstituted aromatic fused ring systems, and wherein the aromatic fused ring system comprises at least ≥ 1 to ≤ 3 or 2 substituted or unsubstituted unsaturated 5- to 7-member ring of a heterocycle.

According to one embodiment the matrix compound of formula (2) or formula (3) may comprise at least ≥ 1 to ≤ 6 substituted or unsubstituted aromatic fused ring systems, preferably ≥ 2 to ≤ 5 substituted or unsubstituted aromatic fused ring systems, and further preferred 3 or 4 substituted or unsubstituted aromatic fused ring systems, which comprises substituted or unsubstituted heteroaromatic rings, and wherein the aromatic fused ring system comprises at least ≥ 1 to ≤ 3 or 2 substituted or unsubstituted unsaturated 5- to 7-member ring of a heterocycle.

According to one embodiment the matrix compound of formula (2) or formula (3) may comprise:

- a substituted or unsubstituted aromatic fused ring system with at least ≥ 2 to ≤ 6, preferably ≥ 3 to ≤ 5, or 4 fused aromatic rings selected from the group comprising substituted or unsubstituted non-hetero aromatic rings, substituted or unsubstituted hetero 5-member rings, substituted or unsubstituted 6-member rings and/or substituted or unsubstituted unsaturated 5-to 7- member ring of a heterocycle; or
- an unsubstituted aromatic fused ring system with at least ≥ 2 to ≤ 6, preferably ≥ 3 to ≤ 5, or 4 fused aromatic rings selected from the group comprising unsubstituted non-hetero aromatic rings, unsubstituted hetero 5-member rings, unsubstituted 6-member rings and/or unsubstituted unsaturated 5- to 7-member ring of a heterocycle.

It should be noted here that the wording "aromatic fused ring system" may include at least one aromatic ring and at least one substituted or unsubstituted unsaturated 5- to 7- member ring. It should be noted here that the substituted or unsubstituted unsaturated 5- to 7- member ring may not be an aromatic ring.

According to one embodiment the matrix compound of formula (2) or formula (3) may comprise at least at least ≥ 1 to ≤ 6, preferably ≥ 2 to ≤ 5, or further preferred 3 or 4 of the substituted or unsubstituted aromatic fused ring systems with:

- at least one unsaturated 5-member ring, and/or
- at least one unsaturated 6-member ring, and/or
- at least one unsaturated 7-member ring; wherein preferably at least one unsaturated 5- and/or at least one unsaturated 7-member ring comprises at least 1 to 3, preferably 1 hetero-atom.

According to one embodiment the matrix compound of formula (2) or formula (3) may comprise at least at least ≥ 1 to ≤ 6, preferably ≥ 2 to ≤ 5, or further preferred 3 or 4 of the substituted or unsubstituted aromatic fused ring systems with:

- at least one aromatic 5-member ring, and/or
- at least one aromatic 6-member ring, and/or
- at least one aromatic 7-member ring; wherein preferably at least one aromatic 5- and/or at least one aromatic 7-member ring comprises at least 1 to 3, preferably 1 hetero-atom;

wherein the substituted or unsubstituted aromatic fused ring system comprises at least ≥ 1 to ≤ 3 or 2 substituted or unsubstituted unsaturated 5- to 7-member ring of a heterocycle.

According to one embodiment the matrix compound of formula (2) or formula (3) may comprise :

- at least ≥ 6 to ≤ 12, preferably ≥ 7 to ≤ 11, further preferred ≥ 8 to ≤ 10 or 9 aromatic rings; and/or
- at least ≥ 4 to ≤ 11, preferably ≥ 5 to ≤ 10, further preferred ≥ 6 to ≤ 9 or in addition preferred 7 or 8 non-hetero aromatic rings, preferably the non-hetero aromatic rings are aromatic $C_6$ rings; and/or
- at least ≥ 1 to ≤ 4, preferably 2 or 3 aromatic 5-member-rings, preferably hetero aromatic 5-member-rings; and/or
- at least 1 or 2 unsaturated 5- to 7-member-ring of a heterocycle, preferably at least 1 or 2 unsaturated 7-member-ring of a heterocycle;
- at least ≥ 6 to ≤ 12, preferably ≥ 7 to ≤ 11, further preferred ≥ 8 to ≤ 10 or 9 aromatic rings, wherein therefrom

  at least ≥ 4 to ≤ 11, preferably ≥ 5 to ≤ 10, further preferred ≥ 6 to ≤ 9 or in addition preferred 7 or 8 are non-hetero aromatic rings, and

  at least ≥ 1 to ≤ 4, preferably 2 or 3 aromatic rings are hetero aromatic rings, wherein the total number of non-hetero aromatic rings and hetero aromatic rings in total does not exceed 12 aromatic rings; and/or

- at least ≥ 6 to ≤ 12, preferably ≥ 7 to ≤ 11, further preferred ≥ 8 to ≤ 10 or 9 aromatic rings, wherein therefrom

  at least ≥ 4 to ≤ 11, preferably ≥ 5 to ≤ 10, further preferred ≥ 6 to ≤ 9 or in addition preferred 7 or 8 are non-hetero aromatic rings, and
  at least ≥ 1 to ≤ 4, preferably 2 or 3 aromatic rings are hetero aromatic rings, wherein the total number of non-hetero aromatic rings and hetero aromatic rings in total does not exceed 12 aromatic rings; and
  the hole transport compound or the hole transport compound according to formula (II) or (III) comprises at least ≥ 1 to ≤ 4, preferably 2 or 3 aromatic 5-member-rings, preferably hetero aromatic 5-member-rings, and/or the hole transport compound or the hole transport compound according to formula (II) or (III) comprises at least 1 or 2 unsaturated 5- to 7-member-ring of a heterocycle, preferably at least 1 or 2 unsaturated 7-member-ring of a heterocycle.

According to one embodiment the matrix compound of formula (2) or formula (3) may comprise a hetero-atom, which may be selected from the group comprising O, S, N, B or P, preferably the hetero-atom may be selected from the group comprising O, S or N.
According to one embodiment the matrix compound of formula (2) or formula (3) may comprise at least at least ≥ 1 to ≤ 6, preferably ≥ 2 to ≤ 5, or further preferred 3 or 4 of the substituted or unsubstituted aromatic fused ring systems with:

- at least one aromatic 5-member ring, and/or
- at least one aromatic 6-member ring, and/or
- at least one aromatic 7-member ring; wherein preferably at least one aromatic 5- and/or at least one aromatic 7-member ring comprises at least 1 to 3, preferably 1 hetero-atom;

wherein the substituted or unsubstituted aromatic fused ring system optional comprises at least ≥ 1 to ≤ 3 or 2 substituted or unsubstituted unsaturated 5- to 7-member ring of a heterocycle; and wherein the substituted or unsubstituted aromatic fused ring system comprises a hetero-atom, which may be selected from the group comprising O, S, N, B, P or Si, preferably the hetero-atom may be selected from the group comprising O, S or N.
According to one embodiment the matrix compound of formula (2) or formula (3) may be free of hetero-atoms which are not part of an aromatic ring and/or part of an unsaturated 7-member-ring, preferably the hole transport compound or the hole transport compound according to formula (1) may be free on N-atoms except N-atoms which are part of an aromatic ring or are part of an unsaturated 7-member-ring.
According to one embodiment, wherein the hole transport compound comprises at least one naphthyl group, carbazole group, dibenzofurane group, dibenzothiophene group and/or substituted fluorenyl group, wherein the substituents are independently selected from methyl, phenyl or fluorenyl.
According to an embodiment of the electronic device, wherein the covalent matrix compound or substantially covalent matrix compound are selected from F1 to F19:

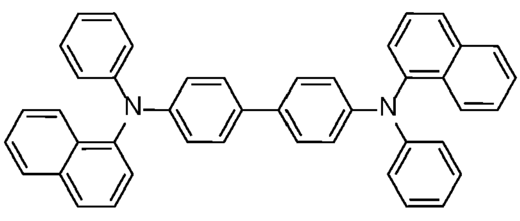
(F1),
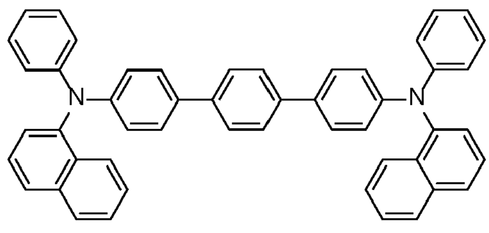
(F2),

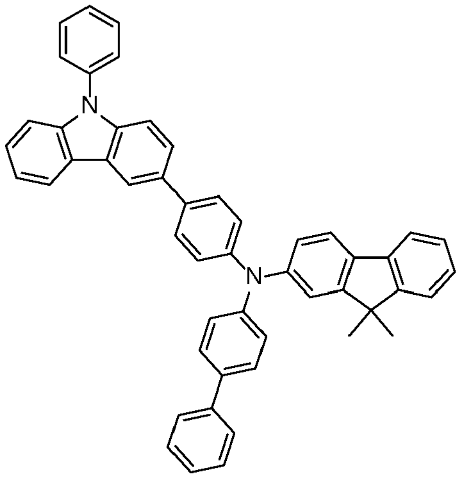

(F3),

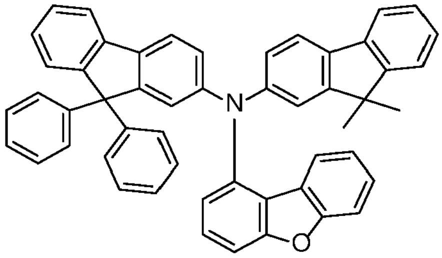

(F4),

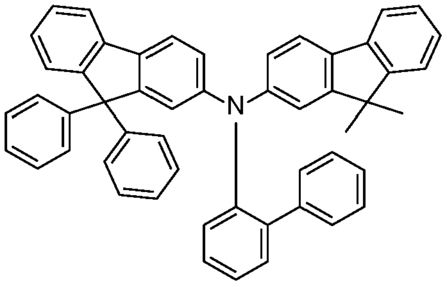

(F5),

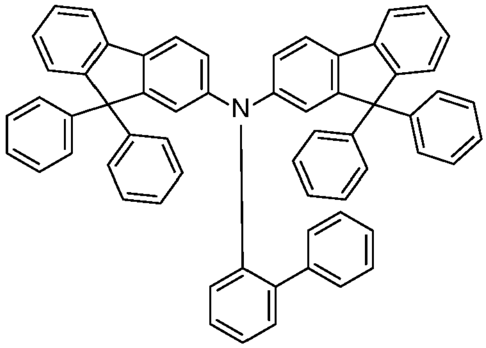

(F6),

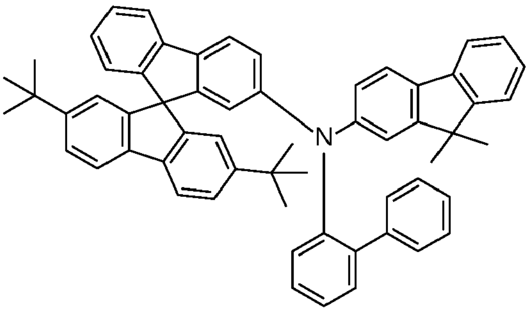

(F7),

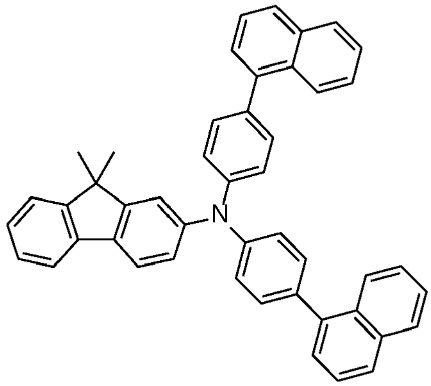

(F8),

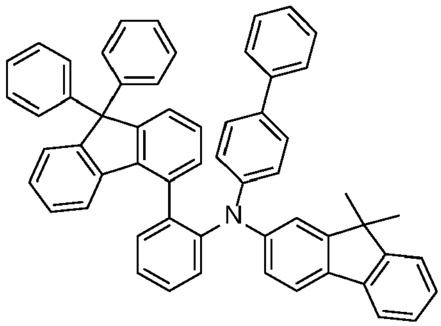
(F9),

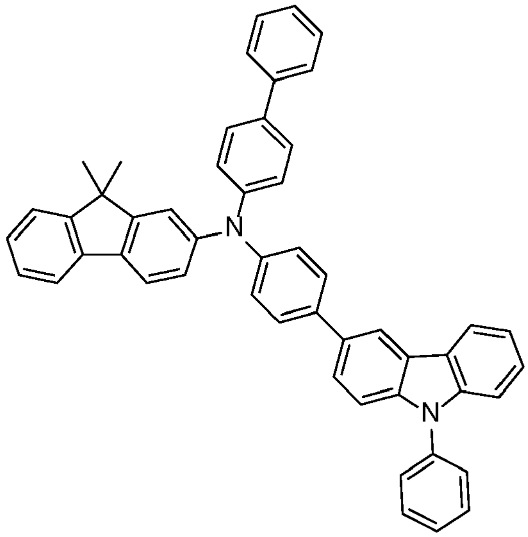
(F10),

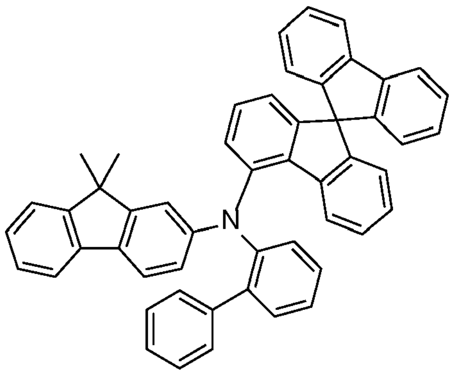
(F11),

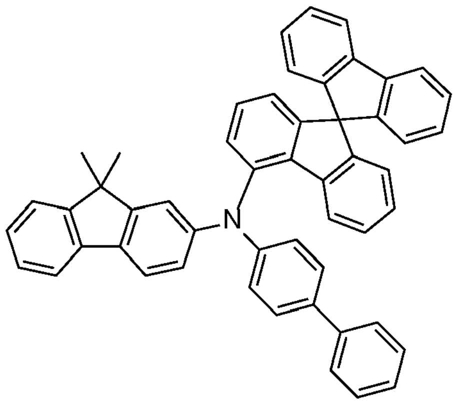
(F12),

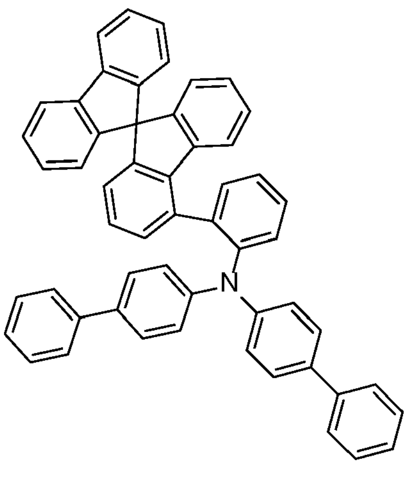
(F13),

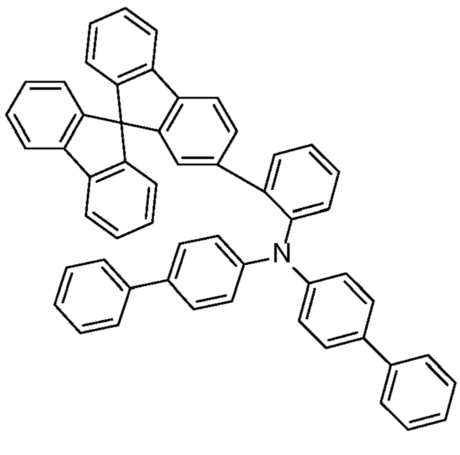
(F14),

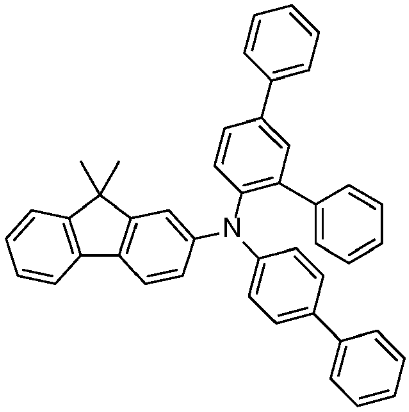
(F15),

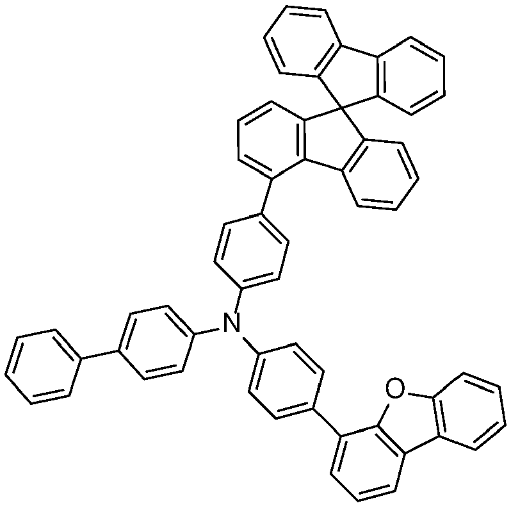
(F16),

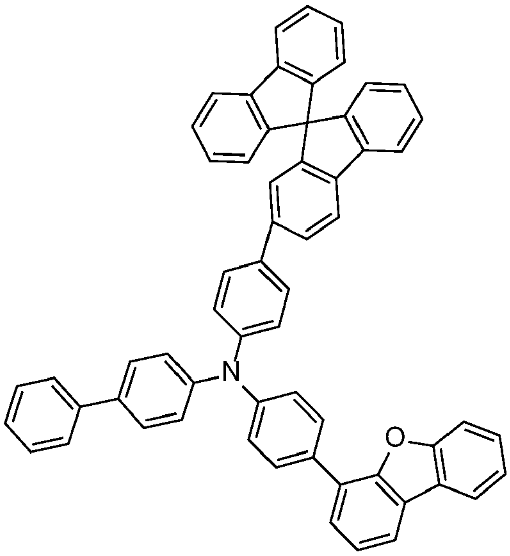
(F17)

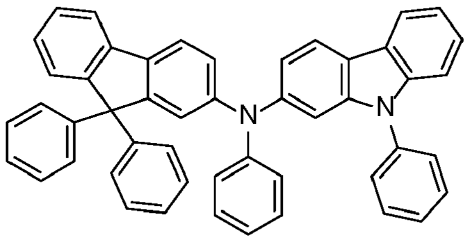
(F18)

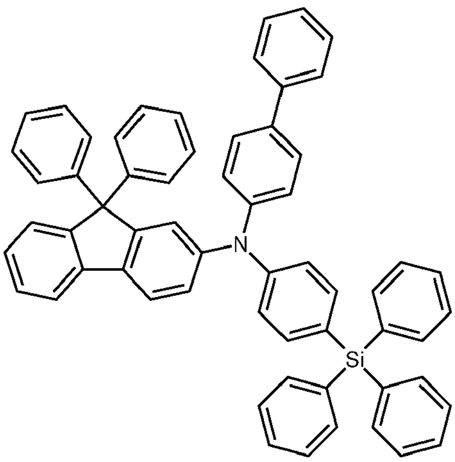
(F19).

The compounds of F1 to F18 are preferred to use as matrix compounds for a hole injection layer. Compound F19 may be used as a matrix compound in an EBL.

The matrix compound of the hole injection layer may be free of HTM014, HTM081, HTM163, HTM222, EL-301, HTM226, HTM355, HTM133, HTM334, HTM604 and EL-22T. The abbreviations denote the manufacturers' names,

for example, of Merck or Lumtec.

According to a furthermore preferred embodiment the covalent matrix compound or substantially covalent matrix compound has the Formula (T-1) to (T-6) as shown in Table 2.

Table 2

| Name | Chemical formula | Calculated HOMO (eV) |
|---|---|---|
| N,N'-Bis-(3-methylphenyl)-N,N'-bis-(phenyl)-benzidine (T-1) | 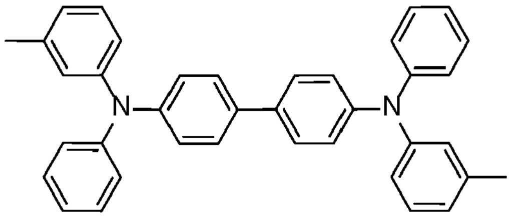 | -4.69 |
| Biphenyl-4-yl(9,9-diphenyl-9H-fluoren-2-yl)-[4-(9-phenyl-9H-carbazol-3-yl) phenyl]-amine (T-2) | 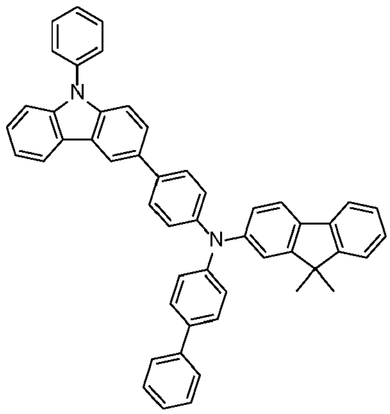 | -4.69 |
| N,N'-Bis(naphthalen-1-yl)-N,N'-bis (phenyl)-benzidine (T-3) | 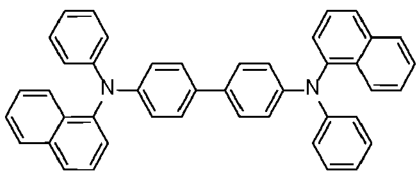 | -4.72 |
| N4,N4,N4',N4'-tetra(biphenyl-4-yl)biphenyl-4,4'-diamine (T-4) | 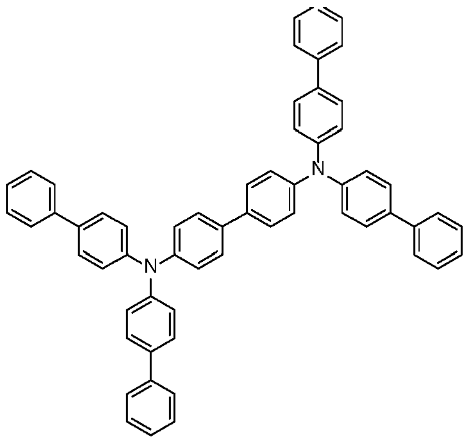 | -4.73 |

(continued)

| Name | Chemical formula | Calculated HOMO (eV) |
|---|---|---|
| N4,N4"-di(naphthalen-1-yl)-N4,N4"-diphenyl-[1,1':4',1"-terphenyl]-4,4"-diamine (T-5) | 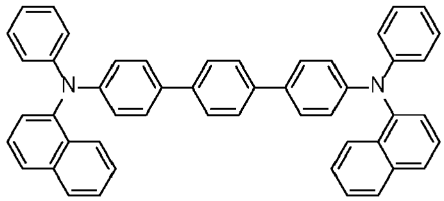 | -4.81 |
| 9,9-dimethyl-N,N-bis(4-(naphthalen-1-yl) phenyl)-9H-fluoren-2-amine (T-6) | 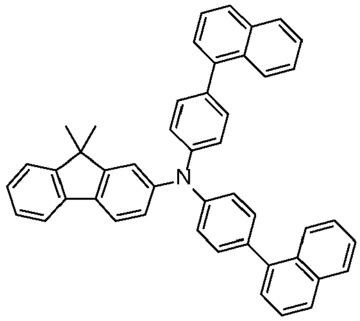 | -4.84 |

According to another aspect, the at least one semiconductor layer further comprises a covalent matrix compound or substantially covalent matrix compound and may comprise:

- at least about ≥ 0.1 wt.-% to about ≤ 50 wt.-%, preferably about ≥ 1 wt.-% to about ≤ 25 wt.-%, and more preferred about ≥ 2 wt.-% to about ≤ 15 wt.-%, of a covalent matrix compound or substantially covalent matrix compound, preferably of formula (2) or formula (3).

According to one embodiment, wherein the semiconductor layer is arranged between the anode layer and the light emitting layer, preferably the semiconductor layer is a hole transport layer or hole injection layer.

According to one embodiment, wherein the semiconductor layer is arranged between the anode layer and the light emitting layer, preferably the semiconductor layer is a hole transport layer or hole injection layer; preferably the at least one semiconductor layer is arranged on the anode layer, and more preferably the at least one semiconductor layer is in direct contact with the anode, wherein the semiconductor layer is preferably a hole injection layer.

Dipole moment and directed dipole moment

The dipole moment $|\vec{\mu}|$ of a molecule containing N atoms is given by:

$$\vec{\mu} = \begin{pmatrix} \mu_x \\ \mu_y \\ \mu_z \end{pmatrix} = \sum_{i=1}^{N} q_i \vec{r_i} = \sum_{i}^{N} q_i \begin{pmatrix} x_i \\ y_i \\ z_i \end{pmatrix}$$

$$|\vec{\mu}| = \sqrt{\mu_x^2 + \mu_y^2 + \mu_z^2}$$

where $q_i$ and $\vec{r_i}$ are the partial charge and position of atom i in the molecule. The directed dipole moment here is referred to the x-component of the dipole vector $\mu_x$ that is defined:

$$\mu_x = \sum_i^N q_i x_i$$

The directed dipole moment and the total dipole moment are determined by a semi-empirical molecular orbital method. The partial charges and atomic positions in the gas phase are obtained using the hybrid functional B3LYP with a 6-31G* basis set as implemented in the program package TURBOMOLE V6.5. Thereby, the center of origin has been set to the atom of the ligand that binds to the metal. The directed dipole moment is calculated using hydrogen, i.e. the ligand fragment radical is calculated with hydrogen.

For the directed dipole moment, the center of origin has been set to the atom of the ligand fragment Y that binds directly or indirectly to the coordinating linker X. The coordinating linker is replaced via a hydrogen atom

Further layers

In accordance with the invention, the organic electroluminescent device may comprise, besides the layers already mentioned above, further layers. Exemplary embodiments of respective layers are described in the following:

Substrate

The substrate may be any substrate that is commonly used in manufacturing of electronic devices, preferably organic electroluminescent devices or OLEDs. If light is to be emitted through the substrate, the substrate shall be a transparent or semitransparent material, for example a glass substrate or a transparent plastic substrate. If light is to be emitted through the top surface, the substrate may be both a transparent as well as a non-transparent material, for example a glass substrate, a plastic substrate, a metal substrate or a silicon substrate.

Anode electrode

The anode electrode also named anode layer may be formed by depositing or sputtering a material that is used to form the anode electrode. The material used to form the anode electrode may be a high work-function material, so as to facilitate hole injection. The anode material may also be selected from a low work function material (i.e. aluminum). The anode electrode may be a transparent or reflective electrode. Transparent conductive oxides, , preferably indium tin oxide (ITO), indium zinc oxide (IZO), tin-dioxide (SnO2), aluminum zinc oxide (AlZO) and zinc oxide (ZnO), may be used to form the anode electrode. The anode electrode may also be formed using metals, typically silver (Ag), gold (Au), or metal alloys.

According to one embodiment, wherein the anode layer may comprise a first anode sub-layer comprising or consisting of Ag or Au, a second anode-sub-layer comprising or consisting of ITO or IZO and optionally a third anode sub-layer comprising or consisting of ITO or IZO. Preferably the first anode sub-layer may comprise or consists of Ag, the second anode-sublayer may comprise or consists of ITO and the third anode sub-layer may comprise or consists of ITO. Preferably, the transparent conductive oxide in the second and third anode sub-layer may be selected the same.

According to one embodiment, wherein the anode layer may comprise a first anode sub-layer comprising Ag or Au having a thickness of 100 to 150 nm, a second anode-sub-layer comprising ITO or IZO having a thickness of 3 to 20 nm and a third anode sub-layer comprising ITO or IZO having a thickness of 3 to 20 nm.

Anode sub-layer

The anode layer may comprise a first anode sub-layer and a second anode sub-layer, wherein the first anode sub-layer comprises a first metal having a work function in the range of ≥ 4 and ≤ 6 eV, and the second anode sub-layer comprises a transparent conductive oxide (TCO).

According to one embodiment the anode layer comprises a first anode sub-layer and a second anode sub-layer and optional a third anode sub layer, wherein the first anode sub-layer comprises a first metal having a work function in the range of ≥ 4 and ≤ 6 eV, and the second anode sub-layer comprises a transparent conductive oxide (TCO); wherein the hole injection layer is arranged between the first light emitting layer and the anode layer, the first anode sub-layer is arranged closer to the substrate, and the second anode sub-layer is arranged closer to the hole injection layer.

First anode sub-layer

According to one embodiment, wherein the first metal of the first anode sub-layer may have a work function in

the range of ≥ 4.2 and ≤ 6 eV. The first metal may be selected from a metal or a metal alloy.

According to one embodiment, wherein the first metal of the first anode sub-layer may be selected from the group comprising Ag, Mg, Al, Cr, Pt, Au, Pd, Ni, Nd, Ir, preferably Ag, Au or Al, and more preferred Ag.

The first anode sub-layer may have a thickness in the range of 5 to 200 nm, alternatively 8 to 180 nm, alternatively 8 to 150 nm, alternatively 100 to 150 nm.

The first anode sub-layer may be formed by depositing the first metal via vacuum thermal evaporation.

It is to be understood that the first anode layer is not part of the substrate.

Second anode sub-layer

According to one embodiment, wherein the transparent conductive oxide may be selected from the group comprising indium tin oxide (ITO) or indium zinc oxide (IZO), more preferred indium tin oxide (ITO). preferably ITO or IZO.

The first anode sub-layer may have a thickness in the range of 3 to 200 nm, alternatively 3 to 180 nm, alternatively 3 to 150 nm, alternatively 3 to 20 nm.

The second anode sub-layer may be formed by sputtering of the transparent conductive oxide.

Third anode sub-layer

According to one embodiment the anode layer of the organic electroluminescent device may comprise at least three anode sub-layers of a first anode sub-layer, a second anode sub-layer and third anode sub-layer. According to one embodiment the anode layer of the organic electroluminescent device may comprise in addition to the first and second anode sub-layers a third anode sub-layer, wherein the third anode sub-layer comprises a transparent conductive oxide, wherein the third anode sub-layer may be arranged between the substrate and the first anode sub-layer.

The third anode sub-layer may have a thickness in the range of 3 to 200 nm, alternatively 3 to 180 nm, alternatively 3 to 150 nm, alternatively 3 to 20 nm.

The third anode sub-layer may be formed by sputtering of the transparent conductive oxide.

It is to be understood that the third anode layer is not part of the substrate.

Hole injection layer

A hole injection layer (HIL) may be formed on the anode electrode by vacuum deposition, spin coating, printing, casting, slot-die coating, Langmuir-Blodgett (LB) deposition, or the like. When the HIL is formed using vacuum deposition, the deposition conditions may vary according to the compound that is used to form the HIL, and the desired structure and thermal properties of the HIL. In general, however, conditions for vacuum deposition may include a deposition temperature of 100° C to 500° C, a pressure of $10^{-8}$ to $10^{-3}$ Torr (1 Torr equals 133.322 Pa), and a deposition rate of 0.1 to 10 nm/sec.

When the HIL is formed using spin coating or printing, coating conditions may vary according to the compound that is used to form the HIL, and the desired structure and thermal properties of the HIL. For example, the coating conditions may include a coating speed of about 2000 rpm to about 5000 rpm, and a thermal treatment temperature of about 80° C to about 200° C. Thermal treatment removes a solvent after the coating is performed.

The HIL may be formed of any compound that is commonly used to form a HIL. Examples of compounds that may be used to form the HIL include a phthalocyanine compound, , preferably copper phthalocyanine (CuPc), 4,4',4"-tris (3-methylphenylphenylamino) triphenylamine (m-MTDATA), TDATA, 2T-NATA, polyaniline/dodecyl benzenesulfonic acid (Pani/DBSA), poly(3,4-ethylenedioxythiophene)/poly(4-styrenesulfonate) (PEDOT/PSS), polyaniline/camphor sulfonic acid (Pani/CSA), and polyaniline)/poly(4-styrenesulfonate (PANI/PSS).

The HIL may comprise or consist of p-type dopant and the p-type dopant may be selected from tetrafluorotetracyanoquinonedimethane (F4TCNQ), 2,2'-(perfluoronaphthalen-2,6-diylidene) dimalononitrile or 2,2',2"-(cyclopropane-1,2,3-triylidene)tris(2-(p-cyanotetrafluorophenyl)acetonitrile) but not limited hereto. The HIL may be selected from a hole-transporting matrix compound doped with a p-type dopant. Typical examples of known doped hole transport materials are: copper phthalocyanine (CuPc), which HOMO level is approximately -5.2 eV, doped with tetrafluorotetracyanoquinonedimethane (F4TCNQ), which LUMO level is about -5.2 eV; zinc phthalocyanine (ZnPc) (HOMO = -5.2 eV) doped with F4TCNQ; α-NPD (N,N'-Bis(naphthalen-1-yl)-N,N'-bis(phenyl)-benzidine) doped with F4TCNQ. α-NPD doped with 2,2'-(perfluoronaphthalen-2,6-diylidene) dimalononitrile. The p-type dopant concentrations can be selected from 1 to 20 wt.-%, more preferably from 3 wt.-% to 10 wt.-%.

The thickness of the HIL may be in the range from about 1 nm to about 100 nm, and for example, from about 1 nm to about 25 nm. When the thickness of the HIL is within this range, the HIL may have excellent hole injecting characteristics, without a substantial penalty in driving voltage.

According to one embodiment of the organic electronic device, wherein the hole injection layer may comprise

a first hole injection sub-layer comprising the metal compound according to the invention and a second hole injection sub-layer comprising the covalent matrix compound or substantially covalent matrix compound.

According to another embodiment of the organic electronic device, wherein the hole injection layer may comprise a first hole injection sub-layer comprising the metal compound according to the invention and a second hole injection sub-layer comprising the covalent matrix compound or substantially covalent matrix compound, wherein the first hole injection sub-layer is arranged closer to the anode layer comprising a first anode sub-layer and a second anode sub-layer and the second hole injection sub-layer is arranged closer to the at least one light emitting layer.

According to one embodiment of the organic electronic device, wherein the hole injection layer may comprise a first hole injection sub-layer comprising the metal compound according to the invention and a second hole injection sub-layer comprising a matrix compound comprising of at least one arylamine compound, diarylamine compound, triarylamine compound, wherein the first hole injection sub-layer is arranged closer to the anode layer and the second hole injection sub-layer is arranged closer to the at least one light emitting layer.

According to one embodiment of the organic electronic device, wherein the hole injection layer may comprise a first hole injection sub-layer comprising the metal compound according to the invention and a second hole injection sub-layer comprising the matrix compound comprising of at least one arylamine compound, diarylamine compound, triarylamine compound, wherein the first hole injection sub-layer is arranged closer to the anode layer comprising a first anode sub-layer and a second anode sub-layer and the second hole injection sub-layer is arranged closer to the at least one light emitting layer.

## Hole transport layer

A hole transport layer (HTL) may be formed on the HIL by vacuum deposition, spin coating, slot-die coating, printing, casting, Langmuir-Blodgett (LB) deposition, or the like. When the HTL is formed by vacuum deposition or spin coating, the conditions for deposition and coating may be similar to those for the formation of the HIL. However, the conditions for the vacuum or solution deposition may vary, according to the compound that is used to form the HTL.

In one embodiment, wherein the organic electroluminescent device further comprises a hole transport layer, wherein the hole transport layer is arranged between the semiconductor layer and the at least one photoactive layer.

In one embodiment, wherein the hole transport layer comprises a covalent matrix compound or substantially covalent matrix compound.

In one embodiment, wherein the at least one semiconductor layer and the hole transport layer comprise a covalent matrix compound or substantially covalent matrix compound, wherein the covalent matrix compound or substantially covalent matrix compound is selected the same in both layers.

The thickness of the HTL may be in the range of about 5 nm to about 250 nm, preferably, about 10 nm to about 200 nm, further about 20 nm to about 190 nm, further about 40 nm to about 180 nm, further about 60 nm to about 170 nm, further about 80 nm to about 160 nm, further about 100 nm to about 160 nm, further about 120 nm to about 140 nm.

When the thickness of the HTL is within this range, the HTL may have excellent hole transporting characteristics, without a substantial penalty in driving voltage.

## Electron blocking layer

The function of an electron blocking layer (EBL) is to prevent electrons from being transferred from a light emitting layer to the hole transport layer and thereby confine electrons to the light emitting layer. Thereby, efficiency, operating voltage and/or lifetime may be improved. Typically, the electron blocking layer comprises a triarylamine compound. The triarylamine compound may have a LUMO level closer to vacuum level than the LUMO level of the hole transport layer. The electron blocking layer may have a HOMO level that is further away from vacuum level compared to the HOMO level of the hole transport layer. The thickness of the electron blocking layer may be selected between 2 and 20 nm.

If the electron blocking layer has a high triplet level, it may also be described as triplet control layer.

The function of the triplet control layer is to reduce quenching of triplets if a phosphorescent green or blue light emitting layer is used. Thereby, higher efficiency of light emission from a phosphorescent light emitting layer can be achieved. The triplet control layer is selected from triarylamine compounds with a triplet level above the triplet level of the phosphorescent emitter in the adjacent light emitting layer. Suitable compounds for the triplet control layer, in particular the triarylamine compounds, are described in EP 2 722 908 A1.

## Photoactive layer (PAL)

The photoactive layer converts an electrical current into photons or photons into an electrical current.

The PAL may be formed on the HTL by vacuum deposition, spin coating, slot-die coating, printing, casting, LB

deposition, or the like. When the PAL is formed using vacuum deposition or spin coating, the conditions for deposition and coating may be similar to those for the formation of the HIL. However, the conditions for deposition and coating may vary, according to the compound that is used to form the PAL.

It may be provided that the photoactive layer does not comprise the metal compound of the present invention.

Light emitting layer (EML)

The EML may be formed on the HTL by vacuum deposition, spin coating, slot-die coating, printing, casting, LB deposition, or the like. When the EML is formed using vacuum deposition or spin coating, the conditions for deposition and coating may be similar to those for the formation of the HIL. However, the conditions for deposition and coating may vary, according to the compound that is used to form the EML.

It may be provided that the light emitting layer does not comprise the metal compound as described in the present invention.

The light emitting layer (EML) may be formed of a combination of a host and an emitter dopant. Example of the host are Alq3, 4,4'-N,N'-dicarbazole-biphenyl (CBP), poly(n-vinylcarbazole) (PVK), 9,10-di(naphthalene-2-yl)anthracene (ADN), 4,4',4"-tris(carbazol-9-yl)-triphenylamine(TCTA), 1,3,5-tris(N-phenylbenzimidazole-2-yl)benzene (TPBI), 3-tert-butyl-9,10-di-2-naphthylanthracenee (TBADN), distyrylarylene (DSA) and bis(2-(2-hydroxyphenyl)benzo-thiazolate)zinc (Zn(BTZ)2).

The emitter dopant may be a phosphorescent or fluorescent emitter. Phosphorescent emitters and emitters which emit light via a thermally activated delayed fluorescence (TADF) mechanism may be preferred due to their higher efficiency. The emitter may be a small molecule or a polymer.

Examples of red emitter dopants are PtOEP, Ir(piq)3, and Btp2lr(acac), but are not limited thereto. These compounds are phosphorescent emitters; however, fluorescent red emitter dopants could also be used.

Examples of phosphorescent green emitter dopants are Ir(ppy)3 (ppy = phenylpyridine), Ir(ppy)2(acac), Ir(mpyp)3.

Examples of phosphorescent blue emitter dopants are F2Irpic, (F2ppy)2Ir(tmd) and Ir(dfppz)3 and ter-fluorene. 4.4'-bis(4-diphenyl amiostyryl)biphenyl (DPAVBi), 2,5,8,11-tetra-tert-butyl perylene (TBPe) are examples of fluorescent blue emitter dopants.

The amount of the emitter dopant may be in the range from about 0.01 to about 50 parts by weight, based on 100 parts by weight of the host. Alternatively, the light emitting layer may consist of a light-emitting polymer. The EML may have a thickness of about 10 nm to about 100 nm, for example, from about 20 nm to about 60 nm. When the thickness of the EML is within this range, the EML may have excellent light emission, without a substantial penalty in driving voltage.

Hole blocking layer (HBL)

A hole blocking layer (HBL) may be formed on the EML, by using vacuum deposition, spin coating, slot-die coating, printing, casting, LB deposition, or the like, in order to prevent the diffusion of holes into the ETL. When the EML comprises a phosphorescent dopant, the HBL may have also a triplet exciton blocking function.

The HBL may also be named auxiliary ETL or a-ETL.

When the HBL is formed using vacuum deposition or spin coating, the conditions for deposition and coating may be similar to those for the formation of the HIL. However, the conditions for deposition and coating may vary, according to the compound that is used to form the HBL. Any compound that is commonly used to form a HBL may be used. Examples of compounds for forming the HBL include oxadiazole derivatives, triazole derivatives, phenanthroline derivatives and triazine derivatives.

The HBL may have a thickness in the range from about 5 nm to about 100 nm, for example, from about 10 nm to about 30 nm. When the thickness of the HBL is within this range, the HBL may have excellent hole-blocking properties, without a substantial penalty in driving voltage.

Electron transport layer (ETL)

The organic electroluminescent device according to the present invention may further comprise an electron transport layer (ETL).

According to another embodiment, the electron transport layer may further comprise an azine compound, preferably a triazine compound.

In one embodiment, wherein the electron transport layer may further comprise a dopant selected from an alkali organic complex, preferably LiQ.

The thickness of the ETL may be in the range from about 15 nm to about 50 nm, for example, in the range from

about 20 nm to about 40 nm. When the thickness of the EIL is within this range, the ETL may have satisfactory electron-injecting properties, without a substantial penalty in driving voltage.

According to another embodiment, the organic electroluminescent device may further comprise a hole blocking layer and an electron transport layer, wherein the hole blocking layer and the electron transport layer comprise an azine compound. Preferably, the azine compound is a triazine compound.

Electron injection layer (EIL)

An optional EIL, which may facilitate injection of electrons from the cathode, may be formed on the ETL, preferably directly on the electron transport layer. Examples of materials for forming the EIL include lithium 8-hydroxyquinolinolate (LiQ), LiF, NaCl, CsF, Li2O, BaO, Ca, Ba, Yb, Mg which are known in the art. Deposition and coating conditions for forming the EIL are similar to those for formation of the HIL, although the deposition and coating conditions may vary, according to the material that is used to form the EIL.

The thickness of the EIL may be in the range from about 0.1 nm to about 10 nm, for example, in the range from about 0.5 nm to about 9 nm. When the thickness of the EIL is within this range, the EIL may have satisfactory electron-injecting properties, without a substantial penalty in driving voltage.

Cathode electrode

The cathode electrode, also named cathode layer, is formed on the ETL or optional EIL. The cathode electrode may be formed of a metal, an alloy, an electrically conductive compound, or a mixture thereof. The cathode electrode may have a low work function. For example, the cathode electrode may be formed of lithium (Li), magnesium (Mg), aluminum (Al), aluminum (Al)-lithium (Li), calcium (Ca), barium (Ba), ytterbium (Yb), magnesium (Mg)-indium (In), magnesium (Mg)-silver (Ag), or the like. Alternatively, the cathode electrode may be formed of a transparent conductive oxide, , preferably ITO or IZO.

The thickness of the cathode electrode may be in the range from about 5 nm to about 1000 nm, for example, in the range from about 10 nm to about 100 nm. When the thickness of the cathode electrode is in the range from about 5 nm to about 50 nm, the cathode electrode may be transparent or semitransparent even if formed from a metal or metal alloy.

It is to be understood that the cathode electrode is not part of an electron injection layer or the electron transport layer.

Organic electroluminescent device (OLED)

The organic electroluminescent device according to the invention may be an organic light-emitting device.

According to one aspect, there is provided an organic electroluminescent device (OLED) comprising: a substrate; an anode electrode formed on the substrate; a semiconductor layer comprising the metal compound as described in the present invention, a hole transport layer, a light emitting layer, an electron transport layer and a cathode electrode.

According to another aspect, there is provided an OLED comprising: a substrate; an anode electrode formed on the substrate; a semiconductor layer comprising a metal compound as described in the present invention, a hole transport layer, an electron blocking layer, a light emitting layer, a hole blocking layer, an electron transport layer and a cathode electrode.

According to another aspect, there is provided an OLED comprising: a substrate; an anode electrode formed on the substrate; a semiconductor layer comprising a metal compound as described in the present invention, a hole transport layer, an electron blocking layer, a light emitting layer, a hole blocking layer, an electron transport layer, an electron injection layer, and a cathode electrode.

According to various embodiments , there may be provided OLEDs layers arranged between the above-mentioned layers, on the substrate or on the top electrode.

According to one aspect, the OLED may comprise a layer structure of a substrate that is adjacent arranged to an anode electrode, the anode electrode is adjacent arranged to a first hole injection layer, the first hole injection layer is adjacent arranged to a first hole transport layer, the first hole transport layer is adjacent arranged to a first electron blocking layer, the first electron blocking layer is adjacent arranged to a first light emitting layer, the first light emitting layer is adjacent arranged to a first electron transport layer, the first electron transport layer is adjacent arranged to an n-type charge generation layer, the n-type charge generation layer is adjacent arranged to a hole generating layer, the hole generating layer is adjacent arranged to a second hole transport layer, the second hole transport layer is adjacent arranged to a second electron blocking layer, the second electron blocking layer is adjacent arranged to a second light emitting layer, between the second light emitting layer and the cathode electrode an optional electron transport layer and/or an optional injection layer are arranged.

The semiconductor layer according to the invention may be the first hole injection layer and p-type charge generation layer.

For example, the OLED according to Fig. 7 may be formed by a process, wherein on a substrate (110), an anode layer (120) that comprises a first anode sub-layer (121) and a second anode sub-layer (122) and a hole injection layer (HIL) (130). The HIL (130) is disposed on the anode layer (120). Onto the HIL (130), a hole transport layer (HTL) (140), an electron blocking layer (EBL) (145), a first light emitting layer (EML) (150), a hole blocking layer (HBL) (155), an electron transport layer (ETL) (160), and a cathode layer (190) are subsequently formed in that order.

Organic electronic device

The organic electroluminescent device according to the invention may be a light emitting device, or a photovoltaic cell, and preferably a light emitting device.

The present invention furthermore relates to a display device comprising an organic electroluminescent device according to the present invention.

According to one embodiment, wherein organic electronic device comprises an organic electroluminescent device , preferably the organic electronic device is a display device or an electroluminescent device.

According to another aspect, there is provided a method of manufacturing an organic electronic device, the method using:

- at least one deposition source, preferably two deposition sources and more preferred at least three deposition sources.

The methods for deposition that can be suitable used comprise:

- deposition via vacuum thermal evaporation;
- deposition via solution processing, preferably the processing is selected from spin-coating, printing, casting; and/or
- slot-die coating.

According to various embodiments , there is provided a method using:

- a first deposition source to release the metal compound as described in the present invention according to the invention, and
- a second deposition source to release the covalent matrix compound or substantially covalent matrix compound;

the method comprising the steps of forming the semiconductor layer; whereby for an organic electroluminescent device (OLED):

- the semiconductor layer is formed by releasing the metal compound as described in the present invention according to the invention from the first deposition source and the covalent matrix compound or substantially covalent matrix compound from the second deposition source.

According to various embodiments , the method may further include forming on the anode electrode, at least one layer selected from the group consisting of forming a hole transport layer or forming a hole blocking layer, and a light emitting layer between the anode electrode and the first electron transport layer.

According to various embodiments , the method may further include the steps for forming an organic electroluminescent device (OLED), wherein

- on a substrate an anode electrode is formed,
- on the anode electrode a semiconductor layer comprising a metal compound as described in the present invention is formed,
- on the semiconductor layer comprising a metal compound as described in the present invention a hole transport layer is formed,
- on the hole transport layer a light emitting layer is formed,
- on the light emitting layer an electron transport layer is formed, optionally a hole blocking layer is formed on the light emitting layer,
- and finally, a cathode electrode is formed,
- optional a hole blocking layer is formed in that order between the first anode electrode and the light emitting layer,
- optional an electron injection layer is formed between the electron transport layer and the cathode electrode.

According to various embodiments, the OLED may have the following layer structure, wherein the layers having the following order:

anode, semiconductor layer comprising a metal compound as described in the present invention according to the invention, first hole transport layer, second hole transport layer, light emitting layer, optional hole blocking layer, electron transport layer, optional electron injection layer, and cathode.

According to another aspect of the invention, it is provided an electronic device comprising at least one organic light emitting device according to any embodiment described throughout this application, preferably, the electronic device comprises the organic electroluminescent device in one of embodiments described throughout this application. More preferably, the electronic device is a display device.

Hereinafter, the embodiments are illustrated in more detail with reference to examples. However, the present disclosure is not limited to the following examples. Reference will now be made in detail to the exemplary aspects.

## Description of the Drawings

The aforementioned components, as well as the claimed components and the components to be used in accordance with the invention in the described embodiments, are not subject to any special exceptions with respect to their size, shape, material selection and technical concept such that the selection criteria known in the pertinent field can be applied without limitations.

Additional details, characteristics and advantages of the object of the invention are disclosed in the dependent claims and the following description of the respective figures which in an exemplary fashion show preferred embodiment according to the invention. Any embodiment does not necessarily represent the full scope of the invention, however, and reference is made therefore to the claims and herein for interpreting the scope of the invention. It is to be understood that both the foregoing general description and the following detailed description are exemplary and explanatory only and are intended to provide further explanation as claimed.

<u>Figures 1 to 7</u>

FIG. 1 is a schematic sectional view of an organic electronic device, according to an exemplary embodiment of the present invention;

FIG. 2 is a schematic sectional view of an organic electronic device, according to an exemplary embodiment of the present invention;

FIG. 3 is a schematic sectional view of an organic electronic device, according to an exemplary embodiment of the present invention.

FIG. 4 is a schematic sectional view of an organic electronic device, according to an exemplary embodiment of the present invention;

FIG. 5 is a schematic sectional view of an organic electronic device, according to an exemplary embodiment of the present invention;

FIG. 6 is a schematic sectional view of an organic electronic device, according to an exemplary embodiment of the present invention;

FIG. 7 is a schematic sectional view of an organic electronic device, according to an exemplary embodiment of the present invention.

Hereinafter, the figures 1 to 7 are illustrated in more detail with reference to examples. However, the present disclosure is not limited to the following figures.

Herein, when a first element is referred to as being formed or disposed "on" or "onto" a second element, the first element can be disposed directly on the second element, or one or more other elements may be disposed there between. When a first element is referred to as being formed or disposed "directly on" or "directly onto" a second element, no other elements are disposed there between.

FIG. 1 is a schematic sectional view of an organic electronic device (100), according to an exemplary embodiment of the present invention. The organic electronic device (100) includes a substrate (110), an anode layer (120) and a hole injection layer (HIL) (130). The HIL (130) is disposed on the anode layer (120). Onto the HIL (130), a hole transport layer (140), a first emission layer (EML) (150), and a cathode layer (190) are disposed.

FIG. 2 is a schematic sectional view of an organic electronic device (100), according to an exemplary embodiment

of the present invention. The organic electronic device (100) includes a substrate (110), an anode layer (120) that comprises a first anode sub-layer (121) and a second anode sub-layer (122), and a hole injection layer (HIL) (130). The HIL (130) is disposed on the anode layer (120) comprising a first anode sub-layer (121) and the second anode sub-layer (122) . Onto the HIL (130), a hole transport layer (140), a first emission layer (EML) (150), and a cathode layer (190) are disposed.

FIG. 3 is a schematic sectional view of an organic electronic device (100), according to an exemplary embodiment of the present invention. The organic electronic device (100) includes a substrate (110), an anode layer (120) that comprises a first anode sub-layer (121), a second anode sub-layer (122) and a third anode sub-layer (123), and a hole injection layer (HIL) (130). The HIL (130) is disposed on the anode layer (120). Onto the HIL (130), a hole transport layer (140), a first emission layer (EML) (150), and a cathode layer (190) are disposed.

FIG. 4 is a schematic sectional view of an organic electronic device (100), according to an exemplary embodiment of the present invention. The organic electronic device (100) includes a substrate (110), an anode layer (120) that comprises a first anode sub-layer (121) and a second anode sub-layer (122), and a hole injection layer (HIL) (130). The HIL (130) is disposed on the anode layer (120). Onto the HIL (130), an hole transport layer (HTL) (140), a first emission layer (EML) (150), a hole blocking layer (HBL) (155), an electron transport layer (ETL) (160), and a cathode layer (190) are disposed.

FIG. 5 is a schematic sectional view of an organic electronic device (100), according to an exemplary embodiment of the present invention. The organic electronic device (100) includes a substrate (110), an anode layer (120) that comprises a first anode sub-layer (121), a second anode sub-layer (122) and a third anode sub-layer (123), and a hole injection layer (HIL) (130). The HIL (130) is disposed on the anode layer (120). Onto the HIL (130), a hole transport layer (HTL) (140), a first emission layer (EML) (150), a hole blocking layer (HBL) (155), an electron transport layer (ETL) (160), and a cathode layer (190) are disposed.

FIG. 6 is a schematic sectional view of an organic electronic device (100), according to an exemplary embodiment of the present invention. The organic electronic device (100) includes a substrate (110), an anode layer (120) that comprises a first anode sub-layer (121), a second anode sub-layer (122) and a third anode sub-layer (123), and a hole injection layer (HIL) (130). The HIL (130) is disposed on the anode layer (120). Onto the HIL (130), a hole transport layer (HTL) (140), an electron blocking layer (EBL) (145), a first emission layer (EML) (150), a hole blocking layer (HBL) (155), an electron transport layer (ETL) (160), and a cathode layer (190) are disposed.

FIG. 7 is a schematic sectional view of an organic electronic device (100), according to an exemplary embodiment of the present invention. The organic electronic device (100) includes a substrate (110), an anode layer (120) that comprises a first anode sub-layer (121), a second anode sub-layer (122) and a third anode sub-layer (123), and a hole injection layer (HIL) (130). The HIL (130) is disposed on the anode layer (120). Onto the HIL (130), a hole transport layer (HTL) (140), an electron blocking layer (EBL) (145), a first emission layer (EML) (150), a hole blocking layer (HBL) (155), an electron transport layer (ETL) (160), an electron injection layer (180) and a cathode layer (190) are disposed.

While not shown in Fig. 1 to Fig. 7, a capping and/or a sealing layer may further be formed on the cathode layer 190, in order to seal the organic electronic device 100. In addition, various other modifications may be applied thereto.

Hereinafter, the embodiments are illustrated in more detail with reference to examples. However, the present disclosure is not limited to the following examples.

**Detailed description**

The invention is furthermore illustrated by the following examples which are illustrative only and non-binding.

The compound may be prepared as described in the literature or alternative compounds may be prepared following similar compounds as described in the literature.

Rate onset temperature

The rate onset temperature ($T_{RO}$) is determined by loading 100 mg compound into a VTE source. As VTE source a point source for organic materials may be used as supplied by Kurt J. Lesker Company (www.lesker.com) or CreaPhys GmbH (http://www.creaphys.com). The VTE source is heated at a constant rate of 15 K/min at a pressure of less than $10^{-5}$ mbar and the temperature inside the source measured with a thermocouple. Evaporation of the compound is detected with a QCM detector which detects deposition of the compound on the quartz crystal of the detector. The deposition rate on the quartz crystal is measured in Angstrom per second. To determine the rate onset temperature, the deposition rate is plotted against the VTE source temperature. The rate onset is the temperature at which noticeable deposition on the QCM detector occurs. For accurate results, the VTE source is heated and cooled three time and only results from the second and third run are used to determine the rate onset temperature.

To achieve good control over the evaporation rate of an organic compound, the rate onset temperature may be in the range of 200 to 255 °C. If the rate onset temperature is below 200 °C the evaporation may be too rapid and therefore difficult to control. If the rate onset temperature is above 255 °C the evaporation rate may be too low which may result in low tact time and decomposition of the organic compound in VTE source may occur due to prolonged exposure to elevated temperatures.

The rate onset temperature is an indirect measure of the volatility of a compound. The higher the rate onset temperature the lower is the volatility of a compound.

Reduction potential

The reduction potential is determined by cyclic voltammetry with potenioststic device Metrohm PGSTAT30 and software Metrohm Autolab GPES at room temperature. The redox potentials given at particular compounds were measured in an argon de-aerated, dry 0.1M THF solution of the tested substance, under argon atmosphere, with 0.1M tetrabutylammonium hexafluorophosphate supporting electrolyte, between platinum working electrodes and with an Ag/AgCl pseudo-standard electrode (Metrohm Silver rod electrode), consisting of a silver wire covered by silver chloride and immersed directly in the measured solution, with the scan rate 100 mV/s. The first run was done in the broadest range of the potential set on the working electrodes, and the range was then adjusted within subsequent runs appropriately. The final three runs were done with the addition of ferrocene (in 0.1M concentration) as the standard. The average of potentials corresponding to cathodic and anodic peak of the studied compound, after subtraction of the average of cathodic and anodic potentials observed for the standard $Fc^+/Fc$ redox couple, afforded finally the values reported above. All studied compounds as well as the reported comparative compounds showed well-defined reversible electrochemical behavior.

Calculated HOMO and LUMO

The HOMO and LUMO are calculated with the program package TURBOMOLE V6.5 (TURBOMOLE GmbH, Litzenhardtstrasse 19, 76135 Karlsruhe, Germany). The optimized geometries and the HOMO and LUMO energy levels of the molecular structures are determined by applying the hybrid functional B3LYP with a 6-31G* basis set in the gas phase. If more than one conformation is viable, the conformation with the lowest total energy is selected. The HOMO and LUMO levels are recorded in electron volt (eV).

Matrix compounds in the hole injection layer and/or hole transport layer

In Table 2 are shown the HOMO levels and rate onset temperatures $T_{RO}$ for matrix compounds. HOMO levels were calculated using TURBOMOLE V6.5 (TURBOMOLE GmbH, Litzenhardtstrasse 19, 76135 Karlsruhe, Germany) by applying the hybrid functional B3LYP with a 6-31G* basis set in the gas phase.

Table 3: Matrix compounds for hole injection layers

| Name | Structure | HOMO level (eV) | $T_{RO}$ (°C) |
|---|---|---|---|
| Biphenyl-4-yl(9,9-diphenyl-9H-fluoren-2-yl)-[4-(9-phenyl-9H-carbazol-3-yl)phenyl]-amine<br>F3 | 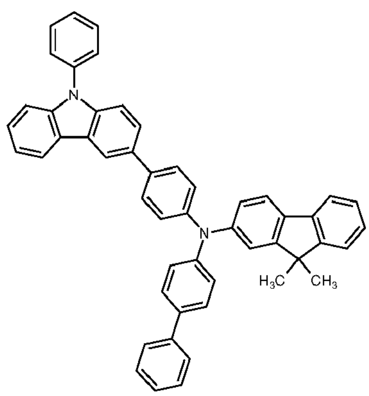 | -4.69 | 265 |

(continued)

| Name | Structure | HOMO level (eV) | $T_{RO}$ (°C) |
|---|---|---|---|
| N,N'-Bis(naphthalen-1-yl)-N,N'-bis(phenyl)-benzidine F1 | 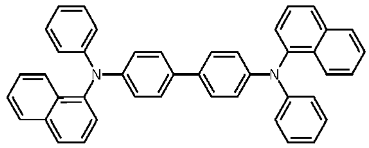 | -4.72 | 272 |
| N4,N4"-di(naphthalen-1-yl)-N4,N4"-diphenyl-[1,1':4',1"-terphenyl]-4,4"-diamine (CAS 139255-16-6) F2 | 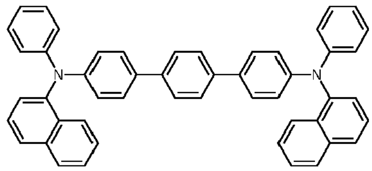 | -4.81 | 260 |
| N-(9,9-dimethyl-9H-fluoren-2-yl)-N-(9,9-diphenyl-9H-fluoren-2-yl)dibenzo[b,d]furan-1-amine F4 | 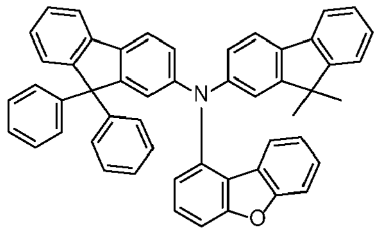 | -4.82 | 219 |
| 9,9-dimethyl-N,N-bis(4-(naphthalen-1-yl)phenyl)-9H-fluoren-2-amine F8 | 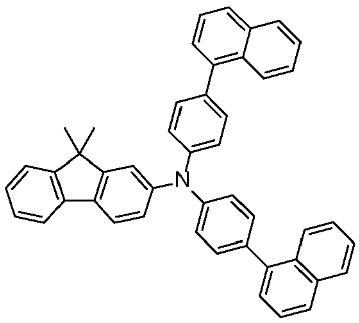 | -4.84 | 232 |
| N-([1,1'-biphenyl]-4-yl)-N-(2-(9,9-diphenyl-9H-fluoren-4-yl)phenyl)-9,9-dimethyl-9H-fluoren-2-amine F9 | 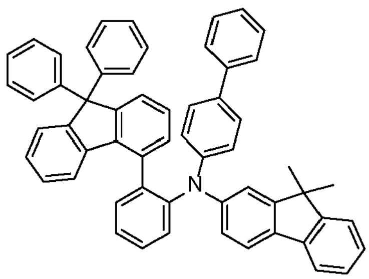 | -4.84 | 218 |
| N-(9,9-diphenyl-9H-fluoren-2-yl)-N,9-diphenyl-9H-carbazol- 2-amine F18 | 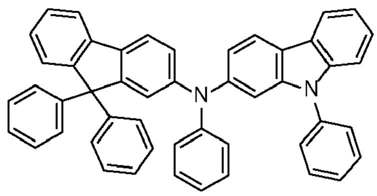 | -4.73 | 216 |

As can be seen in Table 3, the matrix compounds have rate onset temperatures suitable for mass production of organic electronic devices.

## Experimental data

Synthesis of 4-(2,4-dioxopentan-3-yl)-2,6-bis(trifluoromethyl)benzonitrile (Ligand) 28.6g (87.7mmol) caesium carbonate was suspended in 100mL dry DMF under inert conditions. 4.5mL (43.9mmol) acetylacetone and 10g (36.6mmol) of 4-chloro-2,6-bis(trifluoromethyl)benzonitrile were added at room temperature. The mixture was heated to 60°C for 20h. The solid was filtered off and washed with ethyl acetate. The mother liquor was added to 250mL 2N hydrochloric acid. After phase separation, the aqueous layer was extracted with ethyl acetate and the combined organic layers were washed with brine, dried over magnesium sulphate and the solvent removed under reduced pressure. The crude product was slurry washed with methanol/water and ethyl acetate/hexane to obtain 8.7g (71% yield) product as an off-white, crystalline solid.

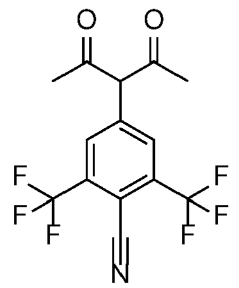

4-(2,4-dioxopentan-3-yl)-2,6-bis(trifluoromethyl)benzonitrile

Synthesis of bis(((Z)-3-(4-cyano-3,5-bis(trifluoromethyl)phenyl)-4-oxopent-2-en-2-yl) oxy)copper 3.0g (8.9mmol) of 4-(2,4-dioxopentan-3-yl)-2,6-bis(trifluoromethyl)benzonitrile were dissolved in 20ml acetonitrile and 0.89g (4.45mmol) copper(II) acetate monohydrate were added. The mixture was stirred at room temperature for one hour. 30ml water were added and the precipitate filtered off, washed with water and hexane and dried in high vacuum. 3.15g (96%) product was obtained as a greyish-violet powder.

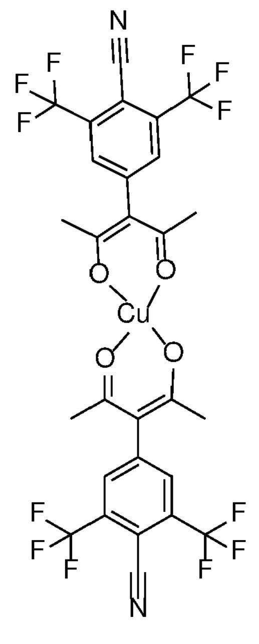

bis(((Z)-3-(4-cyano-3,5-bis(trifluoromethyl)phenyl)-4-oxopent-2-en-2-yl)oxy)copper

General procedure for fabrication of OLEDs

For Examples 1 and 2 and comparative example 1 according to Table 2, a glass substrate with an anode layer comprising a first anode sub-layer of 120 nm Ag, a second anode sub-layer of 8 nm ITO and a third anode sub-layer of 10 nm ITO was cut to a size of 50 mm x 50 mm x 0.7 mm, ultrasonically washed with water for 60 minutes and then with isopropanol for 20 minutes. The liquid film was removed in a nitrogen stream, followed by plasma treatment, see Table

3, to prepare the anode layer. The plasma treatment was performed in an atmosphere comprising 97.6 vol.-% nitrogen and 2.4 vol.-% oxygen.

Then, N-([1,1'-biphenyl]-4-yl)-9,9-dimethyl-N-(4-(9-phenyl-9H-carbazol-3-yl)phenyl)-9H-fluoren-2-amine (F3) as a substantially covalent matrix compound and a metal compound according to the present invention such as bis(((Z)-3-(4-cyano-3,5-bis(trifluoromethyl)phenyl)-4-oxopent-2-en-2-yl)oxy)copper or La(FOD)3 as comparative example were co-deposited in vacuum on the anode layer, to form a hole injection layer (HIL) having a thickness of 10 nm. The composition of the HIL can be seen in Table 3.

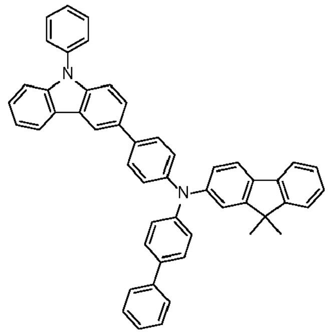

(F3)

Then, the substantially covalent matrix compound (N-([1,1'-biphenyl]-4-yl)-9,9-dimethyl-N-(4-(9-phenyl9H-carbazol-3-yl)phenyl)-9H-fluoren-2-amine was vacuum deposited on the HIL, to form a HTL having a thickness of 128 nm. The formula of the substantially covalent matrix compound in the HTL was identical to the substantially covalent matrix compound used in the HIL.

Then N-([1,1'-biphenyl]-4-yl)-9,9-diphenyl-N-(4-(triphenylsilyl)phenyl)-9H-fluoren-2-amine (CAS 1613079-70-1) (F19) was vacuum deposited on the HTL, to form an electron blocking layer (EBL) having a thickness of 5 nm.

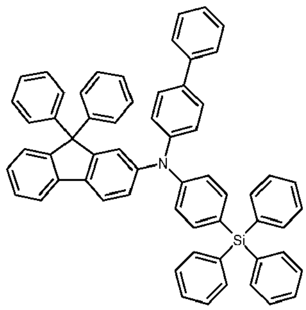

(F19)

Then 97 vol.-% H09 (Sun Fine Chemicals, Korea) as EML host and 3 vol.-% BD200 (Sun Fine Chemicals, Korea) as fluorescent blue emitter dopant were deposited on the EBL, to form a blue-emitting first emission layer (EML) with a thickness of 20 nm.

Then a hole blocking layer was formed with a thickness of 5 nm by depositing 2-(3'-(9,9-dimethyl-9H-fluoren-2-yl)-[1,1'-biphenyl]-3-yl)-4,6-diphenyl-1,3,5-triazine on the emission layer EML.

Then the electron transporting layer having a thickness of 31 nm was formed on the hole blocking layer by depositing 50 wt.-% 4'-(4-(4-(4,6-diphenyl-1,3,5-triazin-2-yl)phenyl)naphthalen-1-yl)-[1,1'-biphenyl]-4-carbonitrile and 50 wt.-% of LiQ.

Then Ytterbium was evaporated at a rate of 0.01 to 1 Å/s at $10^{-7}$ mbar to form an electron injectin layer with a thickness of 2nm on the electron transport layer

Then Ag:Mg (90:10 vol.-%) was evaporated at a rate of 0.01 to 1 Å/s at $10^{-7}$ mbar to form a cathode layer with a thickness of 100 nm on the electron injection layer.

Then, N-([1,1'-biphenyl]-4-yl)-9,9-dimethyl-N-(4-(9-phenyl-9H-carbazol-3-yl)phenyl)-9H-fluoren-2-amine (F3) was deposited on the cathode layer to form a capping layer with a thickness of 75 nm.

The OLED stack is protected from ambient conditions by encapsulation of the device with a glass slide. Thereby, a cavity is formed, which includes a getter material for further protection.

To assess the performance of the inventive examples compared to the prior art, the current efficiency is measured at 20°C. The current-voltage characteristic is determined using a Keithley 2635 source measure unit, by sourcing an operating voltage U in V and measuring the current in mA flowing through the device under test. The voltage applied to the device is varied in steps of 0.1V in the range between 0V and 10V. Likewise, the luminance-voltage characteristics and CIE coordinates are determined by measuring the luminance in cd/$m^2$ using an Instrument Systems CAS-140CT array spectrometer (calibrated by Deutsche Akkreditierungsstelle (DAkkS)) for each of the voltage values.

Lifetime LT of the device is measured at ambient conditions (20°C) and 30 mA/$cm^2$, using a Keithley 2400 sourcemeter, and recorded in hours. The brightness of the device is measured using a calibrated photo diode. The lifetime LT is defined as the time till the brightness of the device is reduced to 97 % of its initial value.

To determine the voltage stability over time U(100h)-(1h), a current density of at 30 mA/$cm^2$ was applied to the device. The operating voltage was measured after 1 hour and after 100 hours, followed by calculation of the voltage stability for the time period of 1 hour to 100 hours.

Technical Effect of the invention

Table 3 shows the technical effect:

Table 3

| | | | | | |
|---|---|---|---|---|---|
| | | 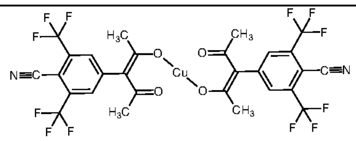 F20 | | 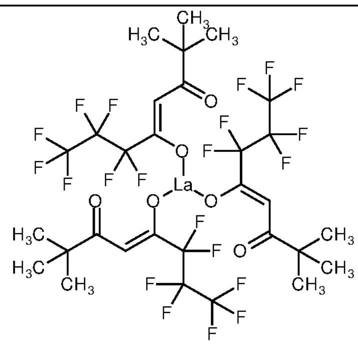 F21 | |
| | | | | For comparative example La(FOD)3 | |
| | Metal complex | Percentage metal complex in semiconductor layer [vol.-%] | U(100 h)-U(1 h) (30mA/ $cm^2$) [V] | LT97 RT (30mA/ $cm^2$) [h] | CEff/ [cd/A] |
| Comparative example 1 | La(fod)$_3$ | 3 | 1.07 | 30 | 142 |
| Comparative example 2 | La(fod)$_3$ | 5 | 0.85 | 24 | 142 |
| Comparative example 3 | La(fod)$_3$ | 10 | 0.89 | 15 | 141 |
| Inventive example 1 | F20 | 5 | 0.293 | 106 | 155,0 |
| Inventive example 2 | F20 | 10 | 0.082 | 87 | 156,1 |
| Inventive example 3 | F20 | 15 | 0.043 | 82 | 156,2 |

In a semiconductor layer comprising a metal compound according to the present invention, preferably according to formula (I), the increase in operating voltage is substantially reduced, the lifetime substantially increased, and the current efficiency substantially increased compared to the state of the art.

A reduced increase in operating voltage over time may be beneficial for improved stability of the electronic device.

An increase in lifetime may be beneficial for improved stability of the electronic device.

A high efficiency may be beneficial for reduced power consumption and improved battery life, in particular in

mobile devices.

The particular combinations of elements and features in the above detailed embodiments are exemplary only; the interchanging and substitution of these teachings with other teachings in this and the patents/applications incorporated by reference are also expressly contemplated. As those skilled in the art will recognize, variations, modifications, and other implementations of what is described herein can occur to those of ordinary skill in the art without departing from the spirit and the scope of the invention as claimed. Accordingly, the foregoing description is by way of example only and is not intended as limiting. In the claims, the word "comprising" does not exclude other elements or steps, and the indefinite article "a" or "an" does not exclude a plurality. The mere fact that certain measures are recited in mutually different dependent claims does not indicate that a combination of these measured cannot be used to advantage. The invention's scope is defined in the following claims and the equivalents thereto. Furthermore, reference signs used in the description and claims do not limit the scope of the invention as claimed.

## Claims

1. An organic electroluminescent device comprising a substrate, an anode layer and a cathode layer, at least one light emitting layer, and at least one semiconductor layer or more, wherein the at least one semiconductor layer comprises at least one metal compound, wherein the metal compound comprises a metal M and at least one ligand L or the metal compound has the formula (I):

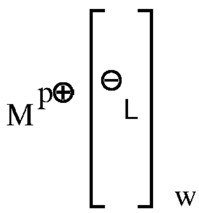

(I),

wherein

M is a metal ion;
p is the valency of M;
w is p
L is a ligand;

wherein the metal compound comprises

- a metal M, and
- at least one ligand L, wherein the at least one ligand L comprises at least one coordinating linker X and at least one ligand fragment Y,
wherein the coordinating linker X and the at least one ligand fragment Y are linked to each other by at least one bond, wherein

- the coordinating linker X is selected from formula (IIa)

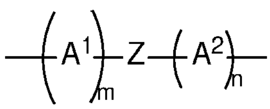

(IIa),

wherein

m and n are independently selected from 0 or 1;
Z is selected from the group comprising $CR^1$, O, N; and
$A^1$ and $A^2$ are independently selected from the group comprising C=O, C-O, $C=NR^2$, $C-NR^3$, SO, $SO_2$, or $P(=O)R^4$;

wherein $A^1$ and $A^2$ together optional form a cycle with Z;

wherein $R^1$, $R^2$, $R^3$ and $R^4$ are independently selected from H, D, CN, substituted or unsubstituted $C_6$ to $C_{24}$ aryl, substituted or unsubstituted $C_2$ to $C_{24}$ heteroaryl, substituted or unsubstituted $C_1$ to $C_{24}$ alkyl, substituted or unsubstituted $C_3$ to $C_{24}$ carbocyclyl, or substituted or unsubstituted $C_2$ to $C_{24}$ heterocyclyl, ligand fragment Y, wherein

the substituents are selected from the group comprising electron-withdrawing group, preferably halogen, F, Cl, CN, perfluorinated $C_1$ to $C_8$ alkyl, perfluorinated $C_6$ to $C_{12}$ aryl, or $C_2$ to $C_{12}$ heteroaryl;

- the at least one ligand fragment Y is independently selected from substituted or unsubstituted $C_6$ to $C_{24}$ aryl, substituted or unsubstituted $C_2$ to $C_{24}$ heteroaryl, substituted or unsubstituted $C_1$ to $C_{24}$ alkyl, substituted or unsubstituted $C_3$ to $C_{24}$ carbocyclyl, substituted or unsubstituted $C_2$ to $C_{24}$ heterocyclyl, perfluorinated $C_1$ to $C_4$ alkyl, substituted $C_6$ to $C_{24}$ aryl in 3-position, substituted $C_6$ to $C_{24}$ aryl in 4-position, substituted 4-pyridyl, or substituted 5-pyrimidyl, wherein
the at least one ligand fragment Y has a directed dipole moment of less than -3.6 debye, and wherein the at least one ligand L is negatively charged; wherein

the anode layer comprises a first anode sub-layer and a second anode sub-layer, wherein

- the first anode sub-layer comprises a first metal having a work function in the range of ≥ 4 and ≤ 6 eV, and
- the second anode sub-layer comprises a transparent conductive oxide (TCO); and the at least one semiconductor layer is arranged between the first light emitting layer and the anode layer,
- the first anode sub-layer is arranged closer to the substrate, and
- the second anode sub-layer is arranged closer to the at least one semiconductor layer;

wherein the following ligand fragments Y are excluded:

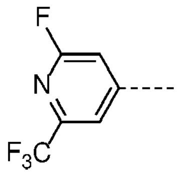, 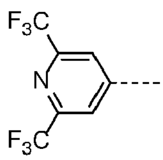, 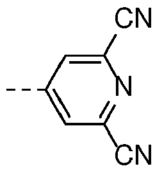, 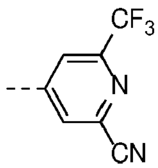, 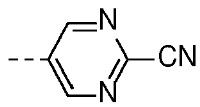,

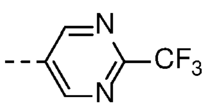, 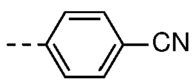, 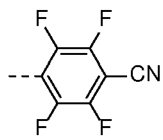 and 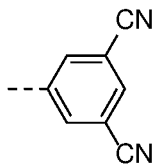.

2. The organic electroluminescent device according to claim 1, wherein the organic electroluminescent device comprising a substrate, an anode layer and a cathode layer, at least one light emitting layer, and at least one semiconductor layer or more, wherein
the at least one or more semiconductor layer comprises a hole injection layer or, a hole injection layer and a hole transport layer, comprising at least one metal compound, wherein the metal compound comprises a metal M and

at least one ligand L or the metal compound has the formula (I):

$$M^{p\oplus}\left[{}^{\ominus}L\right]_w \quad (I),$$

wherein

M is a metal ion;
p is the valency of M;
w is p

L is a ligand;

wherein the metal compound comprises

- a metal M, and
- at least one ligand L, wherein the at least one ligand L comprises a coordinating linker X and at least one ligand fragment Y,

wherein the coordinating linker X and the at least one ligand fragment Y are linked to each other by at least one bond, wherein

- the coordinating linker X is selected from formula (IIa)

$$-\left(A^1\right)_m-Z-\left(A^2\right)_n- \quad \text{(IIa)},$$

wherein

m and n are independently selected from 0 or 1;
Z is selected from the group comprising $CR^1$, O, N; and
$A^1$ and $A^2$ are independently selected from the group comprising C=O, C-O, $C=NR^2$, $C-NR^3$, SO, $SO_2$, or $P(=O)R^4$;
wherein $A^1$ and $A^2$ together optional form a cycle with Z;
wherein $R^1$, $R^2$, $R^3$ and $R^4$ are independently selected from H, D, CN, substituted or unsubstituted $C_6$ to $C_{24}$ aryl, substituted or unsubstituted $C_2$ to $C_{24}$ heteroaryl, substituted or unsubstituted $C_1$ to $C_{24}$ alkyl, substituted or unsubstituted $C_3$ to $C_{24}$ carbocyclyl, or substituted or unsubstituted $C_2$ to $C_{24}$ heterocyclyl, ligand fragment Y, wherein
the substituents are selected from the group comprising electron-withdrawing group, preferably halogen, F, Cl, CN, perfluorinated $C_1$ to $C_8$ alkyl, perfluorinated $C_6$ to $C_{12}$ aryl, or $C_2$ to $C_{12}$ heteroaryl and/or;

- the at least one ligand fragment Y is independently selected from substituted or unsubstituted $C_6$ to $C_{24}$ aryl, substituted or unsubstituted $C_2$ to $C_{24}$ heteroaryl, substituted or unsubstituted $C_1$ to $C_{24}$ alkyl, substituted or unsubstituted $C_3$ to $C_{24}$ carbocyclyl, substituted or unsubstituted $C_2$ to $C_{24}$ heterocyclyl, perfluorinated $C_1$ to $C_4$ alkyl, substituted $C_6$ to $C_{24}$ aryl in 3-position, substituted $C_6$ to $C_{24}$ aryl in 4-position, substituted 4-pyridyl, or substituted 5-pyrimidyl, and more preferred the at least one ligand fragment Y is independently selected from substituted or unsubstituted $C_6$ to $C_{24}$ aryl, substituted or unsubstituted $C_2$ to $C_{24}$ heteroaryl, wherein
the substituents are selected from the group comprising an electron-withdrawing group, halogen, F, Cl, and/or CN; and wherein the at least one ligand fragment Y has a directed dipole moment of less than -3.6 debye, and wherein the at least one ligand L is negatively charged; wherein

the anode layer comprises a first anode sub-layer and a second anode sub-layer, wherein

- the first anode sub-layer comprises a first metal having a work function in the range of ≥ 4 and ≤ 6 eV, and
- the second anode sub-layer comprises a transparent conductive oxide (TCO); and the hole injection layer is arranged between the first light emitting layer and the anode layer,
- the first anode sub-layer is arranged closer to the substrate, and
- the second anode sub-layer is arranged closer to the hole injection layer;

wherein the following ligand fragments Y are excluded:

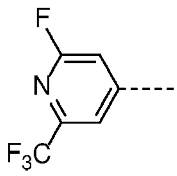, 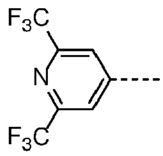, 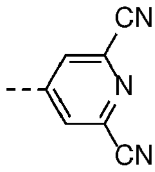, 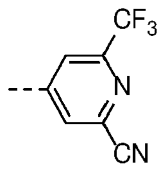, 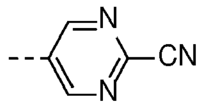,

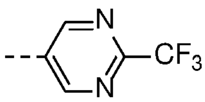, 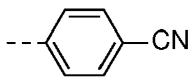, 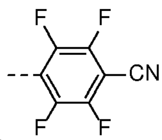 and 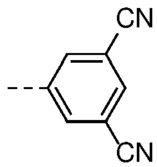.

3. The organic electroluminescent device according to claim 1 or 2, wherein the ligand fragment Y is a substituted $C_6$ to $C_{24}$ aryl or substituted $C_2$ to $C_{24}$ heteroaryl, perfluorinated $C_1$ to $C_4$ alkyl, substituted $C_6$ to $C_{24}$ aryl in 3-position, substituted $C_6$ to $C_{24}$ aryl in 4-position, substituted 4-pyridyl, or substituted 5-pyrimidyl, and more preferred substituted $C_6$ to $C_{24}$ aryl in 4-position, wherein the substituents are selected from the group comprising halogen, F, Cl, and/or CN.

4. The organic electroluminescent device according to any of claims 1 to 3, wherein the ligand fragment Y has a directed dipole moment selected from the group of ≤ -4.0 debye, ≤ -4.2 debye, ≤ -4.4 debye, ≤ -4.6 debye, ≤ -4.8 debye, ≤ -5.0 debye, ≤ -5.1 debye, ≤ -5.2 debye, ≤ -5.3 debye, ≤ -5.4 debye, ≤ -5.5 debye, ≤ -5.6 debye, ≤ -5.7 debye, or ≤ - 5.8 debye, more preferred the ligand fragment Y has a directed dipole moment less than -5.5 debye.

5. The organic electroluminescent device according to any one of claims 1 to 4, wherein the ligand fragment Y is represented by formula (IV)

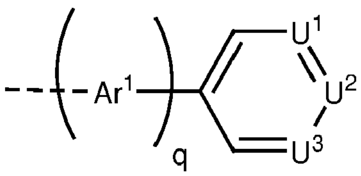 (IV),

wherein

q is selected from 0, 1, 2, and preferably 0 or 1;
$Ar^1$ is selected from substituted or unsubstituted phenyl, substituted or unsubstituted naphthyl, or substituted or unsubstituted anthracenyl, preferably $Ar^1$ is phenyl;
$U^1$ is selected from N or CR';
$U^2$ is selected from N or CR";
$U^3$ is selected from N or CR'''; and wherein R', R'' and R''' are independently selected from H, D, electron-withdrawing group, preferably F, perfluorinated $C_1$ to $C_{24}$ alkyl, preferably $CF_3$, CN, SCN, $OCF_3$, $-NO_2$, $-SF_5$.

6. The organic electroluminescent device according to any one of claims 1 to 5, wherein the ligand fragment Y is represented by formula (IV)

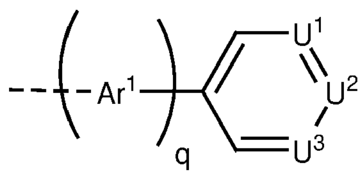 (IV),

wherein

q is selected from 0, 1, 2, and preferably 0 or 1;

$Ar^1$ is selected from unsubstituted phenyl, unsubstituted naphthyl, or unsubstituted anthracenyl, preferably $Ar^1$ is phenyl;
$U^1$ is selected from N or CR';
$U^2$ is selected from N or CR";
$U^3$ is selected from N or CR'"; and wherein R', R'' and R'" are independently selected from H, D, electron-withdrawing group, preferably F, perfluorinated $C_1$ to $C_{24}$ alkyl, preferably $CF_3$, CN, SCN, $OCF_3$, $-NO_2$, $-SF_5$.

**7.** The organic electroluminescent device according to any one of claims 1 to 6, wherein the ligand fragment Y is represented by formula (IV)

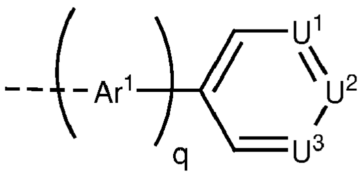 (IV),

wherein

q is selected from 0, 1, 2, and preferably 0 or 1;
$Ar^1$ is selected from unsubstituted phenyl, unsubstituted naphthyl, or unsubstituted anthracenyl, preferably $Ar^1$ is phenyl;
$U^1$ is selected from N or CR';
$U^2$ is selected from N or CR";
$U^3$ is selected from N or CR'"; and wherein R', R'' and R'" are independently selected from H, D, electron-withdrawing group, preferably F, perfluorinated $C_1$ to $C_{24}$ alkyl, preferably $CF_3$, CN, SCN, $OCF_3$, $-NO_2$, $-SF_5$;

wherein at most two of $U^1$, $U^2$ and $U^3$ are N, or if $U^1$, $U^2$ and $U^3$ are CR', CR" and CR"', respectively then at least one of R', R'', R'" is an electron-withdrawing group.

**8.** The organic electroluminescent device according to any one of claims 1 to 7, wherein the ligand fragment Y is selected from a group D1 to D11:

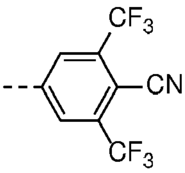 (D1), 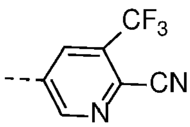 (D2), 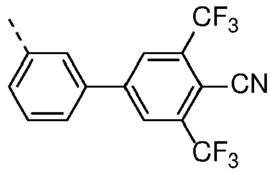 (D3),

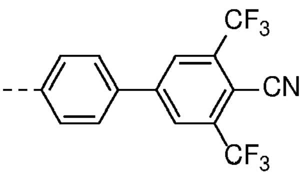 (D4), 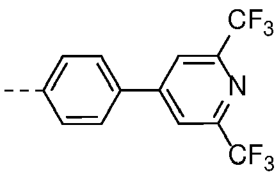 (D5), 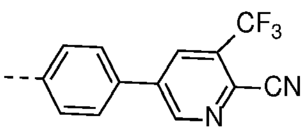 (D6),

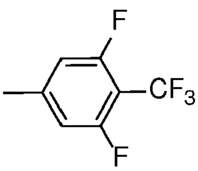 (D7), 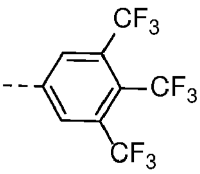 (D8), 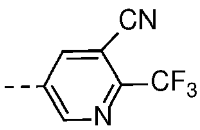 (D9),

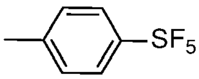 (D10), 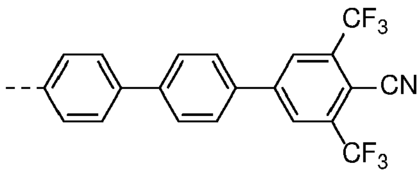 (D11);

wherein more preferred the ligand fragment Y is selected from the group of D1 to D6, D1 to D4 or D5 and D6.

**9.** The organic electroluminescent device according to any one of claims 1 to 8, wherein the coordinating linker X is selected from formula (IIa)

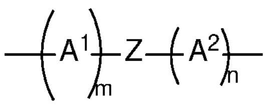 (IIa),

wherein

m and n are independently selected from 0 or 1;
Z is selected from the group comprising $CR^1$, O, N; and
$A^1$ and $A^2$ are independently selected from the group comprising C=O, C-O, SO or $SO_2$;
$R^1$ is independently selected from H, D, substituted or unsubstituted $C_6$ to $C_{24}$ aryl, substituted or unsubstituted $C_2$ to $C_{24}$ heteroaryl, substituted or unsubstituted $C_1$ to $C_{24}$ alkyl, ligand fragment Y;

wherein $A^1$ and $A^2$ together optional form a cycle with Z.

**10.** The organic electroluminescent device according to any one of claims 1 to 9, wherein the coordinating linker X is selected from formula (IIa)

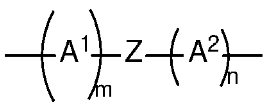 (IIa),

wherein

m and n are selected 1;
Z is selected from the group comprising $CR^1$, N; and
$A^1$ and $A^2$ are independently selected from the group comprising C=O, C-O or $SO_2$;
$R^1$ is independently selected from H, D, substituted or unsubstituted $C_6$ to $C_{24}$ aryl, substituted or unsubstituted $C_2$ to $C_{24}$ heteroaryl, substituted or unsubstituted $C_1$ to $C_{24}$ alkyl, ligand fragment Y;

wherein $A^1$ and $A^2$ together optional form a cycle with Z.

**11.** The organic electroluminescent device according to any one of claims 1 to 10, wherein the metal M is selected from the group comprising Li, Na, K, Cs, Mg, Ca, Sr, Ba, Al, In, Bi, Sc, Ti, Zr, Hf, V, Cr, Mo, W, Mn, Re, Fe, Ru, Co Rh, Ir, Ni, Pd, Pt, Cu, Ag, Au, Zn, La, Ce, Eu, or Yb, preferably the metal is selected from the group comprising Al, Bi, Cr, Mn, Fe, Co, Ni, Cu, Zn, Mo, Ag, Re, Au or Ce, and more preferred the metal is selected from the group comprising Al, Mn, Fe, Cu, Mo, Ag, Au or Ce.

**12.** The organic electroluminescent device according to any one of claims 1 to 11, wherein the at least one coordinating linker X is represented by formula (IIb):

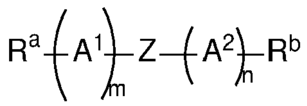

(IIb),

wherein

Z is selected from $CR^1$, N;
$A^1$ and $A^2$ are independently selected from C=O, C-O, or $SO_2$, and wherein $A^1$ and $A^2$ together optional form a cycle with Z;
$R^1$ is selected from H, D, CN, substituted or unsubstituted $C_6$ to $C_{24}$ aryl, substituted or unsubstituted $C_2$ to $C_{24}$ heteroaryl, substituted or unsubstituted $C_1$ to $C_{24}$ alkyl, substituted or unsubstituted $C_3$ to $C_{24}$ carbocyclyl, or substituted or unsubstituted $C_2$ to $C_{24}$ heterocyclyl, ligand fragment Y;
$R^a$ and $R^b$ are independently selected from substituted or unsubstituted $C_6$ to $C_{24}$ aryl, substituted or unsubstituted $C_2$ to $C_{24}$ heteroaryl, substituted or unsubstituted $C_1$ to $C_{24}$ alkyl, substituted or unsubstituted $C_3$ to $C_{24}$ carbocyclyl, or substituted or unsubstituted $C_2$ to $C_{24}$ heterocyclyl, ligand fragment Y, wherein the substituents are selected from the group comprising an electron-withdrawing group, halogen, F, Cl, CN, partially fluorinated or perfluorinated $C_1$ to $C_8$ alkyl, partially fluorinated or perfluorinated $C_6$ to $C_{12}$ aryl, or partially fluorinated or perfluorinated $C_2$ to $C_{12}$ heteroaryl;

wherein at least one of $R^1$, $R^a$ and $R^b$ contains a ligand fragment Y;
wherein at least one of the at least one ligand fragment Y is independently selected from substituted $C_6$ to $C_{24}$ aryl or unsubstituted or substituted $C_2$ to $C_{24}$ heteroaryl, and wherein the at least one of the at least one ligand fragment Y has a directed dipole moment of less than -3.6 debye, and wherein the at least one ligand L is negatively charged.

**13.** The organic electroluminescent device according to any one of claims 1 to 12, wherein $R^a$ and $R^b$ are independently selected from substituted or unsubstituted $C_6$ to $C_{24}$ aryl, substituted or unsubstituted $C_2$ to $C_{24}$ heteroaryl, substituted or unsubstituted $C_1$ to $C_{24}$ alkyl, ligand fragment Y.

**14.** The organic electroluminescent device according to any one of claims 1 to 13, wherein the at least one ligand L is represented by formula (III):

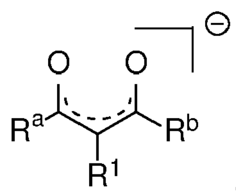

(III),

wherein

$R^1$ is selected from H, D, CN, substituted or unsubstituted $C_6$ to $C_{24}$ aryl, substituted or unsubstituted $C_2$ to $C_{24}$ heteroaryl, substituted or unsubstituted $C_1$ to $C_{24}$ alkyl, substituted or unsubstituted $C_3$ to $C_{24}$ carbocyclyl, or substituted or unsubstituted $C_2$ to $C_{24}$ heterocyclyl, ligand fragment Y;
$R^a$ and $R^b$ are independently selected from substituted or unsubstituted $C_6$ to $C_{24}$ aryl, substituted or unsubstituted $C_2$ to $C_{24}$ heteroaryl, substituted or unsubstituted $C_1$ to $C_{24}$ alkyl, substituted or unsubstituted $C_3$ to $C_{24}$ carbocyclyl, or substituted or unsubstituted $C_2$ to $C_{24}$ heterocyclyl, ligand fragment Y, wherein the substituents are selected from the group comprising an electron-withdrawing group, halogen, F, Cl, CN, partially fluorinated or perfluorinated $C_1$ to $C_8$ alkyl, perfluorinated $C_6$ to $C_{12}$ aryl, or partially fluorinated or perfluorinated $C_2$ to $C_{12}$ heteroaryl;

wherein at least one of $R^1$, $R^a$ and $R^b$ contains a ligand fragment Y;
wherein at least one of the at least one ligand fragment Y is independently selected from substituted $C_6$ to $C_{24}$ aryl or unsubstituted or substituted $C_2$ to $C_{24}$ heteroaryl, and wherein the at least one of the at least one ligand fragment Y has a directed dipole moment of less than -3.6 debye, and wherein the at least one ligand L is negatively charged.

**15.** The organic electroluminescent device according to any one of claims 1 to 14, wherein $R^1$ contains the ligand fragment Y.

**16.** The organic electroluminescent device according to any one of claims 1 to 15, wherein the semiconductor layer is arranged between the anode layer and the light emitting layer, preferably the semiconductor layer is a hole transport layer or hole injection layer.

**17.** The organic electroluminescent device according to any one of claims 1 to 16, wherein the semiconductor layer is arranged between the anode layer and the light emitting layer, preferably the semiconductor layer is a hole transport layer or hole injection layer; preferably the at least one semiconductor layer is arranged on the anode layer, and more preferably the at least one semiconductor layer is in direct contact with the anode, wherein the semiconductor layer is preferably a hole injection layer.

**18.** The organic electroluminescent device according to any one of claims 1 to 17, wherein the semiconductor layer comprises at least one matrix compound, wherein the at least one matrix compound is a non-polymeric compound, preferably the matrix compound is a covalent matrix compound or a substantially covalent matrix compound.

**19.** The organic electronic device according to claims 1 to 18, wherein the organic electronic device is an organic light emitting diode, a display device or an electroluminescent device, preferably an organic light emitting diode.

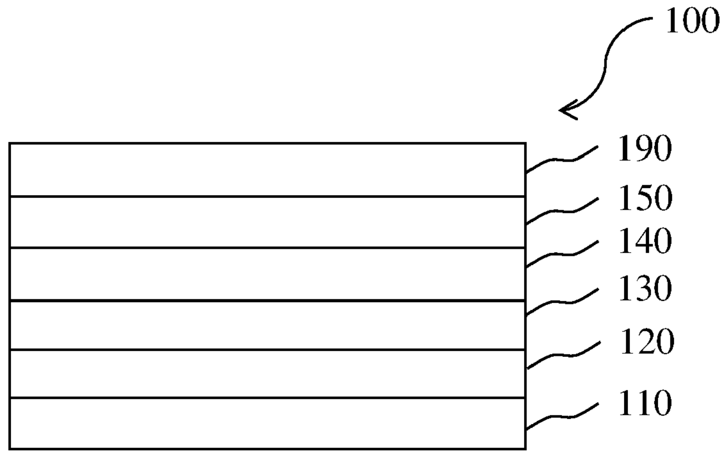


Fig. 1

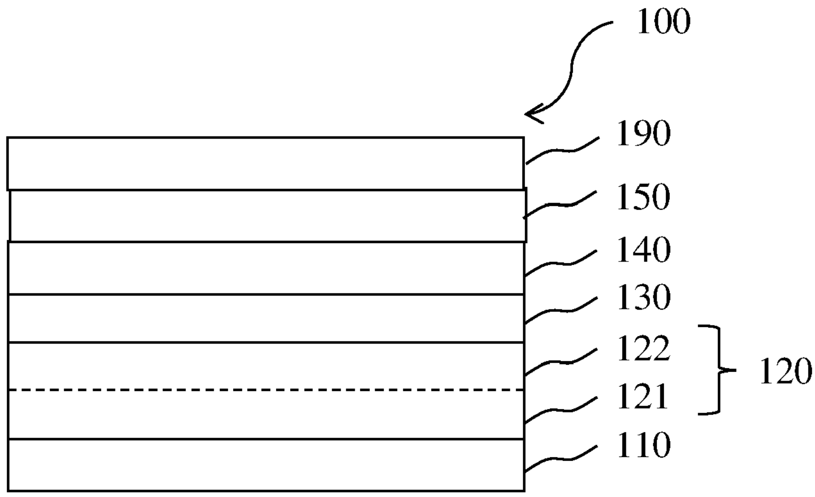


Fig.2

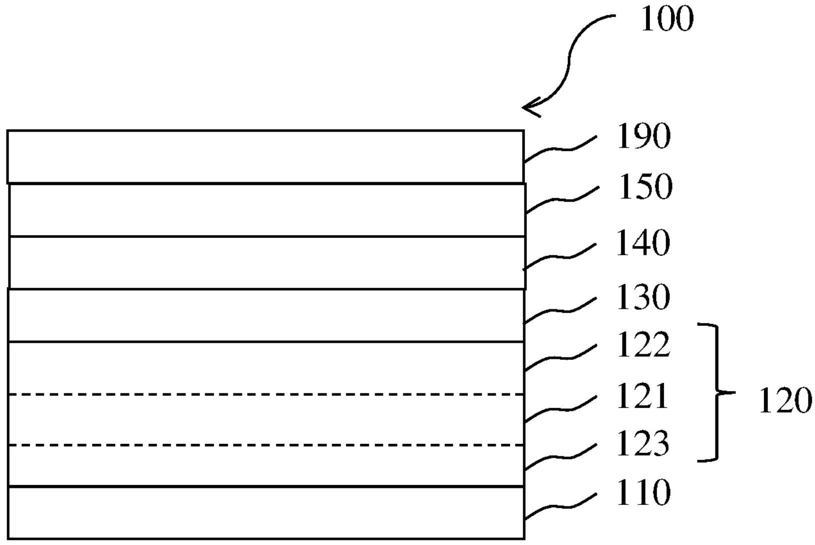


Fig.3

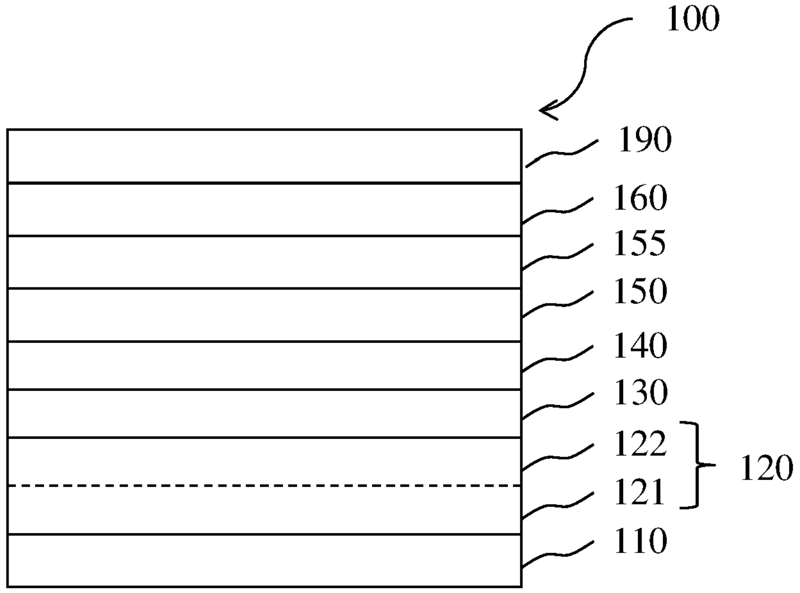
100
190
160
155
150
140
130
122
121
120
110

Fig.4

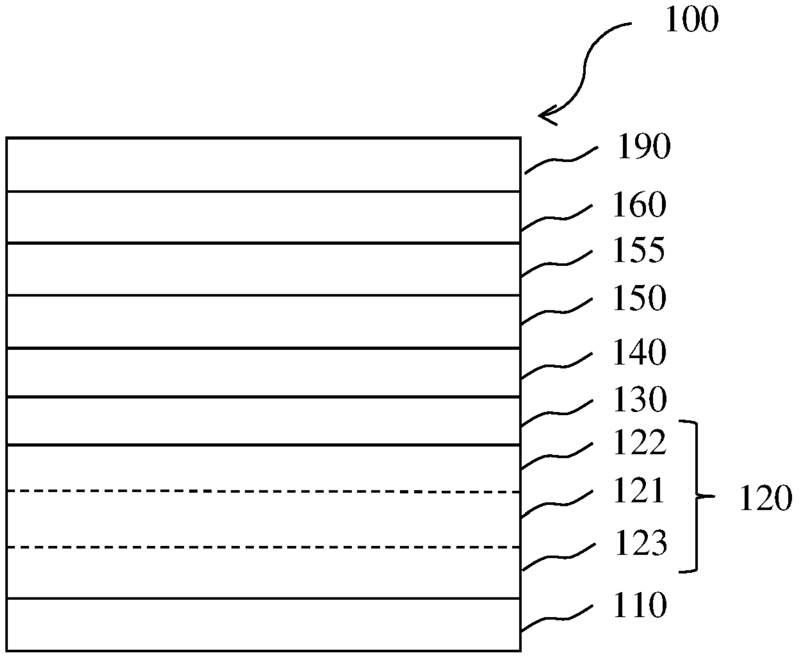
100
190
160
155
150
140
130
122
121
123
120
110

Fig.5

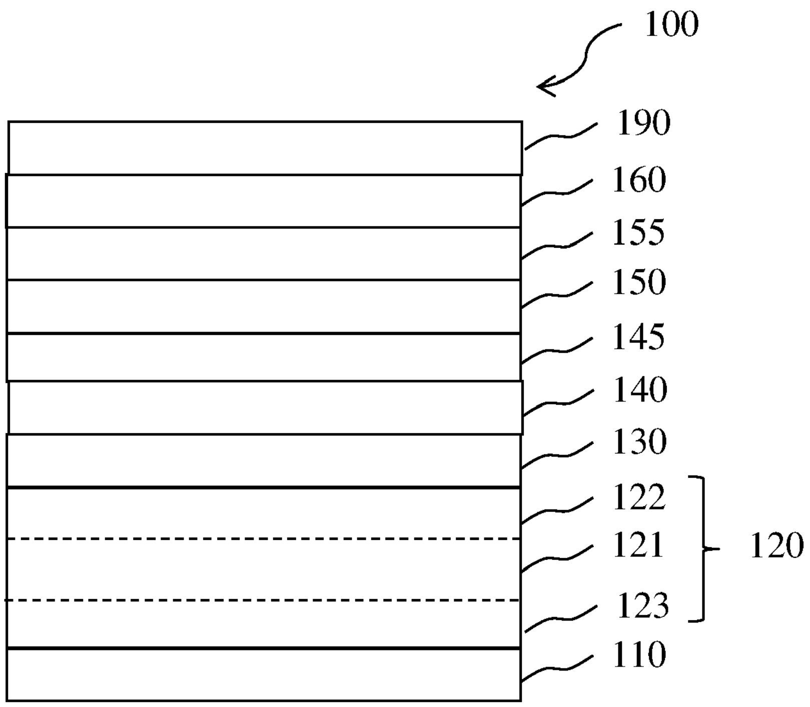


Fig.6

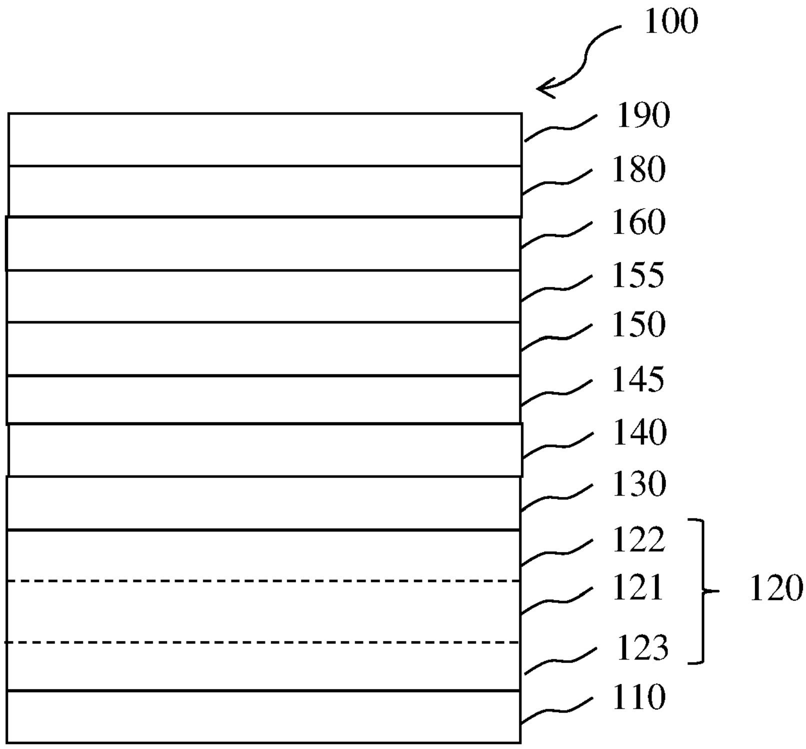


Fig.7

Europäisches Patentamt
European Patent Office
Office européen des brevets

# EUROPEAN SEARCH REPORT

Application Number
EP 21 18 0305

<table>
<tr><th colspan="3">DOCUMENTS CONSIDERED TO BE RELEVANT</th><th></th></tr>
<tr><th>Category</th><th>Citation of document with indication, where appropriate, of relevant passages</th><th>Relevant to claim</th><th>CLASSIFICATION OF THE APPLICATION (IPC)</th></tr>
<tr><td>X</td><td>EP 3 133 663 A1 (NOVALED GMBH [DE]) 22 February 2017 (2017-02-22)<br>* paragraphs [0009], [0070], [0092], [0185]; figures 1-3; tables 2,3 *</td><td>1-19</td><td rowspan="3">INV.<br>C07D213/61<br>C07D209/86<br>C09K11/06<br>C09B57/00<br>C09B57/10<br>H01L31/00<br>H01L31/04<br>H01L51/00<br>H01L51/50<br>H01M14/00</td></tr>
<tr><td>A</td><td>US 2015/048336 A1 (TANAKA YOHEI [JP]) 19 February 2015 (2015-02-19)<br>* paragraphs [0029] - [0032]; figures 1,2 *</td><td>1</td></tr>
<tr><td>X</td><td>WO 2016/188604 A1 (MERCK PATENT GEMBH [DE]) 1 December 2016 (2016-12-01)<br>* page 8, line 28 *<br>* page 76; metal complexes D1 to D3 *<br>* claims 1,11 *</td><td>1-19</td></tr>
<tr><td>A</td><td>JP 2012 119195 A (NAT INST FOR MATERIALS SCIENCE) 21 June 2012 (2012-06-21)<br>* page 5, paragraph [0018]; complex 5 *<br>* page 6 to page 8; paragraphs [0020], [0021], [0029]; ligands with substituents R2 I-2, I-3, I-11 *<br>* page 14; paragraph [0065]; example 37 *<br>* compounds 3; page 15; paragraph [0071] *<br>* example 38; compound 6; page 16 *<br>* paragraph [0079]; example 3; compounds 8-10 *</td><td>1-19</td><td rowspan="2">TECHNICAL FIELDS SEARCHED (IPC)<br><br>C07D<br>C09K<br>C09B<br>H01L<br>H01M</td></tr>
<tr><td>A</td><td>EP 0 506 999 A1 (MITSUI TOATSU CHEMICALS [JP]; PIONEER ELECTRONIC CORP [JP]) 7 October 1992 (1992-10-07)<br>* page 5; table 1; compound (6) *<br>* page 7; table 2; compound (19) *<br>* page 10; table 1; compound (36) *</td><td>1,14</td></tr>
<tr><td colspan="4">The present search report has been drawn up for all claims</td></tr>
</table>

1

| Place of search | Date of completion of the search | Examiner |
|---|---|---|
| The Hague | 22 November 2021 | Chin, Patrick |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

# ANNEX TO THE EUROPEAN SEARCH REPORT ON EUROPEAN PATENT APPLICATION NO. EP 21 18 0305

This annex lists the patent family members relating to the patent documents cited in the above-mentioned European search report.
The members are as contained in the European Patent Office EDP file on
The European Patent Office is in no way liable for these particulars which are merely given for the purpose of information.

22-11-2021

| Patent document cited in search report | Publication date | Patent family member(s) | Publication date |
|---|---|---|---|
| EP 3133663 A1 | 22-02-2017 | CN 107925014 A | 17-04-2018 |
| | | EP 3133663 A1 | 22-02-2017 |
| | | EP 3338313 A1 | 27-06-2018 |
| | | JP 6813569 B2 | 13-01-2021 |
| | | JP 2018525835 A | 06-09-2018 |
| | | JP 2021064797 A | 22-04-2021 |
| | | KR 20180041164 A | 23-04-2018 |
| | | US 2018240996 A1 | 23-08-2018 |
| | | WO 2017029370 A1 | 23-02-2017 |
| US 2015048336 A1 | 19-02-2015 | JP 5912174 B2 | 27-04-2016 |
| | | JP WO2013150592 A1 | 14-12-2015 |
| | | KR 20140146070 A | 24-12-2014 |
| | | US 2015048336 A1 | 19-02-2015 |
| | | WO 2013150592 A1 | 10-10-2013 |
| WO 2016188604 A1 | 01-12-2016 | EP 3298638 A1 | 28-03-2018 |
| | | KR 20180011202 A | 31-01-2018 |
| | | WO 2016188604 A1 | 01-12-2016 |
| JP 2012119195 A | 21-06-2012 | JP 5713285 B2 | 07-05-2015 |
| | | JP 2012119195 A | 21-06-2012 |
| EP 0506999 A1 | 07-10-1992 | DE 69101833 T2 | 20-10-1994 |
| | | EP 0506999 A1 | 07-10-1992 |
| | | US 5158703 A | 27-10-1992 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 111018921
- EP 2722908 A1


### Non-patent literature cited in the description


- **ALLEN, LELAND C.** Electronegativity is the average one-electron energy of the valence-shell electrons in ground-state free atoms. *Journal of the American Chemical Society,* 1989, vol. 111 (25), 9003-9014
- *CHEMICAL ABSTRACTS,* 207739-72-8
- *CHEMICAL ABSTRACTS,* 139255-16-6
- *CHEMICAL ABSTRACTS,* 1613079-70-1